(19) Europäisches Patentamt
European Patent Office
Office européen des brevets

(11) EP 4 212 874 A1

(12) **EUROPEAN PATENT APPLICATION**
published in accordance with Art. 153(4) EPC

(43) Date of publication:
**19.07.2023 Bulletin 2023/29**

(21) Application number: **21864467.2**

(22) Date of filing: **06.09.2021**

(51) International Patent Classification (IPC):
***G01N 33/53*** *(2006.01)*

(52) Cooperative Patent Classification (CPC):
**G01N 33/53**

(86) International application number:
**PCT/JP2021/032730**

(87) International publication number:
**WO 2022/050416 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.09.2020 JP 2020150142**

(71) Applicants:
- **MITSUBISHI GAS CHEMICAL COMPANY, INC.
Chiyoda-ku
Tokyo 100-8324 (JP)**
- **National University Corporation Chiba University
Chiba-shi, Chiba 263-8522 (JP)**

(72) Inventors:
- **SAWANO Erika
Tokyo 100-8324 (JP)**
- **TAKASE Yuki
Tokyo 100-8324 (JP)**
- **SHIMOJO Naoki
Chiba-shi, Chiba 260-8670 (JP)**
- **YAMAIDE Fumiya
Chiba-shi, Chiba 260-8677 (JP)**
- **NAKANO Taiji
Chiba-shi, Chiba 260-8677 (JP)**

(74) Representative: **Müller-Boré & Partner
Patentanwälte PartG mbB
Friedenheimer Brücke 21
80639 München (DE)**

Remarks:
The complete document including Reference Table(s) and the Sequence Listing(s) can be downloaded from the EPO website

(54) **ALLERGIC DISEASE DETERMINATION METHOD AND DETERMINATION SYSTEM**

(57) The present invention relates to an allergic disease determination method and the like comprising: a step for causing two or more types of probes selected from an allergen and a fragment thereof and a biological sample collected from a subject to contact with each other; a step for detecting a reaction between the probes and an antibody contained in the biological sample; a step for determining a type of the antibody reacted with the probes; a step for inputting data associated with the reaction of each of the at least two types of probes with the antibody and data associated with a type of the bound antibody into a prediction model and acquiring a prediction result; and a step for determining an allergic disease on the basis of the prediction result, wherein the prediction model is a nonlinear model or a model having two or more explanatory variables and two or more terms, the model being created using training data associated with the reaction of at least the probes with the antibody and training data associated with the type of the bound antibody.

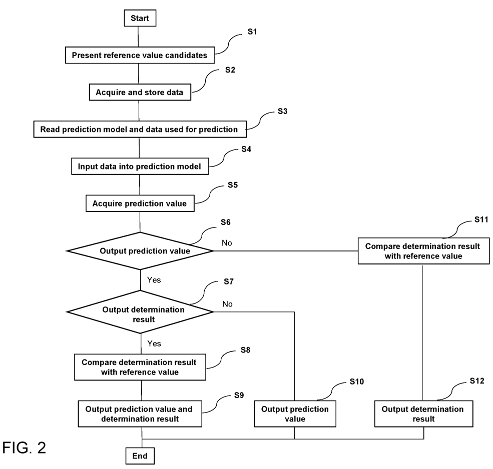

FIG. 2

EP 4 212 874 A1

## Description

Technical Field

The present invention relates to a method of determining an allergic disease and a kit, a biochip, a system used therefor, etc.

Background Art

The number of patients suffering from allergic diseases tends to increase, and countermeasures against them have become an issue. Allergic diseases are diverse in that there are many allergens that cause them, and the symptoms and responsiveness to treatment vary from patient to patient. In addition, although research on allergy has progressed dramatically over the past several decades, its pathology is more complicated than imagined and still largely remains unknown. Such diversity and complexity make it difficult to cope with allergic diseases. Accurate diagnosis is important for dealing with allergic diseases, but it is difficult to say that diagnostic methods for diverse and complicated allergic diseases have been sufficiently established. Measurement of allergen-specific IgE is the most common method for diagnosing allergic diseases, and in recent years, attempts have been made to combine measurement results of IgG4 in addition to IgE (Patent Literature 1).

Citation List

Patent Literature

Patent Literature 1: WO2010/110454

Summary of Invention

Technical Problem

There is a demand for an improved method for diagnosing allergic diseases.

Solution to Problem

In one embodiment, the present invention provides the following.

[1] A method of determining an allergic disease, comprising:

a step of allowing two or more probes selected from an allergen and a fragment thereof to come into contact with a biological sample collected from a subject;
a step of detecting a reaction between the probe and an antibody contained in the biological sample;
a step of identifying type of the antibody that reacted with the probe;
a step of inputting data on the reaction with the antibody for each of the two or more probes and data on the type of the antibody bound into a prediction model so as to obtain a prediction result; and
a step of determining the allergic disease based on the prediction result, wherein the prediction model is a nonlinear model or a model with two or more explanatory variables and two or more terms, which is created using at least training data on the reaction with the antibody for the probes and training data on the type of the antibody bound.

[2] The method according to the above [1], wherein determining the allergic disease is selected from determining whether the allergic disease is mild or severe and predicting a total ASCA score.
[3] A method of obtaining an index for allergic disease determination, comprising:

a step of acquiring data on a reaction between two or more probes selected from an allergen and a fragment thereof and an antibody contained in a biological sample collected from a subject, and data on the type of the antibody reacted, the data being obtained by allowing the two or more probes to come into contact with the biological sample; and
a step of inputting the data into a prediction model so as to obtain a prediction result,

wherein the prediction model is a nonlinear model or a model with two or more explanatory variables and two or more terms, which is created using at least training data on the reaction with the antibody for the probes and training data on the type of the antibody bound.

[4] The method according to any one of the above [1] to [3], wherein the type of the antibody is selected from IgE and IgG4.

[5] The method according to any one of the above [1] to [4], wherein the probe comprises an overlapping fragment of the allergen.

[6] The method according to any one of the above [1] to [5], wherein the probe is solid-phase immobilized.

[7] The method according to any one of the above [1] to [6], wherein the data on the reaction with the antibody are of intensity of reaction with the antibody.

[8] The method according to any one of the above [1] to [7], wherein additional training data are used for creating the prediction model.

[9] The method according to any one of the above [1] to [8], wherein the allergen is selected from a food allergen, an inhalant allergen, and a contact allergen.

[10] The method according to any one of the above [1] to [9], wherein the food allergen is selected from casein, lactalbumin, lactoglobulin, ovomucoid, ovalbumin, conalbumin, lysozyme, gliadin, an amylase inhibitor, albumin, globulin, gluten, and tropomyosin.

[11] The method according to any one of the above [1] to [10], which is used in a monitoring of the allergic disease, wherein the biological sample collected from the subject comprises two or more biological samples collected at different times, and wherein the step of determining an allergic disease comprises comparing prediction results obtained for the two or more biological samples with each other.

[12] The method according to any one of the above [1] to [11], wherein the allergic disease is food allergy.

[13] A kit for use in the method according to any one of the above [1] to [12], comprising two or more probes selected from an allergen and a fragment thereof.

[14] A biochip for use in the method of any one of [1] to [12], wherein the two or more probes selected from an allergen and a fragment thereof are immobilized on a substrate.

[15] An allergic disease determination system, comprising:

a data acquisition unit that acquires data on a reaction between two or more probes selected from an allergen and a fragment thereof and an antibody contained in a biological sample collected from a subject, and data on the type of the antibody reacted, the data being obtained by allowing the two or more probes to come into contact with the biological sample;
a data processing unit that inputs the data into a prediction model so as to obtain a prediction result;
a determination unit that determines an allergic disease based on the prediction result; and

an output unit that outputs a determination,
wherein the prediction model is a nonlinear model or a model with two or more explanatory variables and two or more terms, which is created using at least training data on the reaction with the antibody for at least the probes and training data on the type of the antibody bound.

[16] A program for causing a computer to execute the method according to any one of the above [1] to [12].

[17] A storage medium storing the program according to the above [16]. Advantageous Effects of Invention

The present invention has one or two or more of the following effects, depending on the embodiment thereof.

(1) It is possible to predict the degree of a symptom of an allergic disease.
(2) It is possible to predict a response to a challenge test such as an oral food challenge test.
(3) Diagnostic performance is improved compared to conventional specific IgE-based techniques.

Brief Description of Drawings

Figure 1 is a block diagram showing the functional configuration of an allergic disease determination system.
Figure 2 is a flowchart showing an example of data processing in the system of the present invention.
Figure 3 is a block diagram showing an example of the hardware configuration of a computer that implements the stand-alone system of the present invention.
Figure 4 is a schematic diagram of partial peptides immobilized on beads.

Figure 5 is a decision tree of prediction model 1-4. A prediction result "1" means severe, and "0" means mild.
Figure 6 is a decision tree of prediction model 1-5. A prediction result " 1" means severe, and "0" means mild.
Figure 7 is a ROC curve created from the prediction values of each subject obtained by prediction model 1-1.
Figure 8 is a dot histogram created from the prediction values of each subject obtained by prediction model 1-1.
Figure 9 is a ROC curve created from prediction values for each subject obtained by prediction model 1-2.
Figure 10 is a dot histogram created from prediction values for each subject obtained by prediction model 1-2.
Figure 11 is a ROC curve created from prediction values for each subject obtained by prediction model 1-3.
Figure 12 is a dot histogram created from prediction values for each subject obtained by prediction model 1-3.
Figure 13 is a ROC curve created from prediction values for each subject obtained by prediction model 1-4.
Figure 14 is a dot histogram created from prediction values for each subject obtained by prediction model 1-4.
Figure 15 is a ROC curve created from prediction values for each subject obtained by prediction model 1-5.
Figure 16 is a dot histogram created from prediction values for each subject obtained by prediction model 1-5.
Figure 17 is a ROC curve created from prediction values for each subject obtained by prediction model 1-6.
Figure 18 is a dot histogram created from prediction values for each subject obtained by prediction model 1-6.
Figure 19 is a ROC curve created from prediction values for each subject obtained by prediction model 1-7.
Figure 20 is a dot histogram created from prediction values for each subject obtained by prediction model 1-7.
Figure 21 is a ROC curve created from prediction values for each subject obtained by prediction model 1-8.
Figure 22 is a dot histogram created from prediction values for each subject obtained by prediction model 1-8.
Figure 23 is a decision tree of prediction model 2-3. The numbers in the prediction results each represent the sufficiency rate of the ASCA score.
Figure 24 is a decision tree of prediction model 2-3. The numbers in the prediction results each represent the sufficiency rate of the ASCA score.
Figure 25 is a scatter diagram created from the prediction values of each subject obtained by prediction model 2-1 and observed values.
Figure 26 is a scatter diagram created from the prediction values of each subject obtained by prediction model 2-2 and observed values.
Figure 27 is a scatter diagram created from the prediction values of each subject obtained by prediction model 2-3 and observed values.
Figure 28 is a scatter diagram created from the prediction values of each subject obtained by prediction model 2-4 and observed values.
Figure 29 is a scatter diagram created from the prediction values of each subject obtained by prediction model 2-5 and observed values.
Figure 30 is a scatter diagram created from the prediction values of each subject obtained by prediction model 2-6 and observed values.

## Description of Embodiments

Hereinafter, the embodiments of the present invention will be described. It is to be noted that the below-described materials, features and the like are not intended to limit the present invention, but may be combined with one another or may be modified in various ways within the range of the spirit of the present invention.

All technical terms and scientific terms used in the present description have the same meanings as those that are generally understood by those skilled in the art, unless otherwise specified. All patents, patent applications, and other publications (including online information), which are cited in the present description, are incorporated herein by reference in their entirety. Moreover, the present description includes the contents as disclosed in the description and/or drawings of Japanese patent application (Japanese Patent Application No. 2020-150142), which are priority documents of the present application filed on September 7, 2020.

### 1. Allergic disease determination method

One aspect of the present invention relates to a method of determining an allergic disease (hereinafter sometimes referred to as the "determination method of the present invention"). The determination method of the present invention comprises:

a step of allowing two or more probes selected from an allergen and a fragment thereof to come into contact with a biological sample collected from a subject;
a step of detecting a reaction between the probe and an antibody contained in the biological sample;
a step of identifying type of the antibody that reacted with the probe;
a step of inputting data on the reaction with the antibody for each of the two or more probes and data on the type

of the antibody bound into a prediction model so as to obtain a prediction result; and
a step of determining an allergic disease based on the prediction result, wherein the prediction model is a nonlinear model or a model with two or more explanatory variables and two or more terms, which is created using at least training data on the reaction with the antibody for the probes and training data on the type of the antibody bound.

Allergic diseases are diseases in which the immune system overreacts to allergens. Examples of allergic diseases include food allergy, atopic dermatitis, allergic rhinitis, allergic conjunctivitis, bronchial asthma, urticaria, anaphylactic shock, and drug allergy. In one embodiment, the allergic disease is a disease associated with IgE. In another embodiment, the allergic disease belongs to type I allergy. In particular embodiments, the allergic disease is food allergy.

In the determination method of the present invention, an allergen and/or a fragment thereof is used as a probe for detecting an antibody contained in a biological sample (hereinafter sometimes referred to as "target antibody"). Examples of an allergen used as the probe include, but are not limited to, food allergens, inhalant allergens, and contact allergens.

Examples of food allergens include, but are not limited to, allergens such as milk and dairy products, eggs, fish, meat, mollusks, shellfish, cereals, nuts, fruits, vegetables, mushrooms, and potatoes. Examples of fish allergens include allergens such as salmon, mackerel, salmon roe, bass, flatfish, and cod. Examples of meat allergens include allergens such as chicken, pork, and gelatin. Examples of mollusk allergens include allergens such as abalone and squid. Examples of shellfish allergens include allergens such as shrimp and crab. Examples of cereal allergens include allergens such as wheat, rye, barley, oats, spelt wheat, kamut wheat, buckwheat, peanuts, sesame, soybeans, and lupine. Examples of nut allergens include allergens such as almonds, cashew nuts, walnuts, hazelnuts, walnuts, pecan nuts, brazil nuts, pistachio nuts, and macadamia nuts. Examples of fruit allergens include allergens such as oranges, kiwifruit, bananas, peaches, and apples. Examples of vegetable allergens include allergens such as celery and mustard.

In some embodiments, examples of allergens include allergens of ingredients listed as specified ingredients or items equivalent to specified ingredients in the Japanese Food Labeling Standards. Examples of specified ingredients include shrimp, crab, wheat, buckwheat, eggs, milk, and peanuts. Examples of items equivalent to semi-specified ingredients include almonds, abalone, squid, salmon roe, oranges, cashew nuts, kiwifruit, beef, walnuts, sesame, salmon, mackerel, soybeans, chicken, bananas, pork, matsutake mushrooms, peaches, yams, apples, and gelatin.

In another embodiment, examples of allergens include allergens for which labeling is mandatory under Regulation No. 1169/2011 of the European Parliament and of the Council. Examples of such allergens include allergens such as cereals containing gluten (wheat, rye, barley, oats, spelt wheat, and kamut wheat), shellfish, eggs, fish, peanuts, soybeans, milk and dairy products, nuts (almonds, hazelnuts, walnuts, cashew nuts, pecan nuts, brazil nuts, pistachio nuts, and macadamia nuts), celery, mustard, sesame, lupine, and mollusks.

In another embodiment, examples of allergens include food allergens for which labeling is mandatory under the U.S. Consumer Protection Law. Examples of such foods include milk, eggs, fish (such as bass, flounder, and cod), shellfish (such as crab, lobster, and shrimp), nuts (such as almonds, walnuts, and pecan nuts), peanuts, wheat, and soybeans.

In another embodiment, examples of food allergens include animal and plant allergens. Examples of animal allergens include casein (e.g., αs1-casein, αs2-casein, β-casein, or κ-casein), albumin (e.g., lactalbumin, ovalbumin, conalbumin, or serum albumin), globulin (e.g., lactoglobulin or immunoglobulin), tropomyosin, and parvalbumin (e.g., β-parvalbumin). Examples of plant allergens include cupin (e.g., 7S globulin (vicilin), 11S globulin (legmin), or 13S globulin), prolamin (e.g., α-amylase inhibitor, 2S albumin, or non-specific lipid transfer protein (nsLTP)), PR-10 (Bet v 1 homolog), profilin, and oleosin. In some embodiments, allergens are selected from casein, albumin, globulin, tropomyosin, parvalbumin, cupin, prolamin, PR-10 (Bet v 1 homolog), profilin, and oleosin.

In particular embodiments, examples of milk allergens encompass αs1-casein (Bos d 9), αs2-casein (Bos d 10), β-casein (Bos d 11), κ-casein (Bos d 12), α-lactalbumin (Bos d 4), serum albumin (Bos d 6), β-lactoglobulin (Bos d 5), and immunoglobulin (Bos d 7).

In particular embodiments, examples of egg allergens encompass ovomucoid (Gal d 1), ovalbumin (Gal d 2), ovotransferrin (conalbumin, Gal d 3), and lysozyme C (Gal d 4).

In particular embodiments, examples of fish allergens encompass Atlantic salmon tropomyosin (Sal s 4), Atlantic salmon β-parvalbumin (Sal s 1), β-parvalbumin (Gad c 1), tilapia *(Oreochromis mossambicus)* tropomyosin (Ore m 4), herring β-parvalbumin (Clu h 1), carp β-parvalbumin (Crp c 1), flounder β-parvalbumin (Lep w 1), rainbow trout β-parvalbumin (Onc m 1), sardine β-parvalbumin (Sar sa 1), yellowfin tuna β-parvalbumin (Thu a 1), and swordfish β-parvalbumin (Xip g 1).

In particular aspects, examples of mollusk allergens encompass abalone tropomyosin (Hal l 1), sagittated calamary *(Todarodespacificus)* tropomyosin (Tod p 1), brown garden snail tropomyosin (Hel as 1), Japanese oyster tropomyosin (Cra g 1), and Sydney Rock oyster tropomyosin (Sac g 1).

In particular aspects, examples of shellfish allergens encompass Indian shrimp (*Fenneropenaeus indicus*) tropomyosin (Pen i 1), greasyback shrimp *(Metapenaeus ensis)* tropomyosin (Met e 1), American lobster (*Homarus*

*americanus*) tropomyosin (Hom a 1), black tiger shrimp (Penaeus monodon) tropomyosin (Pen m 1), brown shrimp (*Crangon crangon*) tropomyosin (Pen a 1), whiteleg shrimp (*Litopenaeus vannamei*) tropomyosin (Lit v 1), stone shrimp (*Melicertus latisulcatus*) tropomyosin (Mel l 1), Alaskan pink shrimp (*Pandalus eous*) tropomyosin (Pan b 1), fresh water shrimp *(Macrobrachium nipponense*) tropomyosin (Mac r 1), California bay shrimp (Crangon crangon) tropomyosin (Cra c 1), Japanese spiny lobster (*Panulirus japonicus*) tropomyosin (Pan s 1), flower crab *(Portunus pelagicus)* tropomyosin (Por p 1), and Asian paddle crab (*Charybdis japonica*) tropomyosin (Cha f 1).

In particular embodiments, examples of cereal allergens encompass wheat gluten : wheat ω-5gliadin (Tri a 19), wheat α/β-gliadin (Tri a 21), wheat γ-gliadin (Tri a 20), wheat high-molecular-weight glutenin (Tri a 26), wheat low-molecular-weight glutenin (Tri a 36), wheat α-amylase inhibitor (monomer: Tri a 15; dimer: Tri a 28; tetramer: Tri a 29, Tria 30), wheat nsLTP (Tri a 14), barley α-amylase inhibitor (Hor v 15), buckwheat 13S globulin (molecular weight: 76 kDa), buckwheat 13S globulin β-subunit (molecular weight: 24 kDa; Fag e 1), buckwheat 7S globulin (vicilin, Fag e 3), buckwheat 2S albumin (Fag e 2), peanut 7S globulin (vicilin, Ara h 1), peanut 11S globulin (legmin, Ara h 3), peanut 2S albumin (Ara h 2, 6, 7), peanut nsLTP (Ara h 9, 16, 17), peanut Bet v 1 (Ara h 8), peanut profilin (Ara h 5), peanut oleosin (Ara h 10, 11, 14, 15), sesame 7S globulin (vicilin, Ses i 3), sesame 11S globulin (legmin, Ses i 6, 7), sesame 2S albumin (Ses i 1, 2), sesame oleosin (Ses i 4, 5), soybean 7S globulin (vicilin, Gly m 5), soybean 11S globulin (legmin, Gly m 6), soybean nsLTP (Gly m 1), soybean 2S albumin (Gly m 8), soybean Bet v 1 (Gly m 4), soybean profilin (Gly m 3), maize nsLTP (Zea m 14), pea 7S globulin (vicilin, Pis s 1), mung bean 7S globulin (vicilin, Vig r 2), mung bean Bet v 1 (Vig r 1), and maize profilin (Zea m 12).

In particular embodiments, examples of nut allergens encompass almond 11S globulin (legmin, Pru du 6), almond nsLTP 1 (Pru du 3), almond profilin (Pru du 4), cashew nut 7S globulin (vicilin, Ana o 1), cashew nut 11S globulin (legmin, Ana o 2), cashew nut 2S albumin (Ana o 3), walnut 7S globulin (vicilin, Jug r 2), walnut 11S globulin (legmin, Jug r 4), walnut 2S albumin (Jug r 1), walnut nsLTP (Jug r 3), walnut PR-10 (Jug r 5), walnut profilin (Jug r 7), hazelnut 7S globulin (vicilin, Cor a 11), hazelnut 11S globulin (legmin, Cor a 9), hazelnut 2S albumin (Cor a 14), hazelnut nsLTP (Cor a 8), hazelnut Bet v 1 (Cor a 1), hazelnut profilin (Cor a 2), hazelnut oleosin (Cor a 12, 13), pecan nut 7S globulin (vicilin, Car i 2), pecan nut 1 1S globulin (legmin, Car i 4), pecan nut 2S albumin (Car i 1), brazil nut 11S globulin (legmin, Ber e 2), brazil nut 2S albumin (Ber e 1), pistachio nut 7S globulin (vicilin, Pis v 3), pistachio nut 11S globulin (legmin, Pis v 2, 5), pistachio nut 2S albumin (Pis v 1), and black walnut 11S globulin (legmin, Jung n 4).

In particular embodiments, examples of fruit allergens encompass orange nsLTP (Cit s 3), orange profilin (Cit s 2), green kiwi 2S albumin (Act d 13), golden kiwi nsLTP (Act c 10), green kiwi nsLTP (Act d 10), golden kiwi Bet v 1 (Act c 8), green kiwi Bet v 1 (Act d 8), green kiwi profilin (Act d 9), banana nsLTP (Mus a 3), banana profilin (Mus a 1), peach nsLTP (Pru p 3), peach Bet v 1 (Pru p 1), peach profilin (Pru p 4), apple nsLTP (Mal d 3), apple Bet v 1 (Mal d 1), apple profilin (Mal d 4), strawberry nsLTP (Fra a 3), apricot nsLTP (Pru ar 3), cherry nsLTP (Pru av 3), European plum nsLTP (Pru d 3), pomegranate nsLTP (Pun g 1), red raspberry nsLTP (Rub 13), grape nsLTP (Vit v 1), mashipear nsLTP (Pyr c 3), strawberry Bet v 1 (Fra a 1), apricot Bet v 1 (Pru ar 1), cherry Bet v 1 (Pru av 1), nashi pear Bet v 1 (Pyr c 1), raspberry Bet v 1 (Rub i 1), strawberry profilin (Fra a 4), cherry profilin (Pru av 4), European pear profilin (Pyr c 4), watermelon profilin (Citr 12), muskmelon profilin (Cuc m 2), lychee profilin (Lit c 1), and pineapple profilin (Ana c 1).

In particular embodiments, examples of fruit allergens encompass celery nsLTP (Api g 2, 6), celery Bet v 1 (Api g 1), celery profilin (Api g 4), yellow mustard 2S albumin (Sin a 1), yellow mustard profilin (Sin a 4), asparagus nsLTP (Aspa o 1), lettuce nsLTP (Lac s 1), cabbage nsLTP (Bra o 3), tomato nsLTP (Sola l 13, 16, 17), carrot Bet v 1 (Dau c 1), tomato Bet v 1 (Sola 14), carrot profilin (Dau c 4), bell pepper profilin (Cap a 2), and tomato profilin (Sola l 1).

Examples of inhalant allergens include, but are not limited to, indoor allergens, pollen allergens, and mold allergens. Examples of indoor allergens include, but are not limited to, allergens such as dust, dust mites, tatami mats, buckwheat husks, pet hair, clothing, and bedding (e.g., cotton, silk, wool, feathers). Examples of pollen allergens include, but are not limited to, allergens such as hogweed *(Ambrosia artemisiifolia*), Japanese hop (*Humulus japonicus*), cryptomeria (*Cryptomeria japonica*), Japanese red pine *(Pinus densiflora),* miscanthus (*Miscanthus sinensis Anderss*), cattail (Typha domingensis Pers), Japanese mugwort (*Artemisia princeps Pampan*), olive *(Olea europaea),* plane tree (*Platanus orientalis),* spreading pellitory (*Parietaria judaica*), European alder *(Alnus glutinosa*), Japanese white birch (*Betula platyphylla* var. *japonica*), European chestnut *(Carpinus betulus),* sweet chestnut (*Castanea sativa*), hazelnut (*Corylus avellana*), European beech (*Fagus sylvatica*), European hop-hornbeam (*Ostrya carpinifolia*), white oak *(Quercus alba),* redroot amaranth *(Amaranthus retroflexus L.),* white goosefoot *(Chenopodium album L.),* hazelnut *(Corylus avellana*), saffron *(Crocus sativus),* Bahama grass *(Cynodon dactylon),* annual mercury (*Mercurialis annua*), timothy grass (*Phleum pratense L.*), date palm *(Phoenix dactylifera*), English plantain *(Plantago lanceolata L.),* Mesquite, *Brassica rapa,* saltwort (*Salsola komarovii),* and lilac (*Syringa vulgaris).* Examples of mold allergens include, but are not limited to, allergens such as Alternaria, Penicillium, Candida, Cladosporium, and Aspergillus.

In particular embodiments, examples of pollen allergens encompass hogweed nsLTP (Amb a 6), Japanese mugwort nsLTP (Art v 3), olive nsLTP (Ole e 7), plane tree nsLTP (Pla or 3), spreading pellitory nsLTP (Par j 1), European alder Bet v 1(Aln g 1), Japanese white birch Bet v 1(Bet v 1), European chestnut Bet v 1(Car b 1), sweet chestnut Bet v 1(Cas s 1), hazelnut Bet v 1(Cor a 1), European beech Bet v 1(Fag s 1), hop-hornbeam Bet v 1(Ost c 1), white oak

Bet v 1(Qua a 1), redroot amaranth profilin (Ama r 2), hogweed profilin (Amb a 8), Japanese mugwort profilin (Art v 4), Japanese white birch profilin (Bet v 2), white goosefoot profilin (Che a 2), hazelnut profilin (Cor a 2), saffron profilin (Cro s 2), Bahama grass profilin (Cyn d 12), olive profilin (Ole e 2), annual mercury profilin (Mer a 1), spreading pellitory profilin (Par j 3), timothy grass profilin (Phl p 12), date palm profilin (Pho d 2), English plantain profilin (Pla 12), Mesquite profilin (Pro j 2), European alder polcalcin (Aln g 4), hogweed polcalcin (Amb a 9), Japanese mugwort polcalcin (Art v 5), Japanese white birch polcalcin (Bet v 4), Brassica rapa polcalcin (Bra r 5), white goosefoot polcalcin (Che a 3), Bahama grass polcalcin (Cyn d 7), olive polcalcin (Ole e 3), spreading pellitory polcalcin (Par j 4), timothy grass polcalcin (Phi p 7), saltwort polcalcin (Sal k 7), and lilac polcalcin (Syr v 3).

Examples of contact allergens include, but are not limited to, allergens such as cosmetics, paints, clothing, latex (rubber), bedding, and detergents. In particular embodiments, latex allergens encompass latex nsLTP (Heb v 12).

In one embodiment, allergens have different reactivity with IgE and IgG4 depending on their fragments. Examples of such allergens include αs1-casein, αs2-casein, β-casein, x-casein, β-lactoglobulin, and brown shrimp tropomyosin.

In preferred embodiments, the allergen used as a probe is a polypeptide. The allergen fragment used as a probe is not particularly limited as long as it has a length that allows an antibody to bind thereto, and may have 3 to 50 amino acids in length, preferably 5 to 30 amino acids in length, more preferably 10 to 20 amino acids in length, and particularly preferably 12 to 18 amino acids in length. The allergen fragment may have a continuous amino acid sequence, or when the allergen contains a structural epitope comprising a discontinuous amino acid sequence, it may be a fragment containing the amino acid sequence of the structural epitope. When the fragment is a continuous fragment, it may comprise overlapping fragments in which adjacent fragments partially overlap each other. One example thereof is a set of overlapping fragments set forth in SEQ ID NOS: 2 to 21 of αs1-casein (SEQ ID NO: 1) shown in Table 1 below.

Table 1 Example of overlapping fragment set of αs1-casein

| Peptide Name | Sequence | Amino Acid Position | SEQ ID NO |
|---|---|---|---|
| Kaz1 | RPKHPIKHQGLPQEV | 1 to 15 | 2 |
| Kaz2 | LPQEVLNENLLRFFV | 11 to 25 | 3 |
| Kaz3 | LRFFVAPFPEVFGKE | 21 to 35 | 4 |
| Kaz4 | VFGKEKVNELSKDIG | 31 to 45 | 5 |
| Kaz5 | KVNELSKDIGSESTE | 36 to 50 | 6 |
| Kaz6 | SESTEDQAMEDIKQM | 46 to 60 | 7 |
| Kaz7 | DIKQMEAESISSSEE | 56 to 70 | 8 |
| Kaz8 | SSSEEIVPNSVEQKH | 66 to 80 | 9 |
| Kaz9 | VEQKHIQKEDVPSER | 76 to 90 | 10 |
| Kaz10 | VPSERYLGYLEQLLR | 86 to 100 | 11 |
| Kaz11 | EQLLRLKKYKVPQLE | 96 to 110 | 12 |
| Kaz12 | KVPQLEIVPNSAEER | 105 to 119 | 13 |
| Kaz13 | AEERLHSMKEGIHAQ | 116 to 130 | 14 |
| Kaz14 | GIHAQQKEPMIGVNQ | 126 to 140 | 15 |
| Kaz15 | IGVNQELAYFYPELF | 136 to 150 | 16 |
| Kaz16 | YPELFRQFYQLDAYP | 146 to 160 | 17 |
| Kaz17 | LDAYPSGAWYYVPLG | 156 to 170 | 18 |
| Kaz18 | YVPLGTQYTDAPSFS | 166 to 180 | 19 |
| Kaz19 | APSFSDIPNPIGSEN | 176 to 190 | 20 |
| Kaz20 | PIGSENSEKTTMPLW | 185 to 199 | 21 |

The number of probes used in the determination method of the present invention may vary depending on the length of the amino acid sequence of the allergen to be detected and the number of types of allergens, but it may be, for example, 2 to 10,000, 3 to 1,000, 9 to 512, or 18 to 144. A probe may be made up of a plurality of fragments of the

same allergen, or one or more fragments from two or more different allergens. In addition, a probe may be made up of a full-length allergen or an allergen fragment, or may contain both a full-length allergen and an allergen fragment.

A probe may be solid-phase immobilized. The solid-phase immobilization technique is not particularly limited, and any known technique can be used.

Specifically, for example, a method for directly adsorbing a probe to a measurement plate or chip, a method for binding a probe to a carrier such as a bead and fixing the probe-bound bead to a measurement plate or chip (WO2018/154814, WO2019/050017), and the like can be exemplified. Particular embodiments of immobilizing a probe to a chip are described in detail in the biochip section below.

A subject in the determination method of the present invention may or may not have an allergic disease. In one embodiment, the subject is a subject suspected of having an allergic disease. In another embodiment, the subject is a subject who has been diagnosed with an allergic disease. In some embodiments, the subject is a subject who is scheduled to undergo tests that may induce severe allergic symptoms such as anaphylaxis, such as challenge tests (e.g., oral food challenge test, inhalation challenge test, nasal provocation test, and ocular reaction) and intradermal tests for allergic diseases. In particular embodiments, the subject is a subject who is scheduled to undergo an oral food challenge test.

The biological sample used in the determination method of the present invention is not particularly limited as long as it can contain an antibody to be detected, and examples thereof include blood, plasma, and serum.

The biological sample may be two or more samples from the same subject which were collected at different times. Allergic diseases can be monitored by obtaining determination results for each of these samples and comparing them with each other or with predetermined reference values. For example, when the determination is whether the allergic disease is mild or severe, it can be further determined whether the allergic disease remains mild or severe, changed from mild to severe, or changed from severe to mild. For example, by collecting samples before and after the start of treatment or at two different time points after the start of treatment, it becomes possible to determine therapeutic effects.

Examples of types of antibodies identified by the determination method of the present invention include, but are not limited to, IgA1, IgA2, IgD, IgE, IgG1, IgG2a, IgG2b, IgG3, IgG4, and IgM. In one embodiment, the types of antibodies identified are IgE, IgG1, IgG2a, IgG2b, IgG3, and IgG4. In particular embodiments, the types of antibodies identified are IgE and IgG4.

The step of allowing the probe to come into contact with the biological sample can be performed by any known method that enables specific binding between an antigen and an antibody, or the method described in the Examples below. Specifically, for example, a probe immobilized on a substrate or carrier is loaded with a liquid containing a biological sample. A biological sample may be used as it is or after being diluted.

The step of detecting a reaction between a probe and a target antibody can be performed by any known method for detecting a reaction between an antigen and an antibody or the method described in the Examples below. Specifically, the step can be carried out by, for example, allowing a probe and a biological sample to come into contact with each other so as to react for a predetermined time under conditions that allow binding between a probe and an antibody, removing an antibody that is not bound to the probe, applying an antibody (secondary antibody) specific to a target antibody to a target antibody with a detectable label so as to remove an unbound secondary antibody, and then detecting the label. Examples of the label include, but are not limited to, a fluorescent substance, a luminescent substance, an enzyme (e.g., ALP or HRP), and a radioactive isotope. Examples of the method for detecting the label include, but are not limited to, spectrophotometry, absorptiometry, fluorometry, colorimetry, and autoradiography. Detection may be qualitative or quantitative. Before allowing the probe to come into contact with the biological sample, a blocking agent can be used to suppress the non-specific adsorption of an antibody. In an embodiment using a biochip in which a biotinylated probe is bound to a substrate or an immobilization carrier modified with avidin or a derivative thereof, the blocking agent may contain biotin. The signal-to-noise (SN) ratio can be enhanced by using a blocking agent containing biotin.

The step of identifying type of the antibody that reacted with the probe can be performed by, for example, using a secondary antibody specific to the desired type of antibody in the step of detecting the reaction between the probe and the antibody in the biological sample. The type of antibody may be identified one by one in each step of detecting the reaction between the probe and the antibody in the biological sample, or a plurality of types may be identified at the same time. Simultaneous determination of the plurality of types can be performed by, for example, changing the type of label for each type of secondary antibody. Specifically, for example, a secondary antibody specific to a type A antibody and a secondary antibody specific to a type B antibody can be labeled with fluorescent labels having different wavelengths. In addition, by using the same probe for two or more reaction regions and allowing a different secondary antibody to act on each reaction region, a plurality of types of antibodies can be detected substantially simultaneously while using the same label. A monoclonal antibody or a polyclonal antibody may be used as the secondary antibody. By using a monoclonal antibody, non-specific adsorption can be suppressed and the SN ratio can be increased.

The step of identifying type of the antibody that reacted with the probe may be performed simultaneously with the step of detecting the reaction between the probe and the target antibody, or may be performed separately.

Data on the reaction between the probe and the antibody may be quantitative or qualitative. In one embodiment, the data are quantitative data. Quantitative data may vary depending on reaction detection methods and the like, and include, but are not limited to, reaction intensity (e.g., absorbance, luminescence intensity, or radiation intensity).

The prediction model used in the determination method of the present invention is a model for obtaining an index that serves as a basis for allergic disease determination. The prediction model is a nonlinear model or a model with two or more explanatory variables and two or more terms, which is created using training data on the reaction with the antibody for at least the probes and training data on the type of the antibody bound. The number of explanatory variables is not particularly limited as long as it is two or more. Non-limiting examples of the number of explanatory variables may vary depending on the type of prediction model, but may include 2 to 100 (i.e., 2, 3, 4, 5, 6, 7, 8, 9, 10, 11, 12, 13, 14, 15, 16, 17, 18, 19, 20, 21, 22, 23, 24, 25, 26, 27, 28, 29, or 30 explanatory variables), 2 to 50 explanatory variables, 2 to 30 explanatory variables, 2 to 20 explanatory variables, 2 to 15 explanatory variables, 2 to 10 explanatory variables, 3 to 100 explanatory variables, 3 to 50 explanatory variables, 3 to 30 explanatory variables, 3 to 20 explanatory variables, 3 to 15 explanatory variables, 3 to 10 explanatory variables, 4 to 100 explanatory variables, 4 to 50 explanatory variables, 4 to 30 explanatory variables, 4 to 20 explanatory variables, 4 to 15 explanatory variables, 4 to 10 explanatory variables, 5 to 100 explanatory variables, 5 to 50 explanatory variables, 5 to 20 explanatory variables, 5 to 15 explanatory variables, and 5 to 10 explanatory variables. The nonlinear model may have less than two explanatory variables, for example, one explanatory variable.

Variables related to the reaction between the probe and the antibody (e.g., luminescence intensity, absorbance, and fluorescence intensity), variables related to the type of antibody bound (e.g., isotype, such as IgE or IgG4) as well as the subject's age, gender, medical history, family history, underlying disease, complications, genetic predisposition, total IgE level, and IgE dilution ratio (ratio for diluting a sample from a subject having a high total IgE level to the extent that specific IgE can be detected), test results for other allergens, and the like can be used as explanatory variables. Examples of other allergens include those listed above, as well as non-protein allergens such as formaldehyde, VOCs, metals, Japanese lacquer (urushiol), and iodine. Examples of allergen test results include specific IgE antibody test results by RAST, the CAP method, the MAST method, and the like, and skin test results such as prick test, scratch test, and intradermal test. As explanatory variables, unprocessed numerical values may be directly used, or numerical values subjected to preprocessing (variable transformation) such as logarithmic transformation may be used.

The prediction model can take the form of prediction formula, decision tree, or the like depending on the statistical method used to create the model. The prediction formula may include four arithmetic operations, a hinge function, etc., and the number of terms is not particularly limited as long as it is two or more terms. Non-limiting examples of the number of terms in the prediction formula may vary depending on the type of prediction model, but may include 2 to 150 terms (i.e., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 terms), 2 to 100 terms, 2 to 50 terms, 2 to 30 terms, 2 to 20 terms, 2 to 15 terms, 2 to 10 terms, 4 to 100 terms, 4 to 50 terms, 4 to 30 terms, 4 to 20 terms, 4 to 15 terms, 4 to 10 terms, 6 to 150 terms, 6 to 100 terms, 6 to 50 terms, 6 to 30 terms, 6 to 20 terms, 6 to 15 terms, 6 to 10 terms, 8 to 150 terms, 8 to 100 terms, 8 to 50 terms, 8 to 30 terms, 8 to 20 terms, 8 to 15 terms, 8 to 10 terms, 10 to 150 terms, 10 to 100 terms, 10 to 50 terms, 10 to 30 terms, 10 to 20 terms, and 10 to 15 terms. Note that the nonlinear model may have less than two terms, for example, one term.

The training data is data from a plurality of subjects whose presence or absence of an allergic disease to be determined is known for use in creating, evaluating, or the like of a prediction model. For example, when the determination method of the present invention determines whether an allergic reaction to allergen A is mild or severe, the training data include data from a subject with a known degree of allergic reaction to allergen A, and when the determination method of the present invention predicts the ASCA score for allergen A, the training data include data from a subject with a known ASCA score for allergen A. Various statistical methods are used based on such training data so as to create a model that can favorably distinguish between mildly affected subjects and severely affected subjects and a model that can predict ASCA scores that are closer to the actual ASCA scores.

Examples of statistical methods used for creating and evaluating a prediction model include, but are not limited to, the least-squares method, Ridge regression, LASSO regression, the CART method, and MARS method. The CART method, the MARS method, and the like are used to create a nonlinear model, and the least-squares method, Ridge regression, LASSO regression, and the like are used to create a linear model. The accuracy and generalizability of a prediction model can be improved through cross-validation (CV) or the like. In cross-validation, for example, training data are divided into a plurality of sets, some of them are input as training data and the rest are input as validation data into a prediction model to obtain prediction results, and it is confirmed whether there is any bias in the results. Examples of a specific method of cross-validation include leave-one-out cross-validation (LOOCV), 10-fold cross-validation, and generalized cross-validation. As long as similar results are obtained with a plurality of sets of training data and validation data, there is a high probability (generalizability) that similar results will be obtained even when the prediction model is applied to samples used for actual determination. The prediction model may be created manually or by machine learning. The prediction model can be evaluated by sensitivity, specificity, correlation coefficient (prediction rate), AUC in the ROC

curve, dot histogram, and the like.

In one embodiment, the prediction model has a correlation coefficient of 0.60 or more. The higher the correlation coefficient, the better. The correlation coefficient may be, but is not limited to, for example, 0.60 or more, 0.62 or more, 0.64 or more, 0.66 or more, 0.68 or more, 0.70 or more, 0.72 or more, 0.74 or more, 0.76 or more, 0.78 or more, 0.80 or more, 0.82 or more, 0.84 or more, 0.86 or more, 0.88 or more, 0.90 or more, 0.92 or more, 0.94 or more, 0.96 or more, or 0.98 or more.

In one embodiment, the prediction model has an AUC of 0.900 or more when the vertical axis of the ROC curve is sensitivity ranging from 0 to 1 and the horizontal axis is (1-specificity) ranging from 0 to 1. The higher the AUC, the better. The AUC may be, but is not limited to, for example, 0.900 or more, 0.905 or more, 0.910 or more, 0.915 or more, 0.920 or more, 0.925 or more, 0.930 or more, 0.935 or more, 0.940 or more, 0.945 or more, 0.950 or more, 0.955 or more, 0.960 or more, 0.965 or more, 0.970 or more, 0.975 or more, 0.980 or more, 0.985 or more, 0.990 or more, or 0.995 or more.

In addition to the data on the reaction between the probe and the antibody and the data on the type of the antibody bound, additional training data related to the determination of an allergic disease can be used to create a predictive model. Examples of such data include, but are not limited to, those described above for explanatory variables.

Examples of determination of allergic diseases by the determination method of the present invention include, but are not limited to, the determination of whether an allergic disease is mild or severe (e.g., whether a symptom due to being exposed to an allergen is mild or severe), prediction of the grade (severity), and prediction of the total ASCA score. The criteria for mild and severe allergic diseases may differ depending on the circumstances in which the determination results are used. For example, the criteria for mild allergic diseases include induced symptoms of grade 1, an ASCA score less than a predetermined value, and the degree to which medical measures are not necessary (the degree to which follow-up can be performed). The criteria for mild and severe allergic diseases can be determined appropriately by a physician depending on the situation. The grade is an index of the severity of anaphylaxis, and is determined based on the organ symptoms with the highest symptom grade based on the grade table below (Yanagida et al., Jpn. J. Pediatr. Allergy Clin. Immunol, 2014;28:201).

Table 2 Grade table

| | | Grade 1 (mild) | Grade 2 (moderate) | Grade 3 (severe) |
|---|---|---|---|---|
| Skin/ mucosal symptoms | Erythema/ Urticaria/ Wheals | Localized | Systemic | Systemic |
| | Pruritus | Mild pruritus (tolerable) | Intense pruritus (intolerable) | Intense pruritus (intolerable) |
| | Lip/eyelid swelling | Localized | Swelling over the face | Swelling over the face |
| Gastrointestinal symptoms | Oral and/or pharynx discomfort | Mouth and/or throat itching or discomfort | Sore throat | Sore throat |
| | Abdominal pain | Weak abdominal pain | Intense abdominal pain (tolerable) | Persistent and intense abdominal pain (intolerable) |
| | V omiting/ Diarrhea | Nausea/ Single vomiting/ Diarrhea | A plurality of episodes of vomiting/diarrhea | Repeated vomiting and fecal incontinence |

(continued)

| | | Grade 1 (mild) | Grade 2 (moderate) | Grade 3 (severe) |
|---|---|---|---|---|
| Respiratory symptoms | Cough, Runny nose, Nasal congestion, Sneezing | Intermittent cough, Nasal discharge, Nasal congestion, Sneezing | Intermittent cough | Persistent and intense cough, Barking cough |
| | Wheezing, Difficulty of breathing | - | Wheezing on auscultation, Mild shortness of breath | Obvious wheezing, Dyspnea, Cyanosis, Respiratory arrest, $SpO_2 \leq 92\%$, Tightness, Hoarseness, Difficulty swallowing |
| Cardiovascular symptoms | Pulse, Blood pressure | - | Tachycardia ( + 15 times/min) Slight decrease in blood pressure (less than 1 year old: < 80 mmHg; 1 to 10 years old: < [80 + (2 × age)] mmHg; 11 years old to adults: < 100 mmHg), Pale face | Arrhythmia, Hypotension (less than 1 year old: < 70 mmHg; 1 to 10 years old: < [70 + (2 × age)] mmHg; 11 years old to adults: < 90 mmHg), Severe bradycardia, Cardiac arrest |
| Neurological symptoms | State of consciousness | Low on energy | Drowsiness, Mild headache, Fear | Laziness, Restlessness, Incontinence, Unconsciousness |

Anaphylaxis Scoring Aichi (ASCA) is a scoring system that quantitatively assesses the severity of induced symptoms in an oral food challenge test. Induced symptoms are classified into five categories (i.e., respiratory, skin-mucosal, gastrointestinal, psycho-neurological, and cardiovascular symptoms), divided into subjective symptoms and objective findings, and arranged in order of severity so as to set organ scores (1 to 60), and then the highest scores for each organ in the series of challenge tests are summed so as to obtain a total score (maximum: 240) (see, for example, Hino et al., Arerugi. 2013;62(8):968-79, Sakai et al., Asia Pac Allergy. 2017;7(4):234-242). When it is possible to determine whether a symptom due to being exposed to an allergen is mild or severe before allergen exposure, it can contribute to determining whether or not to conduct tests involving allergen exposure (e.g., challenge tests such as oral food challenge test, inhalation challenge test, nasal mucosa challenge test, and ocular reaction, and intradermal test) and, in the case of performing the tests, whether or not to perform the tests on an outpatient basis or an inpatient basis.

Determination of an allergic disease based on the prediction result may vary depending on the prediction model and determination items. However, for example, the determination can be carried out by comparing the prediction result with a reference value. In the case of a prediction model based on a prediction formula, the prediction result (prediction value) obtained by the prediction formula can be compared with a preset reference value (e.g., a cutoff value) so as to make a positive or negative determination. In a case where the reference value is set such that when the prediction value is higher than the reference value, the probability of a severe case increases while when the prediction value is lower than the reference value, the probability of a mild case increases, if the prediction value is higher than the reference value, it can be determined as a severe case, and if it is lower than the reference value, it can be determined as a mild case. A cutoff value can be set by a known method such as the Youden index. In addition, in the case of an embodiment in which a prediction model directly outputs determination items (e.g., to determine a severe case/mild case using a decision tree or to predict the ASCA score using a prediction formula/a decision tree), the prediction result can also be used directly as the determination result.

2. Method for obtaining index for allergic disease determination

Another aspect of the present invention relates to a method for obtaining an index for allergic disease determination, comprising:

acquiring data on a reaction between two or more probes selected from an allergen and a fragment thereof and an

antibody contained in a biological sample collected from a subject, the data being obtained by allowing the two or more probes to come into contact with the biological sample, and data on the type of the antibody reacted; and inputting the data into a prediction model so as to obtain a prediction result as an index for allergic disease determination,

wherein the prediction model is a nonlinear model or a model with two or more explanatory variables and two or more terms, which is created using training data on the reaction with the antibody for at least the probes and training data on the type of the antibody bound (hereinafter sometimes referred to as the "index acquisition method of the present invention").

The index acquisition method of the present invention is the same as the determination method of the present invention, except that determining an allergic disease based on the prediction result is not essential. The prediction result obtained by the index acquisition method of the present invention can be used as an index for allergic disease determination. Specifically, for example, the presence or absence of a medical condition can be determined by comparing the prediction result with a reference value (such as a cutoff value) related to the medical condition to be determined. In addition, in the case of a prediction model in an embodiment in which determination items are directly output (e.g., determination of a severe case/a mild case using a decision tree and prediction of the ASCA score using a prediction formula/a decision tree), the prediction result can also be used directly as the determination result.

## 3. Allergic disease determination kit

Another aspect of the present invention relates to a kit for use in the determination method or index acquisition method of the present invention, which comprises two or more probes selected from an allergen and a fragment thereof (hereinafter sometimes referred to as the "kit of the present invention").

The probe included in the kit of the present invention is as described above for the determination method of the present invention. In the kit of the present invention, the probe may be solid-phase immobilized or modified for solid-phase immobilization. Examples of modification for solid-phase immobilization include the addition of avidin or a derivative thereof, biotin or a derivative thereof, alkyne, azide, a phenolic OH group, a hydroxyl group, an amino group, a carboxyl group, a photoreactive group, a His tag, and an antibody.

The kit of the present invention may further comprise a reagent for detecting a target antibody. Examples of such a reagent encompass, but are not limited to, a labeled secondary antibody that specifically binds to the target antibody, and optionally a reagent for detecting the label (e.g., a luminescent reagent or chromogenic reagent). The label added to the secondary antibody is as described above for the determination method of the present invention. The kit of the present invention may further include a standard sample, instructions showing the method of using the kit, etc. such as, for example, an instruction manual, information of sites including information regarding the use method (e.g., URL and 2D code), and a medium recording information regarding the use method, such as, for example, a flexible disc, CD, DVD, a Blu-ray disc, a memory card, or a USB memory.

## 4. Biochip for allergic disease determination

Another aspect of the present invention relates to a biochip for use in the determination method or index acquisition method of the present invention, wherein two or more probes selected from an allergen and a fragment thereof are immobilized on a substrate (hereinafter sometimes referred to as the "biochip of the present invention").

The probe to be immobilized on the substrate of the biochip of the present invention is as described above for the determination method of the present invention. The probe may be directly immobilized on the substrate or may be immobilized via a carrier (immobilization carrier). Hereinafter, the probe or the carrier on which the probe is immobilized may be referred to as the material to be immobilized.

The biochip substrate of the present invention is not particularly limited, as long as it does not excessively affect test samples or biochemical reactions. For example, a resin material, a glass material, or the like can be used. The type of the resin material is not limited, and for example, a thermosetting resin or a thermoplastic resin can be used. In particular, if a light transmissive resin material such as polypropylene, polycarbonate, acryl, polystyrene, polyethylene terephthalate, a cycloolefin polymer or a cycloolefin copolymer is used, favorable visible light transmittance can be ensured. The type of polypropylene is not limited, and for example, a random copolymer of homopolypropylene or polypropylene and polyethylene can be used. In addition, the acryl is not limited, and for example, a copolymer of methyl polymethacrylate or methyl methacrylate and a monomer such as methacrylic acid ester, acrylic acid ester or styrene can be used. Moreover, a light non-transmissive resin material can also be used. Examples of such a light non-transmissive resin material may include, but are not limited to, resin materials such as polypropylene, polycarbonate, acryl, polystyrene, polyethylene terephthalate, a cycloolefin polymer, and a cycloolefin copolymer, to which coloring agents for resins (e.g. masterbatch, etc.) are added. The light non-transmissive resin material preferably has high light blocking

properties, and the color of the light non-transmissive resin material is preferably black. The thickness of the substrate is not particularly limited. Since the substrate desirably has a certain extent of non-deforming property in the production process thereof, the thickness of the substrate is preferably 0.3 mm to 3.0 mm, more preferably 0.5 mm to 1.5 mm, and particularly preferably 0.7 mm to 1.0 mm.

In some aspect, a hydrophobic resin material is used in the substrate. In some aspect, the resin material is formed from a water-insoluble polymer. In some aspect, the resin material does not comprise dextran, polyethylene glycol, or a derivative thereof.

The hydrophilization treatment may be performed on the substrate. According to the hydrophilization treatment, non-specific adsorption on the substrate can be prevented, and also, a probe (or a probe-binding carrier) can be strongly immobilized on the substrate. The hydrophilization treatment may be performed on one or several portions on the surface of the substrate, or may also be performed on the entire surface of the substrate. When the hydrophilization treatment is performed on a portion(s) on the substrate surface, the treatment may be performed, for example, on the entire upper surface of the substrate, or on one or several portions on the upper surface of the substrate. The hydrophilization treatment is not particularly limited, but examples of the hydrophilization treatment may include: the coating of the surface with inorganic materials having hydrophilicity, such as silica, or surfactants; chemical hydrophilization treatments such as a plasma treatment, a UV-ozone treatment, a corona treatment, or a flame treatment; and the imparting of hydrophilicity by physically forming a fine nanostructure (WO2011/024947). When the substrate is made of a resin, it is more preferable to apply chemical hydrophilization treatments such as a plasma treatment, a UV-ozone treatment, a corona treatment, or a flame treatment, by which the chemical bonds of molecules on the surface of the resin can be cleaved, and functional groups having polarity, such as, for example, OH (hydroxyl groups), CO (carbonyl groups), COOH (carboxyl groups), methoxy groups, peroxide groups, or polar ether groups can be thereby generated depending on the type of the resin; and it is particularly preferable to apply a UV-ozone treatment.

In one embodiment, the substrate has a surface comprising a hydrophilic reaction area, and preferably, a resin-made surface comprising a hydrophilic reaction area. The reaction area means a region on the substrate, on which a material to be immobilized is immobilized and is allowed to react with a detection target substance. The reaction area may be either a portion of the surface of the substrate, or the entire surface of the substrate. When the reaction area is a portion of the surface of the substrate, the surface of the substrate other than the reaction area may be either a hydrophilic or hydrophobic surface. When the reaction area is a portion of the surface of the substrate, the reaction area may have various shapes such as, for example, a round, oval, or polygonal shape, or a combination thereof. The reaction area may be either continuous or discontinuous. For example, the reaction area may form a plurality of spots that are not contacted with one another. The reaction area may be surrounded by a boundary capable of retaining a liquid therein. Otherwise, the reaction area may be covered with functional groups having polarity generated as a result that bonds associated with carbon of the resin are cleaved and the cleaved sites bind with oxygen. Examples of such a functional group may include, but are not limited to, a hydroxyl group, a carbonyl group, a carboxyl group, a methoxy group, a peroxide group, and a polar ether group.

A coating layer for preventing non-specific adsorption and immobilizing a probe on the substrate can be established on the substrate. By immobilizing the material on the substrate via such a coating layer, non-specific adsorption can be prevented and detection sensitivity can be enhanced. The above-described coating layer is not particularly limited, as long as it enables the promotion of the immobilization of a material to be immobilized, and/or suppression of non-specific adsorption. The coating layer can be constituted, for example, with various polymers. Among such polymers, a water-soluble polymer is preferable. By using such a water-soluble polymer, the use of water or a non-aqueous solvent other than alcohol, which may degenerate the material to be immobilized, can be avoided. Hence, in the present invention, in order to prevent the degeneration of the material to be immobilized, a water-soluble polymer is preferably used. Moreover, the water-soluble polymer is advantageous in that it has an excellent effect of suppressing non-specific adsorption. Herein, the term "water-soluble" is used to mean that, for example, the solubility of the polymer in water (grams of the polymer dissolved in 100 g of water) is 5 or more. The number average molecular weight of the above-described polymer is not particularly limited, and it is generally approximately 3,500,000 to 5,000,000. By setting the molecular weight of the polymer to be approximately 500 to several hundreds of thousands, the number of crosslinks between polymers can be moderately maintained, and the reaction of the material to be immobilized with a substance to be subjected to the reaction with the material to be immobilized (e.g., a photocrosslinking agent, etc.) can be promoted.

Examples of the water-soluble polymer may include bipolar polymers such as phosphorylcholine-containing polymers, and nonionic polymers. Examples of the bipolar polymers may include polymers comprising, as a main component, 2-methacryloyloxy phosphorylcholine (MPC) (e.g. "LIPIDURE®" manufactured by NOF CORPORATION (LIPIDURE®-CR2001, LIPIDURE®-CM5206, etc.)). Examples of the nonionic polymers may include: polyalkylene glycol, such as polyethylene glycol (PEG) or polypropylene glycol; nonionic vinyl polymers comprising, as a constituent element(s), a single form of, or a mixture of monomer units, such as vinyl alcohol, methyl vinyl ether, vinyl pyrrolidone, vinyl oxazolidone, vinyl methyl oxazolidone, 2-vinyl pyridine, 4-vinyl pyridine, N-vinyl succinimide, N-vinyl formamide, N-vinyl-N-methyl formamide, N-vinyl acetamide, N-vinyl-N-methyl acetamide, 2-hydroxyethyl methacrylate, polyethylene glycol

methacrylate, polyethylene glycol acrylate, acrylamide, methacrylamide, N,N-dimethyl acrylamide, N-isopropyl acrylamide, diacetone acrylamide, methylol acrylamide, acryloyl morpholine, acryloyl pyrrolidine, acryloyl piperidine, styrene, chloromethyl styrene, bromomethyl styrene, vinyl acetate, methyl methacrylate, butyl acrylate, methylcyano acrylate, ethylcyano acrylate, n-propylcyano acrylate, isopropylcyano acrylate, n-butylcyano acrylate, isobutylcyano acrylate, tert-butylcyano acrylate, glycidyl methacrylate, ethylvinyl ether, n-propylvinyl ether, isopropylvinyl ether, n-butylvinyl ether, isobutylvinyl ether, or tert-butylvinyl ether; and natural polymers, such as gelatin, casein, collagen, gum Arabic, xanthan gum, tragacanth gum, guar gum, pullulan, pectin, sodium alginate, hyaluronic acid, chitosan, a chitin derivative, carrageenan, starches (carboxymethyl starch and aldehyde starch), dextrin, or cyclodextrin, and natural polymers including water-soluble cellulose derivatives, such as methyl cellulose, viscose, hydroxyethyl cellulose, hydroxyethylmethyl cellulose, carboxymethyl cellulose, or hydroxypropyl cellulose, but the examples of the water-soluble polymer are not limited thereto. Moreover, commercially available products of photocrosslinked-type water-soluble polymers formed on the basis of these polymers, for example, "BIOSURFINE-AWP" manufactured by Toyo Gosei Co., Ltd., which was produced on the basis of polyvinyl alcohol, or "LIPIDURE®-CR2001" manufactured by NOF CORPORATION, which was produced on the basis of 2-methacryloyloxy phosphorylcholine (MPC), etc., can be used. Among these polymers, polyethylene glycol polymers (e.g. a vinyl polymer of polyethylene glycol (meth)acrylate), polymers comprising MPC as a main component (e.g. LIPIDURE®-CR2001), and the like are preferable.

In order to enhance the adhesiveness between the coating layer and the substrate, a surface treatment can be performed on the substrate. The surface treatment is not particularly limited, and examples of the surface treatment may include the treatments of cleaving the chemical bonds of molecules on the resin surface of the substrate and generating hydrophilic functional groups such as OH (hydroxyl groups), CO (carbonyl groups) and COOH (carboxyl groups), methoxy groups, peroxide groups, or polar ether group depending on the type of the resin, including, for example, a plasma treatment, a UV-ozone treatment, a corona treatment, or a flame treatment.

The solid carrier is not particularly limited, and examples of the solid carrier may include microparticle carriers such as organic microparticles and inorganic microparticles. The microparticle carriers may also be magnetic microparticles. Examples of the magnetic microparticles may include, but are not limited to, beads having a uniform particle diameter, in which magnetizable materials such as $\gamma Fe_2O_3$ and $Fe_3O_4$ are uniformly covered with polymers having hydrophilicity (e.g., water-soluble polymers such as glycidyl methacrylate). Examples of such beads as commercially available products may include Dynabeads manufactured by Thermo Fisher Scientific, FG Beads manufactured by TAMAGAWA SEIKI Co., Ltd., Sera-Mag Magnetic Beads manufactured by GE HealthCare, and Magnosphere manufactured by JSR Life Sciences. The immobilization carrier may be subjected to various types of surface treatment useful for immobilizing the probe. Non-limiting examples of surface treatment include, but are not limited to, modification with reactive groups such as avidin or a derivative thereof, biotin or a derivative thereof, alkyne, azide, epoxy, a hydroxyl group, an amino group, a carboxyl group, a succinimide group, a tosyl group, protein A, and protein G.

The probe may also have modifications useful for immobilization to a substrate or immobilization carrier. Non-limiting examples of modification include, but are not limited to, modification with avidin or a derivative thereof, biotin or a derivative thereof, alkyne, azide, a phenolic OH group, a hydroxyl group, an amino group, a carboxyl group, a His tag, and an antibody. In particular embodiments, the probe is bound to biotin via a spacer, the immobilization carrier is avidinized, and the probe and the immobilization carrier are bound by biotin-avidin interaction. The spacer is not particularly limited, as long as it does not inhibit the interaction between a material to be immobilized (e.g., a peptide) and an antibody, and the interaction between a reactive group and a group reacting therewith, and is also non-cleavable at the use of the biochip of the present invention. Examples of the spacer may include a carbon chain and polyethylene glycol (PEG). The length of such a spacer is not limited, and may be, for example, 5 to 150 Å, 6 to 100 Å, 7 to 80 Å, 8 to 60 Å, 9 to 50 Å, etc. When the spacer is a carbon chain, it may have a length of, for example, C1-C20 linear alkyl, C3-C10 linear alkyl, C4-C8 linear alkyl, or in particular, C6 linear alkyl. When the spacer is PEG, it may have a length of, for example, a dimer to a 24-mer, a dimer to a dodecamer, a dimer to an octamer, or in particular, a dimer to a tetramer. By using the spacer, the interaction between the substance to be immobilized and the antibody is less likely to be inhibited due to steric hindrance or the like.

The biochip of the present invention can be produced by any known method or the method described in the Examples. For example, a biochip (biochip A) in which a probe-bound carrier is immobilized on a hydrophilized substrate can be produced as follows. Specifically, a substrate is produced by a step of performing a hydrophilization treatment on a substrate (hereinafter referred to as "Step 1-1 ") and a step of spotting, at least, a material to be immobilized, a photocrosslinking agent having at least two photoreactive groups in a single molecule, and a thickener and/or a surfactant on the substrate, and then applying a light to the substrate (hereinafter referred to as "Step 1-2"), thereby immobilizing the material to be immobilized on the substrate.

Hereinafter, the illustrative method for producing a biochip A will be described.

Step 1-1

The hydrophilization treatment of the substrate is not particularly limited, as long as it is a treatment of hydrophilizing the surface of the substrate. Examples of the hydrophilization treatment may include: a method comprising applying silica, a surfactant, and the like that are dissolved or suspended in a liquid onto a substrate according to spin-coating, coating, spraying, immersion, etc., and then drying it; a method of performing a chemical hydrophilization treatment such as a plasma treatment, a UV-ozone treatment, a corona treatment, or a flame treatment on a substrate; a method of transcribing a fine nanostructure on a substrate, or roughening a substrate with a liquid medicine to physically form a nanostructure on the substrate, so as to impart hydrophilicity to the substrate; and a method of coating a substrate with a hydrophilic polymer. A method of performing a chemical hydrophilization treatment such as a plasma treatment, a UV-ozone treatment, a corona treatment, or a flame treatment on a substrate, in which the chemical bonds of molecules on the resin surface are cleaved and thereby hydrophilic functional groups having polarity, such as OH (hydroxyl groups), CO (carbonyl groups), COOH (carboxyl groups), methoxy groups, peroxide groups, or polar ether groups can be generated depending on the type of the resin, is more preferable, and a method involving a UV-ozone treatment is particularly preferable.

A step of forming a boundary on a substrate to form a reaction area can be established. The method of forming a reaction area is not particularly limited. Examples of the method of forming a reaction area that can be used herein may include: a method of allowing a ring that has previously been formed with the above-described rubber composition or the above-described resin material to adhere to a substrate, using an adhesive or the like; and a method of subjecting a substrate constituted with the above-described resin material to various types of resin molding methods such as injection molding or vacuum forming, or to mechanical cutting or the like, so as to mold a ring. The adhesive used herein is not particularly limited, as long as it does not affect the test sample or the biochemical reaction. The adhesive can be appropriately selected from commercially available adhesives.

Step 1-2

Step 1-2 comprises a step of spotting, at least, a material to be immobilized, a photocrosslinking agent having at least two photoreactive groups in a single molecule, and a thickener and/or a surfactant, in the form of grid-like spots, on the above-described substrate, and a step of applying a light to the substrate.

The method for producing a biochip (biochip B) having a coating layer and a reaction area surrounded by a boundary capable of retaining a liquid therein may comprise:

producing a substrate by a step selected from a step of establishing a coating layer for preventing non-specific adsorption and immobilizing biological materials on the substrate, and a step of forming a boundary on the substrate to form a reaction area (hereinafter referred to as "Step 2-1"); and then, a step of spotting, at least, a material to be immobilized, a photocrosslinking agent having at least two photoreactive groups in a single molecule, and a thickener and/or a surfactant on the above-described substrate, and then applying a light to the substrate (hereinafter referred to as "Step 1-2").

Hereinafter, the illustrative method for producing a biochip B will be described.

Step 2-1

Step 2-1 comprises a step selected from a step of establishing a coating layer for preventing non-specific adsorption and immobilizing biological materials on the substrate and a step of forming a boundary on the substrate to form a reaction area. As long as Step 2-1 comprises the above-described steps, the order of performing these steps may be changed. In addition, Step 2-1 may comprise a step of performing a surface treatment on the substrate, before the step of establishing a coating layer.

The method of performing a surface treatment in the step of performing a surface treatment on the substrate is not particularly limited, as long as the method can enhance the adhesiveness between the substrate and the coating layer. For example, a known surface modification method such as a plasma treatment, a UV-ozone treatment, or a corona treatment can be applied.

A coating layer for preventing non-specific adsorption on a substrate and immobilizing biological materials on the substrate can be formed according to a known method such as the spin-coating, coating or spraying of a coating solution comprising a water-soluble polymer, or immersion of the substrate in a coating solution. For example, the coating solution can be prepared by dissolving a water-soluble polymer in a solvent. As such solvents, water, a lower alcohol that is mixed with water at any given ratio, and a mixture thereof can be used. As such lower alcohols, methanol, ethanol, and isopropanol are preferable. Among others, it is preferable to use a mixed solvent of ethanol and water.

The concentration of the polymer in the above-described coating solution is not particularly limited. The polymer concentration can be set at, for example, 0.0001 to 10 parts by mass, and preferably 0.001 to 1 part by mass. The

concentration of the photocrosslinking agent can be set at, for example, 1 to 20 parts by mass, and preferably 2 to 10 parts by mass, with respect to the above-described polymer.

The coating solution containing the above-described polymer preferably comprises a photocrosslinking agent having at least two photoreactive groups in a single molecule. In the present invention, the "photoreactive group" means a group that generates radicals as a result of light irradiation.

In the above-described photocrosslinking agent, photoreactive groups generate radicals as a result of light irradiation, so that covalent bonds can be formed with amino groups, carboxyl groups, carbon atoms constituting an organic compound, etc. Thereby, a coating solution comprising the above-described photocrosslinking agent is applied onto the substrate, followed by light irradiation, so that the substrate can be bound to the polymer via the photocrosslinking agent, and so that a polymer layer having a non-specific adsorption preventing effect can be formed on the substrate. Besides, in the present invention, without using the above-described photocrosslinking agent, or together with the above-described photocrosslinking agent, a photoreactive group and/or a group capable of covalently or coordinately binding to the substrate surface are introduced into the above-described polymer, so that the substrate can be bound to the polymer by utilizing the groups possessed by the polymer.

The water-soluble polymer and the photocrosslinking agent comprised in the coating layer of the present invention are known. The water-soluble polymer and the photocrosslinking agent can be produced by known production methods and also, are commercially available. The film thickness of the above-described coating layer is not particularly limited, but it is preferably 1 nm to 10 μm, more preferably 2 nm to 1 μm, and particularly preferably 10 nm to 100 nm.

In the present invention, as mentioned above, it is preferable to stabilize a coating layer by applying the coating layer to the substrate and then aging it under constant temperature and humidity conditions. The temperature is preferably 5°C to 40°C, and more preferably 20°C to 30°C. The humidity is preferably 40% to 80%, and more preferably 50% to 70%. The aging period is preferably 1 day to 1 month, and more preferably 3 days to 2 weeks.

The method of forming a boundary on the substrate is not particularly limited. A method of adhering a ring that has previously been formed with the above-described rubber composition or the above-described resin material onto the substrate, using an adhesive or the like, or a method of molding a substrate constituted with the above-described resin material into a ring according to various types of resin molding methods such as injection molding or vacuum forming, mechanical cutting, etc., can be applied. The adhesive is not particularly limited, as long as it does not affect the test sample or the biochemical reaction. Thus, an adhesive can be appropriately selected from commercially available adhesives.

Step 2-2

Step 2-2 comprises: a step of spotting, at least, a material to be immobilized, a photocrosslinking agent having at least two photoreactive groups in a single molecule, and a thickener and/or a surfactant, in the form of grid-like spots, on the above-described substrate; a step of applying a light to the substrate; and a step of removing unreacted components.

In Step 1-2 or 2-2, when the above-described material to be immobilized has previously been immobilized on an immobilization carrier, a known immobilization method can be applied. The immobilization method may be, for example, a method which comprises allowing the above-described magnetic microparticles used as immobilization carriers to react with the previously prepared materials to be immobilized in a solution such as an appropriately selected known buffer, and then recovering the magnetic microparticles on which the materials to be immobilized have been immobilized, but the immobilization method is not limited thereto. For example, the above-described magnetic microparticles may have previously been washed with a known buffer, or when the magnetic microparticles on which the materials to be immobilized have been immobilized are recovered, the recovered magnetic microparticles may be washed with a known buffer.

The photocrosslinking agent having at least two photoreactive groups in a single molecule, which is used in Step 1-2 or 2-2, is not particularly limited. Examples of the photoreactive groups possessed by the above-described photocrosslinking agent may include azide groups (-N3), acetyl groups, benzoyl groups and diazirine groups. In particular, azide groups are preferable because when the azide groups are irradiated with light, nitrogen molecules are dissociated and nitrogen radicals are generated, and these nitrogen radicals can bind not only to functional groups such as amino groups or carboxyl groups, but can also bind to carbon atoms constituting an organic compound, and can form covalent bonds with almost all organic matters. The photocrosslinking agent having such azide groups may be, for example, diazidostilbene.

The photocrosslinking agent is preferably a water-soluble agent. The "water solubility" of the photocrosslinking agent means that an aqueous solution comprising the photocrosslinking agent in a concentration of 0.5 mM or more, and preferably 2 mM or more, can be given.

The above-described material to be immobilized, or an immobilization carrier on which the material to be immobilized has been immobilized, and the above-described photocrosslinking agent are preferably dispersed or dissolved in a solution. The solution is not particularly limited, and a known buffer can be used. Examples of the buffer composition may include a PBS buffer, a HEPES buffer, a Tris buffer, and a MES buffer. When the material to be immobilized is directly used, a phosphate buffered saline is preferably used. When the material to be immobilized is immobilized on an immobilization carrier and is then used, a HEPES buffer is preferably used to prevent agglutination of the immobilization carrier. The solution, in which the material to be immobilized and the photocrosslinking agent are dispersed or dissolved, may be referred to as a stamp solution at times.

The concentration of the above-described material to be immobilized is not particularly limited. When the material to be immobilized is not immobilized on an immobilization carrier, the concentration of the material to be immobilized is preferably 0.05 mg/mL to 2 mg/mL, and more preferably 0.1 mg/mL to 1 mg/mL. When the material to be immobilized is immobilized on an immobilization carrier, the material to be immobilized is allowed to react with the immobilization carrier in the concentration of the material to be immobilized which is preferably 0.5 mg/mL to 50 mg/mL, and more preferably 1 mg/mL to 25 mg/mL. The immobilization carrier, on which the material to be immobilized has been immobilized, is used in a concentration of preferably 1 mg/mL to 10 mg/mL, and more preferably 2.5 mg/mL to 7.5 mg/mL.

The concentration of the above-described photocrosslinking agent is not particularly limited, but it is preferably 0.01 mg/mL to 1 g/mL, and more preferably 0.2 mg/mL to 0.2 g/mL.

The solution, in which the above-described material to be immobilized, or the immobilization carrier on which the material to be immobilized has been immobilized, and the above-described photocrosslinking agent are dispersed or dissolved, preferably may further comprise a thickener and/or a surfactant. By allowing the above-described solution to comprise a thickener, the size of a spot can be controlled when the solution is spotted on a substrate. More specifically, when a solution having a predetermined volume is spotted on a substrate, as the concentration of a thickener in the solution increases, the size of a spot (the area of a spot contacted with the substrate) tends to decrease. Moreover, by allowing the solution to comprise a surfactant, a phenomenon, whereby the material to be immobilized accumulates in the gas-liquid interface and the material to be immobilized is thereby localized in the margin of the spot, can be prevented. Also, such a surfactant contributes to the improvement of the affinity for the substrate. When a solution having a predetermined volume is spotted on a substrate, as the concentration of a surfactant in the solution increases, the size of a spot (the area of a spot in contact with the substrate) tends to increase. Accordingly, a stamp with any given size can be formed by controlling the concentration of the thickener and the surfactant.

The above-described thickener is not particularly limited, as long as it does not affect the test sample or the biochemical reaction. A commercially available thickener can be used. Examples of the thickener may include celluloses and derivatives thereof, polysaccharides, vinyl compounds, vinylidene compounds, polyglycol compounds, polyvinyl alcohol compounds, and polyalkylene oxide compounds. Specific examples of the thickener that can be used herein may include gellan gum, xanthan gum, curdlan, pullulan, a guar gum derivative, locust bean gum, carrageenan, pectin, glucan, tamarind gum, psyllium seed gum, dextran, glycerin, carboxymethyl cellulose, hydroxyethyl cellulose, hydroxymethylpropyl cellulose, lanolin, methyl cellulose, Vaseline, polyethylene glycol, polyvinyl alcohol, polyvinyl pyrrolidone, a carboxyl vinyl polymer, polyvinyl pyrrolidone, polyvinyl alcohol, a dextrin acid fatty ester, and an inulin acid fatty ester. It is preferable to use one or more selected from hydroxyethyl cellulose, methyl cellulose, polyvinyl pyrrolidone, and polyvinyl alcohol, and it is more preferable to use polyvinyl alcohol.

The concentration of the above-described thickener is not particularly limited. For example, when polyvinyl alcohol is used as a thickener, it is used in a concentration of preferably 0.01 part by weight to 1 part by weight, more preferably 0.02 parts by weight to 0.5 parts by weight, and particularly preferably 0.03 parts by weight to 0.3 parts by weight, with respect to 100 parts by weight of the above-described solution.

The above-described surfactant is not particularly limited, as long as it does not affect the test sample or the biochemical reaction. A commercially available nonionic surfactant can be used. Examples of the surfactant that can be used herein may include polyoxyethylene(10) octylphenyl ether [Triton X-100], polyoxyethylene(8) octylphenyl ether [Triton X-114], polyoxyethylene sorbitan monolaurate [Tween 20], polyoxyethylene sorbitan monooleate [Tween 80], polyoxyethylene(23) lauryl ether [Brij35], polyoxyethylene(20) lauryl ether [Brij58], Pluronic F-68, polyethylene glycol, and a mixture of two or more types thereof. It is preferable to use one or more types selected from Triton X-100, Tween 20, and Tween 80, and it is more preferable to use Tween 20.

The concentration of the above-described surfactant is not particularly limited. For example, when Tween 20 is used as a surfactant, it is used in a concentration of preferably 0.001 part by weight to 1 part by weight, more preferably 0.005 parts by weight to 0.5 parts by weight, and particularly preferably 0.01 part by weight to 0.3 parts by weight, with respect to 100 parts by weight of the above-described solution.

The viscosity of the stamp solution is not particularly limited, as long as a spot having a desired shape can be formed. The viscosity of the stamp solution at 25°C may be, for example, 0.4 mPa.s to 40 mPa.s, preferably 0.5 mPa.s to 10 mPa·s, more preferably 0.6 mPa·s to 4 mPa·s, particularly preferably 0.8 mPa·s to 2 mPa·s, and further particularly, approximately 1.3 mPa·s. On the other hand, the viscosity of the stamp solution at 25°C may be 0.6 mPa·s to 10 mPa·s, 0.8 mPa·s to 4 mPa·s, 1.0 mPa·s to 2 mPa.s, 1.12 mPa.s to 2 mPa.s, 1.13 mPa.s to 2 mPa.s, 1.14 mPa.s to 2 mPa.s,

1.15 mPa.s to 2 mPa.s, 1.16 mPa.s to 2 mPa.s, 1.17 mPa.s to 2 mPa.s, 1.18 mPa.s to 2 mPa.s, 1.19 mPa·s to 2 mPa·s, 1.2 mPa·s to 2 mPa·s, or the like.

The method of spotting a material to be immobilized on a substrate or the immobilization carrier on which the material to be immobilized has been immobilized and a photocrosslinking agent is not particularly limited. For example, a method of spotting the dispersed solution using a non-contact dispenser, a method of spotting the dispersed solution using a micropipette or the like, a spotting method involving a pin method, a spotting method involving a piezoelectric method, etc. can be used. The method of spotting the dispersed solution using a non-contact dispenser can apply a more precise amount of dispersed solution onto the substrate, than methods that need contact with the substrate, such as the spotting method involving a pin method such that variation in the amount of the material immobilized on each spot can be reduced, and thus, the spotting method using a non-contact dispenser is preferable. In a specific embodiment, there can be used a non-contact dispenser having a nozzle whose diameter is 100 to 2000 times, preferably 150 to 1000 times, more preferably 200 to 700 times, and particularly preferably 500 times greater than the average maximum diameter of the solid carrier. The spot diameter can be, for example, 400 μm to 800 μm, preferably 500 μm to 700 μm.

By spotting with a non-contact dispenser, the coefficient of variation of the amount (volume) of the material immobilized on each spot can be less than 11.8%. Therefore, one embodiment of the biochip of the present invention has a plurality of spots on which a material to be immobilized is immobilized, and the coefficient of variation of the volume of the material to be immobilized on each spot is less than 11.8%, for example, 11.5% or less, 11.0% or less, 10.5% or less, 10.0% or less, 9.5% or less, 9.0% or less, 8.5% or less, 8.0% or less, 7.5% or less, 7.0% or less, 6.5% or less, 6.0% or less, 5.5% or less, 5.0% 4.5% or less, 4.0% or less, 3.5% or less, or 3.0% or less. Since the volume of the material to be immobilized per spot is roughly proportional to the volume of a solid content per spot, the volume variation coefficient of the material to be immobilized per spot can be indicated with the volume variation coefficient of a solid content per spot. The volume of a solid content present in a spot can be measured, for example, by subjecting a 3D image obtained using a laser microscope (in particular, a shape analysis laser microscope, such as VK-X Series, manufactured by Keyence Corporation) to image analysis software (Multi-analysis Application, manufactured by Keyence Corporation, etc.).

Immobilization of a material to be immobilized, or an immobilization carrier on which a material to be immobilized has been immobilized, can be carried out by applying a light to a substrate, after application of a solution onto the substrate, preferably after the drying of the applied solution. The light is not particularly limited, as long as the used photoreactive groups can generate radicals under the light. In particular, when azide groups are used as such photoreactive groups, ultraviolet rays (for example, with a wavelength of 10 to 400 nm) are preferable. The irradiation time can be set at, for example, 10 seconds to 120 minutes, preferably 30 seconds to 60 minutes, and more preferably 1 minute to 30 minutes. Since the material to be immobilized is promptly immobilized by light irradiation, the irradiation time is almost equal to the time required for immobilization. The dose of the irradiated light is not particularly limited, but it is generally approximately 1 mW to 100 mW per $cm^2$. The photoreactive groups contained in the photocrosslinking agent (or when the polymer has photoreactive groups, the photoreactive groups contained in the polymer) generate radicals as a result of light irradiation. When the material to be immobilized is not immobilized on the immobilization carrier, the polymer layer can be bound to the material to be immobilized via the photocrosslinking agent. When the material to be immobilized is immobilized on the immobilization carrier, the polymer layer can be bound to the material to be immobilized and/or the immobilization carrier via the photocrosslinking agent.

After the desired material has been immobilized on a substrate as described above, the substrate is washed according to a known method, so that unreacted components and the like can be removed. However, such washing is not essential, and the immobilized material may be subjected to productization, while the stamp solution remains. Thus, a biochip, on which the desired material to be immobilized has been immobilized, can be obtained. When the washing is not carried out, the immobilized material, as well as stamp solution components such as a thickener and/or a surfactant, may adhere onto the spot on the biochip. Such a stamp solution residue can be removed by washing the biochip, as appropriate, before the use thereof.

## 5. Allergic Disease Determination System

Another aspect of the present invention relates to an allergic disease determination system, comprising:

a data acquisition unit for acquiring data on a reaction between two or more probes selected from an allergen and a fragment thereof and an antibody contained in a biological sample collected from a subject, the data being obtained by allowing the two or more probes to come into contact with the biological sample, and data on the type of the antibody reacted;
a data processing unit for inputting the data into a prediction model so as to obtain a prediction result;
a determination unit for determining an allergic disease based on the prediction result; and

an output unit for outputting a determination,
wherein the prediction model is a nonlinear model or a model with two or more explanatory variables and two or more terms, which is created using training data on the reaction with the antibody for at least the probes and training data on the type of the antibody bound (hereinafter sometimes referred to as the "system of the present invention").

The system of the present invention will be described below with reference to the drawings. The allergic disease determination system described below is intended to illustrate the present invention and not to limit the present invention. In addition, the configuration of the system of the present invention which is common to the determination method of the present invention, such as an allergic disease, a probe, data on a reaction between a probe and an antibody contained in a biological sample, data on type of antibody, a prediction model, etc., is as described above for the determination method of the present invention.

Figure 1 is a block diagram showing the functional configuration of an allergic disease determination system 100. The allergic disease determination system 100 has a data acquisition unit 101, a data processing unit 102, a determination unit 103, and an output unit 104.

The data acquisition unit 101 acquires and stores data on a reaction between a probe and a target antibody and data on type of an antibody reacted, and may optionally acquire and store other data for use in a prediction model. Data can be acquired from a detector that detects the reaction between the probe and the target antibody or an input device such as a keyboard or a touch panel, by reading digitized data through a communication interface such as a storage medium interface or a network interface, or by combining these acquisition means. Data may be stored on any writable data storage device, such as ROM, RAM, a magnetic disk, or a magneto-optical disks.

The data processing unit 102 accesses data stored in the data acquisition unit 101 to read out the information necessary for the prediction model, and input the information into the prediction model stored in the data processing unit 101, thereby obtaining a prediction result. The prediction result is sent to the determination unit 103 where the prediction result is linked with a determination result such that the determination result is sent to the output unit 104. Linking with the determination result can be carried out by various methods. For example, in the case of determining noncontinuous items in the prediction model (e.g., binary classification of severe case/mild case) by a prediction formula, linking with the determination result can be carried out by comparing the prediction result (prediction value) obtained by the prediction formula with a preset reference value (e.g., a cutoff value). Meanwhile, in the case of an embodiment in which the prediction model directly outputs determination items (e.g., to output noncontinuous items using a decision tree or to output continuous items (such as the ASCA score) using a prediction formula), the prediction result can also be used directly as the determination result. The output unit 104 outputs the determination result sent. The output format is not particularly limited. For example, display on various displays, output by a printer, transmission of electronic data, storage in a transportable storage medium, and the like are possible. It is also possible to configure the output unit 104 to store the determination result sent from the determination unit 103.

In addition to the items described above, the system of the present invention may have additional functions and configurations that are useful for allergic disease determination. For example, the system of the present invention can output relevant information related to the determination items together with the determination result (relevant information output function). For example, in a case where a determination item is whether a symptom due to being exposed to an allergen is mild or severe, the determination result may include not only information on a mild case/severe case but also, for example, relevant information on various challenge tests (e.g., oral food challenge test). Examples of such relevant information include information that challenge tests can be performed on an outpatient basis in the mild case and information that challenge tests should be performed on an inpatient basis or should not be performed in the severe case.

The prediction model may be updated appropriately by optimization with additional training data or the like. Update of the prediction model may be carried out manually by a user or automatically by the system that accesses the server or the like, in which the updated prediction model is stored, so as to obtain the updated prediction model (prediction model update function).

The reference value used for linking with the determination result can be changed. For example, the system of the present invention may be configured to allow the reference value to be selected from a plurality of candidates or to allow a user to input the reference value. Therefore, in the system of the present invention, for example, the determination unit 103 may have a function that allows a user to select the reference value from a plurality of candidates or a function that allows a user to input the reference value (reference value change function).

The system of the present invention may comprise a detection unit that detects the reaction between the probe and the target antibody. The detection unit may comprise: a detector that detects the reaction between the probe and the target antibody; and a transmitter that transmits data on the reaction between the probe and the target antibody, data on the type of antibody, etc. to the data acquisition unit.

The system of the present invention may also comprise: a user interface that allows the operation of the system and data input; a power supply; and a communication interface that makes it possible to take in external information via

the Internet, etc.

An example of data processing in the system of the present invention will be described based on the flowchart of Figure 2 in an embodiment in which the prediction result (prediction value) is compared with the reference value for linking the prediction result with the determination result. First, once the system is booted, the determination unit 103 presents the candidate reference values stored in the storage unit to a user (S1). Once the user selects a reference value, the selected reference value is stored in the storage unit of the determination unit 103. Next, the data acquisition unit 101 requires the user to input data, and the input data are stored in the storage unit of the data acquisition unit 101 (S2). Then, the data processing unit 102 reads data stored in the storage unit of the data acquisition unit 101 and the prediction model stored in the storage unit of the data processing unit 102 (S3) and inputs the data into the prediction model (S4), thereby obtaining a prediction value (S5). In the case of outputting the prediction value alone ("Yes" in S6, "No" in S7), the obtained prediction value is sent to the output unit 104, and thus, the prediction value is output (S10), leading to the end of processing. The obtained prediction value can be used directly as an index for allergic disease determination. In particular, in a case where the prediction value is continuous (e.g., calculation result by the prediction formula), it is possible to assist a judgment on the sense of level, etc. based on perception. In the case of outputting the prediction value and the determination result ("Yes" in S6, "Yes" in S7), the prediction value is sent to the determination unit 103 and compared with the reference value selected by the user stored in the storage unit of the determination unit 103 (S8). The prediction value and the determination result are sent to the output unit 104, and thus, the prediction value and the determination result are output (S9), leading to the end of processing. In the case of outputting the determination result alone ("No" in S6), the prediction value is sent to the determination unit 103 and compared with the reference value selected by the user (S11), the determination result alone is sent to the output unit 104, and thus, the determination result is output (S12), leading to the end of processing. The determination result may include information on the determination items (e.g., information on whether a symptom due to being exposed to an allergen is mild or severe) as well as accompanying information for the information (e.g., information regarding the embodiment of challenge tests as described above), etc.

The system of the present invention can also be implemented by a computer. Figure 3 is a block diagram showing an example of the hardware configuration of a computer 200 that implements the stand-alone system of the present invention. The computer 200 comprises a monitor 210, a communication interface 220, a CPU 230, a RAM 240, and a storage 250. The storage 250 stores a determination program 251 for executing various forms of processing of the system of the present invention, an acquired data 252 utilized for allergic disease determination, a prediction model 253, and a reference value 254. The CPU 230 reads out and executes codes of the determination program 251 stored in the storage 250. Therefore, the CPU 230 implements the functions of the data processing unit 102, the determination unit 103, and the like in the system of the present invention. The storage 250 implements the storage function of the data acquisition unit 101, the processing unit 102, and the determination unit 103 in the system of the present invention. The RAM 240 stores intermediate data, etc. during program execution. The monitor 210 implements the function of the output unit 104, the user interface, and the like. The communication interface 220 implements the function of the data acquisition unit 101, the output unit 104, and the like. In addition, OS and other software running on the computer may implement part or all of the processing according to the system of the present invention as the codes of the determination program 251 are read out.

The present invention also relates to a program for implementing the determination method and/or index acquisition method of the present invention by a computer or the like, and a storage medium storing the program (e.g., a flexible disk, a hard disk, a magnetic disk, a magneto-optical disc such as CD-ROM, MO, DVD, or BD, magnetic tape, or a semiconductor memory such as a flash memory).

Examples

Hereinafter, the present invention will be described in more detail in the following examples. However, these examples are not intended to limit the content of the present invention.

< Example 1: Production of biochip >

A polycarbonate sheet (manufactured by MITSUBISHI GAS CHEMICAL COMPANY, INC., MR58U, thickness: 0.8 mm) was irradiated with ultraviolet rays, using a UV-ozone irradiation device (manufactured by SEN LIGHTS Co., Ltd., SSP16-110, UV lamp: SUV110GS-36L) at an irradiation distance of 50 mm for 2 minutes. Subsequently, a polyethylene glycol monomethacrylate polymer (manufactured by Sanyu Chemical Co., Ltd.; the molecular weight of a polyethylene glycol moiety: 350) and 4,4'-diazidostilbene-2,2'-disulfonic acid (manufactured by TOKYO CHEMICAL INDUSTRY CO., LTD.; 98%; hereinafter referred to as "bisazide") were each dissolved in a 75% ethanol aqueous solution to result in 0.5 parts by weight and 0.025 parts by weight, respectively, thereby obtaining a coating solution of non-specific adsorption preventing agent. Using a spin-coater (manufactured by MIKASA, MS-A100), the above-described

UV-irradiated polycarbonate was coated with 15 mL of this coating solution under conditions of 800 rpm for 5 seconds, and 5000 rpm for 10 seconds. After completion of the coating, using a UV irradiation device (manufactured by UVP, CL-1000), the substrate was irradiated with ultraviolet rays at 120 mW/cm$^2$ for 10 minutes. A silicon rubber-made O ring (14 j) was adhered to the thus coated substrate, using an adhesive, so as to form a reaction area. The aging operation was performed at a temperature of 25°C at a humidity of 65% for 4 days, so as to obtain a biochip substrate.

Sodium chloride, potassium chloride, disodium hydrogen phosphate dodecahydrate, and potassium dihydrogen phosphate were each dissolved in ultrapure water to result in 0.8 parts by weight, 0.02 parts by weight, 0.29 parts by weight, and 0.02 parts by weight, respectively, so as to obtain a PBS solution. On the other hand, 4-(2-hydroxyethyl)-1-piperazineethanesulfonic acid was dissolved in ultrapure water to result in 0.59 parts by weight, so as to obtain a HEPES solution. Polyvinyl alcohol (manufactured by Wako Pure Chemical Industries, Ltd., 160-03055) and Tween 20 (manufactured by Sigma-Aldrich, P7949-100ML) were each dissolved in the HEPES solution to result in 0.1 part by weight and 0.05 parts by weight, respectively, so as to obtain a solvent for stamp solution. Materials to be immobilized formed by biotinylating the termini of partial peptides of αs1-casein, β-casein, β-lactoglobulin, and αs1-casein (20 overlapping fragments each consisting of 15 amino acid residues (SEQ ID NOS: 2 to 21)) with an aminohexyl (Ahx) group serving as a spacer were allowed to react with Streptavidin beads (particle size: 200 nm; manufactured by TAMAGAWA SEIKI Co., Ltd.) in the PBS solution at 4°C for 1.5 hours, were then purified, and were then dispersed in the solvent for stamp solution, so as to obtain a stamp solution. Figure 4 is a schematic diagram of partial peptides immobilized on beads. In addition, the structure of the biotinylated peptide is shown below.

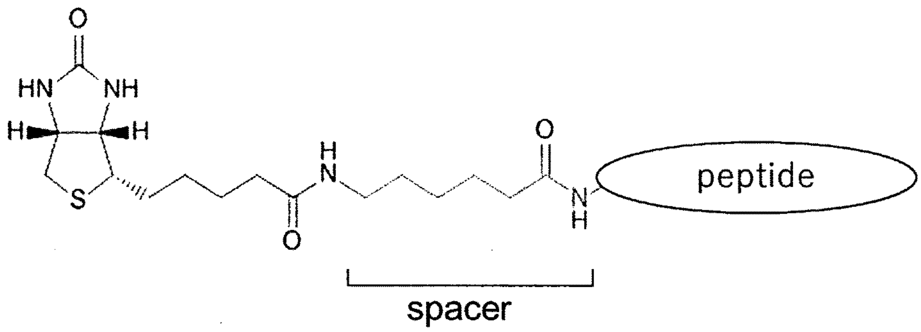


Bisazide was dissolved in ultrapure water to prepare a 10 mg/mL bisazide solution. The 10 mg/mL bisazide solution was diluted by 5 times. The bisazide solution was dissolved in the stamp solution to result in 4.8 parts by weight of the bisazide solution. Using a non-contact dispenser (manufactured by BioDot, Non-Contact Microdispensing System AD1520), the stamp solutions each containing one type of partial peptide were each spotted on the biochip substrate according to a lattice point-type multipoint dispensing spot method, to result in 81 spots in total (9 × 9 spots). After completion of the spotting, the spots were dried using a vacuum dryer at 0.09 MPa for 10 minutes. After completion of the drying, in order to immobilize the partial peptide-attached beads on the substrate using the photocrosslinking agent, the spots were irradiated with ultraviolet rays, using a UV irradiation device (manufactured by UVP, CL-1000) at 4 mW/cm$^2$ for 10 minutes, so as to obtain a biochip.

< Example 2: Measurement using chip >

Using the biochip produced in Example 1, the reaction between the IgE antibody and IgG4 antibody in the sera collected from the subjects and the partial peptide immobilized on the biochip was measured. The subject composition is shown in Table 3 below. Subjects having immunoCAP-specific IgE (casein) levels of less than 0.35 were classified as non-patients. Subjects having immunoCAP-specific IgE (casein) levels of 0.35 or more and ASCA scores of 2 or less and subjects having immunoCAP-specific IgE (casein) levels of 0.35 or more, total milk loads of 6 mL or more, and ASCA scores of 11 or less were classified as mild patients. Others were classified as severe patients.

Table 3 Subject composition

| Subject type | Number of subjects |
|---|---|
| Non-patient | 7 |
| Mild patient | 22 |
| Severe patient | 67 |

A protein-free blocking agent containing biotin (PVDF Blocking Reagent for Can Get Signal (manufactured by Toyobo Co., Ltd., NYPBR01), to which 0.02% by weight of biotin was added) was added to the reaction area of the biochip, and blocking was then carried out at room temperature for 1 hour. Thereafter, the biochip was washed with a TBS-T solution (137 mM sodium chloride, 2.68 mM potassium chloride, 25 mM tris-hydroxymethylaminomethane, pH 7.4, 0.1% by weight of Tween 20) three times. To the reaction area of the biochip, 130 L of 8-fold diluted serum was added as a test sample, and while shaking, it was reacted at room temperature for 8 minutes. The test sample was aspirated and was then washed with a TBS-T solution. After completion of the washing, 130 L of an ALP-labeled anti-human IgE monoclonal antibody (manufactured by Abcam, ab99805) or an ALP-labeled anti-human IgG4 monoclonal antibody (manufactured by Abcam, ab99822)) that had been 2000-fold diluted with Can Get Signal Immunoreaction Enhancer Solution 2 (manufactured by Toyobo Co., Ltd., NKB-301) was added to the resultant, and while shaking, the mixture was reacted at room temperature for 4 minutes. Thereafter, the antibody was aspirated and was then washed with TBS-T. To the resultant, 130 L of a luminescent reagent (Dynalight Substrate with Rapid Glow Enhancer, manufactured by Molecular Probes, 4475406) was added, and the thus obtained mixture was then shaken for 1 minute. Thereafter, using SpotSolver manufactured by Dynacom and Imaged manufactured by NHI, the number of pixels in the luminescent portion was counted, while a portion containing no spot was set as a background, so that the luminescence intensity was measured.

(1) Determination of mild/severe case

A prediction model was created using the following method. In each prediction model, "an" and "cn" are coefficients, "xn" is an explanatory variable, "h()" is a hinge function, and "n" is a natural number. Each coefficient and explanatory variable are independent for each prediction model and are not necessarily the same between different prediction models. For example, the coefficient a1 of prediction model 1-1 below is independent of the coefficient a1 of prediction model 1-2 and can take a different value. In addition, numerical values obtained by normal conversion of measured values (e.g., luminescence intensity of the probe) were used as explanatory variables.

< Prediction model 1-1 >

A prediction model assuming a binomial distribution by LASSO regression was constructed using the luminescence intensity of each probe and the subject's age, total IgE, and immunoCAP-specific IgE (casein, milk) as explanatory variables and whether the patient is a mild case or a severe case as an objective variable. Non-patients and mild patients in Table 3 were classified as mild cases, and severe patients were classified as severe cases (the same applies to prediction models 1-2 to 1-8). For the luminescence intensity of each probe, an interaction term between two elements (the product of two variables) was added to the explanatory variables. Leave one out cross-validation (LOOCV) was performed to obtain prediction model 1-1 represented by the following prediction formula at the position where the prediction error was minimized.

$$a1 + x1 \times a2 + x2 \times a3 + x22 \times x3 \times a4 + x4 \times x2 \times a5 + x3 \times x5 \times a6 + x3 \times x6 \times a7 + x21 \times x6 \times a8 + x7 \times x8 \times a9 + x7 \times x9 \times a10 + x7 \times x10 \times a11 + x11 \times x12 \times a12 + x11 \times x13 \times a13 + x11 \times x9 \times a14 + x11 \times x14 \times a15 + x2 \times x8 \times a16 + x15 \times x6 \times a17 + x15 \times x16 \times a18 + x17 \times x18 \times a19 + x13 \times x19 \times a20 + x20 \times x19 \times a21 + x19 \times x14 \times a22$$

In the prediction formula above, x1 is the immunoCAP-specific IgE (milk), x2 to x7, x11, x12, x15, x17, x21, and x22 are the luminescence intensities of different probes (IgE), and x8 to x10, x13, x14, x16, and x18 to x20 are the luminescence intensities of different probes (IgG4).

< Prediction model 1-2 >

Prediction model 1-2 represented by the following prediction formula was obtained by constructing a prediction model in the same manner as prediction model 1-1, except that only the luminescence intensity of each probe was used as an explanatory variable.

$$a1 + x1 \times a2 + x2 \times x3 \times a3 + x4 \times x5 \times a4 + x3 \times x6 \times a5 + x7 \times x8 \times a6 + x7 \times x9 \times a7 + x10 \times x11 \times a8 + x10 \times x12 \times a9 + x13 \times x11 \times a10 + x13 \times x14 \times a11 + x13 \times x12 \times a12 + x13 \times x15 \times a13 + x13 \times x16 \times a14 + x1 \times x17 \times a15 + x18 \times x9 \times a16 + x18 \times x19 \times a17 + x5 \times x20 \times a18 + x14 \times x15 \times a19 + x21 \times x15 \times a20 + x15 \times x16 \times a21$$

In the prediction formula above, x1 to x11, x13, and x18 are the luminescence intensities of different probes (IgE), and x12, x14 to x17, and x19 to x21 are the luminescence intensities of different probes (IgG4).

< Prediction model 1-3 >

Prediction model 1-3 represented by the following prediction formula was obtained by constructing a prediction model in the same manner as prediction model 1-1, except that the subject's age, total IgE, and immunoCAP-specific IgE (casein, milk) were used as explanatory variables.

$$a1 + x1 \times a2 + x2 \times a3$$

In the prediction formula above, x1 is the immunoCAP-specific IgE (milk), x2 is the immunoCAP-specific IgE (casein), x3 is the total IgE.

< Prediction model 1-4 >

A classification tree was constructed by the CART method using the same explanatory variables and objective variable as in prediction model 1-1. A large tree was constructed first, and then the average misclassification rate was calculated by 10-fold cross-validation and the tree was pruned at the minimum position, thereby obtaining prediction model 1-4 represented by the decision tree in Figure 5. In the figure, x1 is the immunoCAP-specific IgE (milk), and x2 to x4 are the luminescence intensities of different probes (IgE).

< Prediction model 1-5 >

Prediction model 1-5 represented by the decision tree in Figure 6 was obtained in the same procedure as for prediction model 1-4 using the same explanatory variables and objective variable as in prediction model 1-3. In the figure, x1 is the immunoCAP-specific IgE (milk).

< Prediction model 1-6 >

A binomial distribution was assumed by the MARS method using the same explanatory variables and objective variable as in prediction model 1-1, thereby constructing a model considering the interaction between two elements. The position with the maximum R-square value was obtained by 10-fold cross-validation, thereby obtaining prediction model 1-6 represented by the following prediction formula.

$$a1 + h(c1\text{-}x1) \times a2 + h(x1\text{-}c1) \times x2 \times a3 + h(X3\text{-}c2) \times h(c3\text{-}X4) \times a4 + h(c4\text{-}X4) \times a5 + h(c5\text{-}X5) \times a6$$

In the prediction formula above, x1 is the immunoCAP-specific IgE (milk), x2 is the luminescence intensity of the probe (IgG4), x3 is the total IgE, the immunoCAP-specific IgE (casein), and x4 to x5 are the luminescence intensities of different probes (IgE).

< Prediction model 1-7 >

Prediction model 1-7 represented by the following prediction formula was obtained in the same procedure as for prediction model 1-6 using the same explanatory variables and objective variable as in prediction model 1-2.

$$a1 + h(x1\text{-}c1) \times a2 + h(c1\text{-}x1) \times a3 + h(x2\text{-}c2) \times a4 + h(c3\text{-}x1) \times x2 \times a5 + h(x1\text{-}c4) \times a6$$

In the prediction formula above, x1 and x2 are the luminescence intensities of different probes (IgE).

< Prediction model 1-8 >

Prediction model 1-8 represented by the following prediction formula was obtained in the same procedure as for prediction model 1-6 using the same explanatory variables and objective variable as in prediction model 1-3.

$$a1 + h(c1\text{-}x1) \times a2 + h(x2\text{-}c2) \times a3 + h(x2\text{-}c3) \times h(x3\text{-}c4) \times a4$$

In the prediction formula above, x1 is the immunoCAP-specific IgE (milk), x2 is the immunoCAP-specific IgE (casein), and x3 is the total IgE.

ROC curves and dot histograms (Figures 7 to 10, 13 to 14, and 17 to 20) were created from the prediction values of each subject obtained by prediction models 1-1, 1-2, 1-4, 1-6, and 1-7. For comparison, ROC curves and dot histograms (Figures 11 and 12, 15 and 16, and 21 and 22) were also created from the subject's prediction values obtained from prediction models 1-3, 1-5, and 1-8. Table 4 summarizes the AUC (area under curve) of each prediction model.

Table 4 AUC based on prediction models 1-1 to 1-8

| Prediction model | 1-1 | 1-2 | 1-3 | 1-4 | 1-5 | 1-6 | 1-7 | 1-8 |
|---|---|---|---|---|---|---|---|---|
| AUC | 0.962 | 0.972 | 0.784 | 0.930 | 0.783 | 0.938 | 0.963 | 0.878 |

(2) Prediction of ASCA scores

< Prediction model 2-1 >

A prediction model assuming a normal distribution by LASSO regression was constructed using the luminescence intensity of each probe and the subject's age, total IgE, and immunoCAP-specific IgE (casein, milk), and the IgE dilution ratio as explanatory variables, and the subject's ASCA score as an objective variable. For the luminescence intensity of each probe, an interaction term between two elements (the product of two variables) was added to the explanatory variables. Leave one out cross-validation was performed to obtain prediction model 2-1 represented by the following prediction formula at the position where the prediction error was minimized.

$$a1 + x1 \times a2 + x2 \times a3 + x3 \times a4 + x4 \times a5 + x5 \times x3 \times a6 + x6 \times x3 \times a7 + x6 \times x4 \times a8 + x7 \times x8 \times a9 + x7 \times x9 \times a10 + x7 \times x10 \times a11 + x7 \times x11 \times a12 + x12 \times x8 \times a13 + x13 \times x14 \times a14 + x8 \times x9 \times a15 + x3 \times x9 \times a16 + x3 \times x15 \times a17 + x14 \times x16 \times a18 + x14 \times x17 \times a19 + x14 \times x18 \times a20 + x14 \times x19 \times a21 + x20 \times x21 \times a22 + x22 \times x15 \times a23 + x23 \times x15 \times a24 + x23 \times x24 \times a25 + x15 \times x25 \times a26 + x15 \times x26 \times a27 + x15 \times x24 \times a28$$

In the prediction formula above, x1 is the immunoCAP-specific IgE (milk), x2 is the IgE dilution ratio, x3 to x8, x12 to x14, and x20 are the luminescence intensities of different probes (IgE), and x9 to x11, x15 to x19, and x21 to x26 are the luminescence intensities of different probes (IgG4).

< Prediction model 2-2 >

Prediction model 2-2 represented by the following prediction formula was obtained by constructing a prediction model in the same manner as prediction model 1-1, except that the subject's age, total IgE, and immunoCAP-specific IgE (casein, milk) were used as explanatory variables.

$$a1 + x1 \times a2 + x2 \times a3 + x3 \times a4 + x4 \times a5$$

In the prediction formula above, x1 is the subject's age, x2 is the immunoCAP-specific IgE (milk), x3 is the immunoCAP-specific IgE (casein), and x4 is the total IgE.

< Prediction model 2-3 >

A regression tree was constructed by the CART method using the same explanatory variables and obj ective variable as in prediction model 2-1. A large tree was constructed first, and then the average misclassification rate was calculated by 10-fold cross-validation and the tree was pruned at the minimum position, thereby obtaining prediction model 2-3 represented by the decision tree in Figure 23. In the figure, x1 is the luminescence intensity of the probe (IgE) and x2 is the immunoCAP-specific IgE (milk).

< Prediction model 2-4 >

Prediction model 2-4 represented by the decision tree in Figure 24 was obtained in the same procedure as for prediction model 2-3 using the same explanatory variables and objective variable as in prediction model 2-2. In the figure, x1 is the immunoCAP-specific IgE (milk).

< Prediction model 2-5 >

A normal distribution was assumed by the MARS method using the same explanatory variables and objective variable as in prediction model 2-1, thereby constructing a model considering the interaction between two elements. The position with the maximum R-square value was obtained by generalized cross-validation, thereby obtaining prediction model 2-5 represented by the following prediction formula.

$$a1 + h(x1\text{-}c1) \times a2 + h(c2\text{-}x2) \times a3 + h(c2\text{-}x2) \times x3 \times a4 + h(x4\text{-}c3) \times a5 + h(x1\text{-}c1) \times x5 \times a6 + h(x1\text{-}c1) \times h(x6\text{-}c4) \times a7 + h(x1\text{-}c1) \times h(c4\text{-}x6) \times a8 + h(x6\text{-}c5) \times a9 + h(x1\text{-}c1) \times x7 \times a10 + x8 \times h(c2\text{-}x2) \times a11 + h(c6\text{-}x9) \times h(c3\text{-}x4) \times a12 + h(x10\text{-}c7) \times a13 + h(c8\text{-}x11) \times h(c5\text{-}x6) \times a14 + h(c9\text{-}x12) \times h(c3\text{-}x4) \times a15 + h(c5\text{-}x6) \times h(x13\text{-}c10) \times a16 + h(c11\text{-}x14) \times h(c3\text{-}x4) \times a17 + h(c12\text{-}x15) \times h(c3\text{-}x4) \times a18$$

In the prediction formula above, x1 is the immunoCAP-specific IgE (milk), x5 is the IgE dilution ratio, x2, x8 to x9, x12, x14, and x15 are the luminescence intensities of different probes (IgE), and x3, x4, x6, x7, x10, x11, and x13 are the luminescence intensities of different probes (IgG4).

< Prediction model 2-6 >

Prediction model 2-6 represented by the following prediction formula was obtained in the same procedure as for prediction model 1-6 using the same explanatory variables and objective variable as in prediction model 2-2.

$$a1xh(c1\text{-}x1) \times a2 + h(c2\text{-}x2) \times h(x1\text{-}c1) \times a3$$

In the prediction formula above, x1 is the immunoCAP-specific IgE (casein), and x2 is the immunoCAP-specific IgE (milk).

Scatter diagrams (Figures 25, 27, and 29) of prediction values (horizontal axis) and observed values (vertical axis) of each subject obtained by prediction models 2-1, 2-3, and 2-6 were created. For comparison, scatter diagrams of prediction values (horizontal axis) and observed values (vertical axis) of each subject obtained from prediction models 2-2, 2-4, and 2-6 were also created (Figures 26, 28, and 30). Table 5 summarizes the correlation coefficients of the prediction models.

Table 5 Correlation coefficients of prediction models 2-1 to 2-6

| Prediction model | 2-1 | 2-2 | 2-3 | 2-4 | 2-5 | 2-6 |
|---|---|---|---|---|---|---|
| Correlation coefficient | 0.827 | 0.498 | 0.644 | 0.533 | 0.901 | 0.576 |

From the above results, it was clarified that by adding the reactivity of IgE and IgG4 to allergens or their fragments as parameters, it is possible to distinguish between severe and mild cases and predict the ASCA score more accurately.

Reference Signs List

100: Allergic disease determination system
101: Data acquisition unit
102: Data processing unit
103: Determination unit
104: Output unit
200: Computer
210: Monitor
220: Communication interface
230: CPU
240: RAM
250: Storage
251: Determination program
252: Acquired data
253: Prediction model
254: Reference value

## Claims

1. A method of determining an allergic disease, comprising:

    a step of allowing two or more probes selected from an allergen and a fragment thereof to come into contact with a biological sample collected from a subject;
    a step of detecting a reaction between the probe and an antibody contained in the biological sample;
    a step of identifying type of the antibody that reacted with the probe;
    a step of inputting data on the reaction with the antibody for each of the two or more probes and data on the type of the antibody bound into a prediction model so as to obtain a prediction result; and
    a step of determining an allergic disease based on the prediction result,

    wherein the prediction model is a nonlinear model or a model with two or more explanatory variables and two or more terms, which is created using at least training data on the reaction with the antibody for the probes and training data on the type of the antibody bound.

2. The method according to claim 1, wherein determining the allergic disease is selected from determining whether the allergic disease is mild or severe and predicting a total ASCA score.

3. A method of obtaining an index for allergic disease determination, comprising:

    a step of acquiring data on a reaction between two or more probes selected from an allergen and a fragment thereof and an antibody contained in a biological sample collected from a subject, and data on the type of the antibody reacted, the data being obtained by allowing the two or more probes to come into contact with the biological sample; and
    a step of inputting the data into a prediction model so as to obtain a prediction result,

    wherein the prediction model is a nonlinear model or a model with two or more explanatory variables and two or

more terms, which is created using at least training data on the reaction with the antibody for the probes and training data on the type of the antibody bound.

**4.** The method according to any one of claims 1 to 3, wherein the type of the antibody is selected from IgE and IgG4.

**5.** The method according to any one of claims 1 to 4, wherein the probe comprises an overlapping fragment of the allergen.

**6.** The method according to any one of claims 1 to 5, wherein the probe is solid-phase immobilized.

**7.** The method according to any one of claims 1 to 6, wherein the data on the reaction with the antibody are of intensity of reaction with the antibody.

**8.** The method according to any one of claims 1 to 7, wherein additional training data are used for creating the prediction model.

**9.** The method according to any one of claims 1 to 8, wherein the allergen is selected from a food allergen, an inhalant allergen, and a contact allergen.

**10.** The method according to any one of claims 1 to 9, wherein the food allergen is selected from casein, lactalbumin, lactoglobulin, ovomucoid, ovalbumin, conalbumin, lysozyme, gliadin, an amylase inhibitor, albumin, globulin, gluten, and tropomyosin.

**11.** The method according to any one of claims 1 to 10, which is used in a monitoring of the allergic disease, wherein the biological sample collected from the subject comprises two or more biological samples collected at different times, and wherein the step of determining an allergic disease comprises comparing prediction results obtained for the two or more biological samples with each other.

**12.** The method according to any one of claims 1 to 11, wherein the allergic disease is food allergy.

**13.** A kit for use in the method according to any one of claims 1 to 12, comprising two or more probes selected from an allergen and a fragment thereof.

**14.** A biochip for use in the method of any one of claims 1 to 12, wherein the two or more probes selected from an allergen and a fragment thereof are immobilized on a substrate.

**15.** An allergic disease determination system, comprising:

a data acquisition unit that acquires data on a reaction between two or more probes selected from an allergen and a fragment thereof and an antibody contained in a biological sample collected from a subject, and data on the type of the antibody reacted, the data being obtained by allowing the two or more probes to come into contact with the biological sample;
a data processing unit that inputs the data into a prediction model so as to obtain a prediction result;
a determination unit that determines an allergic disease based on the prediction result; and
an output unit that outputs a determination,

wherein the prediction model is a nonlinear model or a model with two or more explanatory variables and two or more terms, which is created using at least training data on the reaction with the antibody for the probes and training data on the type of the antibody bound.

**16.** A program for causing a computer to execute the method according to any one of claims 1 to 12.

**17.** A storage medium storing the program according to claim 16.

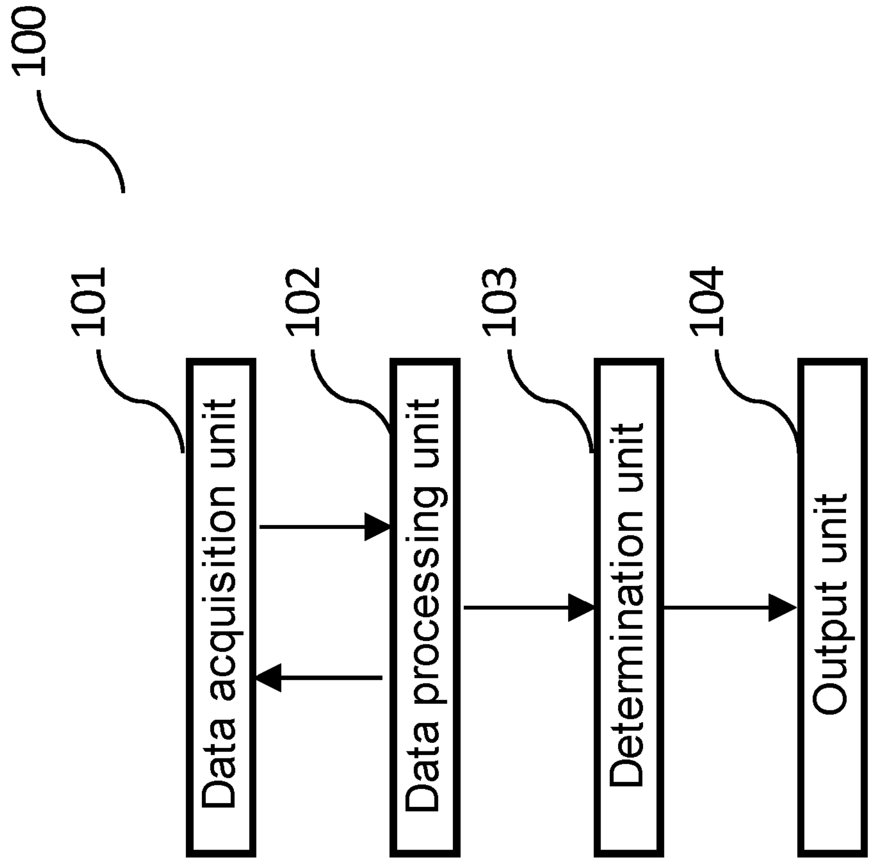
100
101
102
103
104
Data acquisition unit
Data processing unit
Determination unit
Output unit

FIG. 1

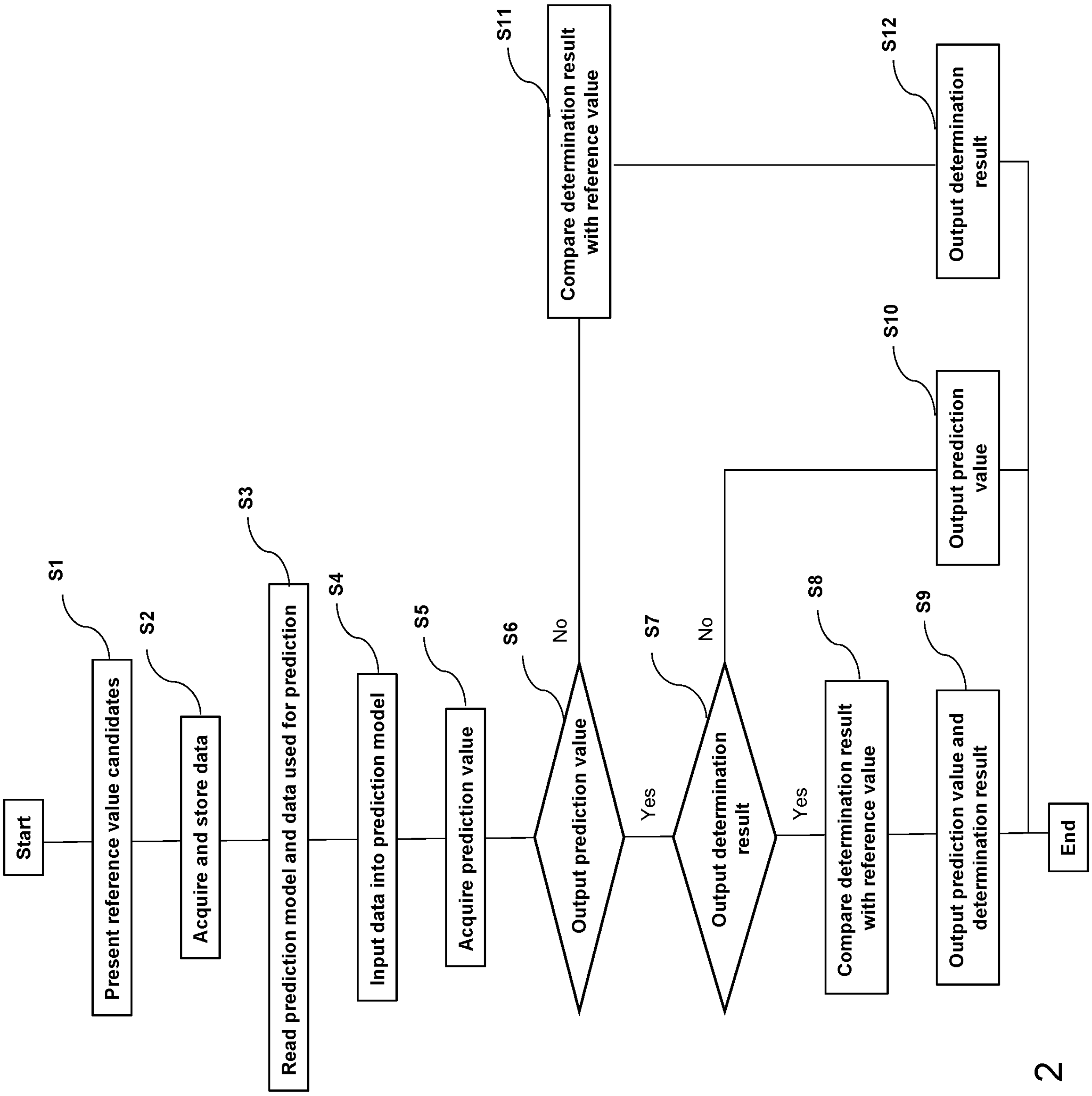
Start
S1
Present reference value candidates
S2
Acquire and store data
S3
Read prediction model and data used for prediction
S4
Input data into prediction model
S5
Acquire prediction value
S6
Output prediction value
No
Yes
S7
Output determination result
No
Yes
S8
Compare determination result with reference value
S9
Output prediction value and determination result
S10
Output prediction value
S11
Compare determination result with reference value
S12
Output determination result
End

FIG. 2

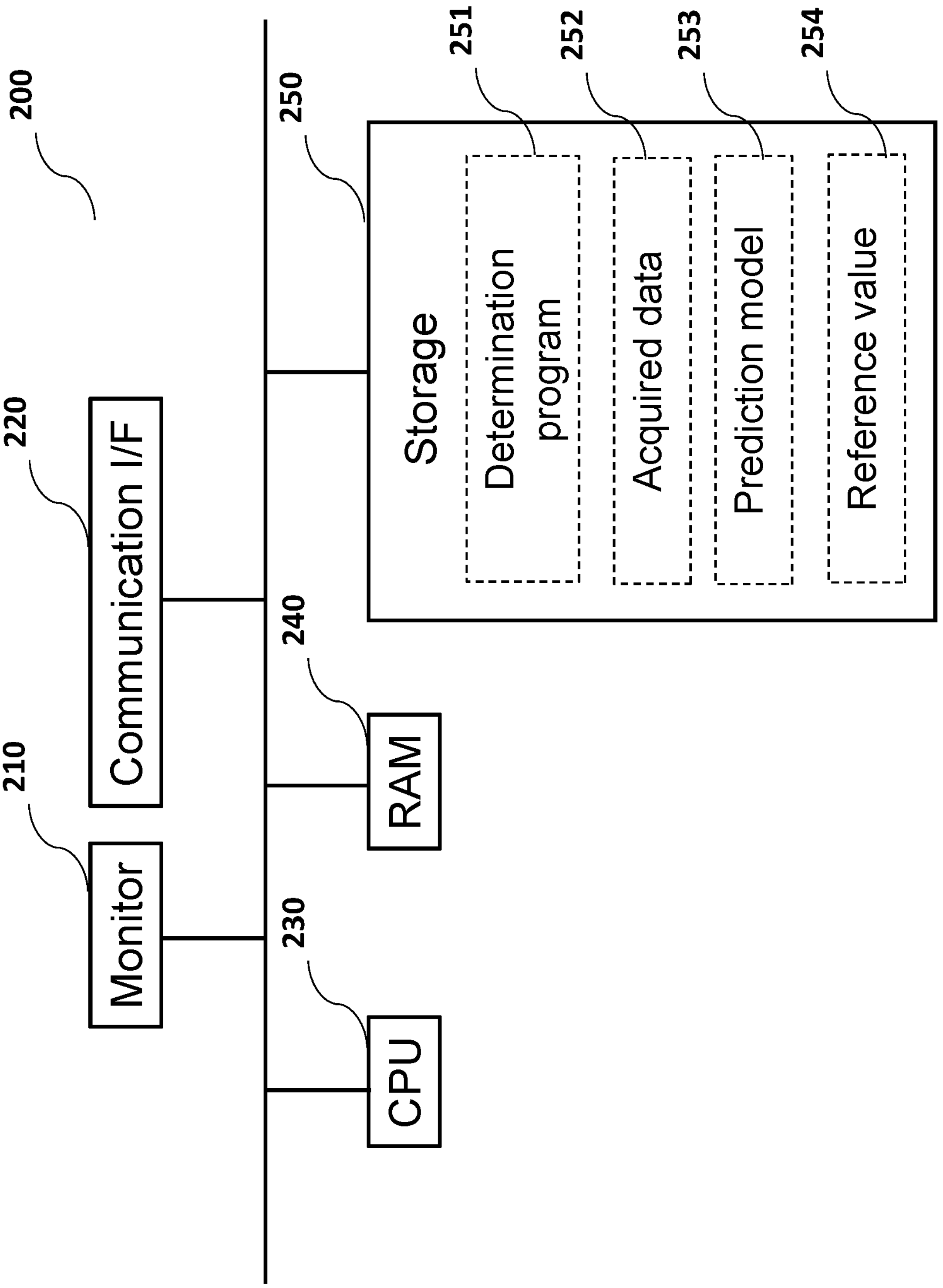
200
210
Monitor
220
Communication I/F
230
CPU
240
RAM
250
Storage
251
Determination program
252
Acquired data
253
Prediction model
254
Reference value

FIG. 3

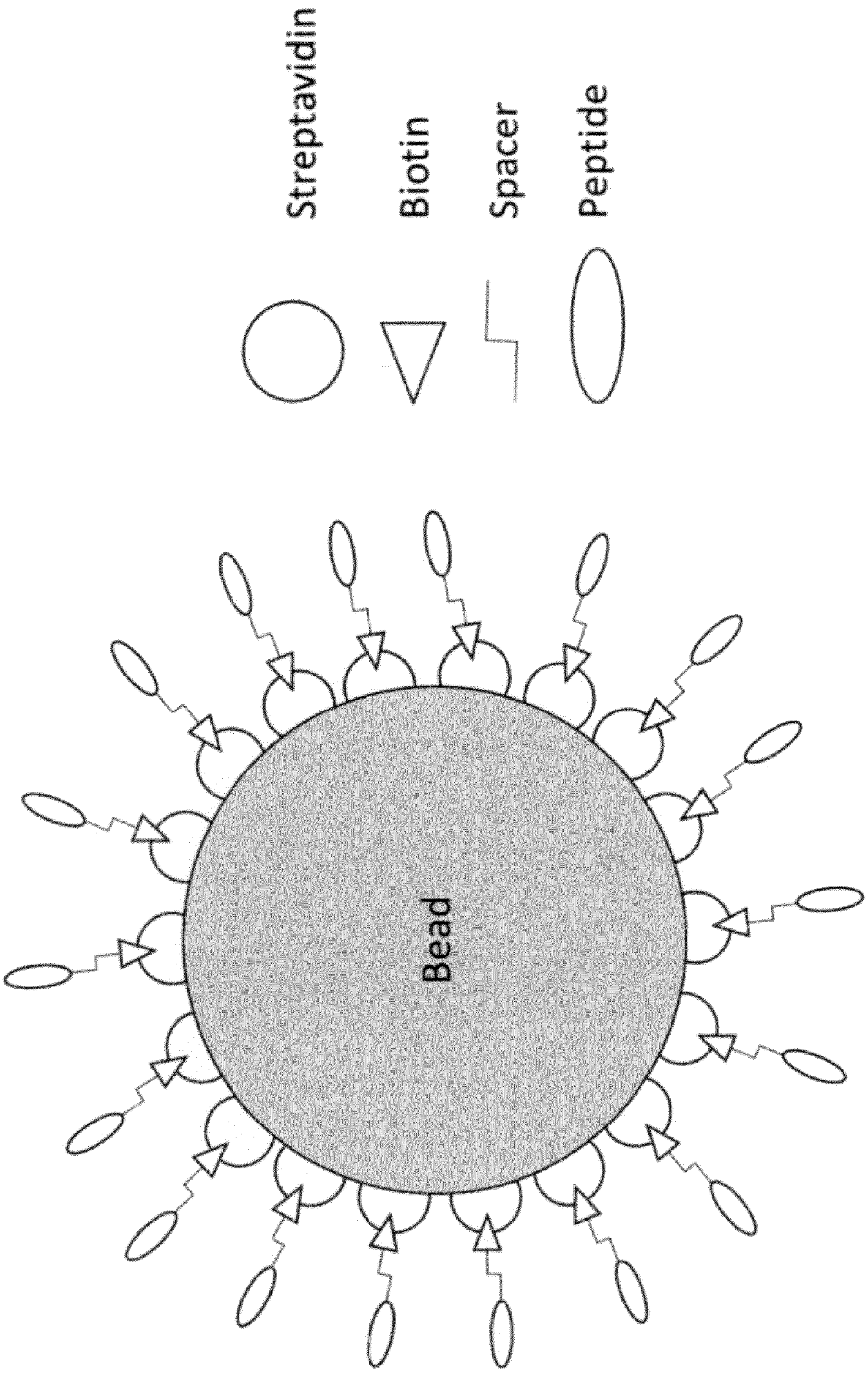


FIG. 4

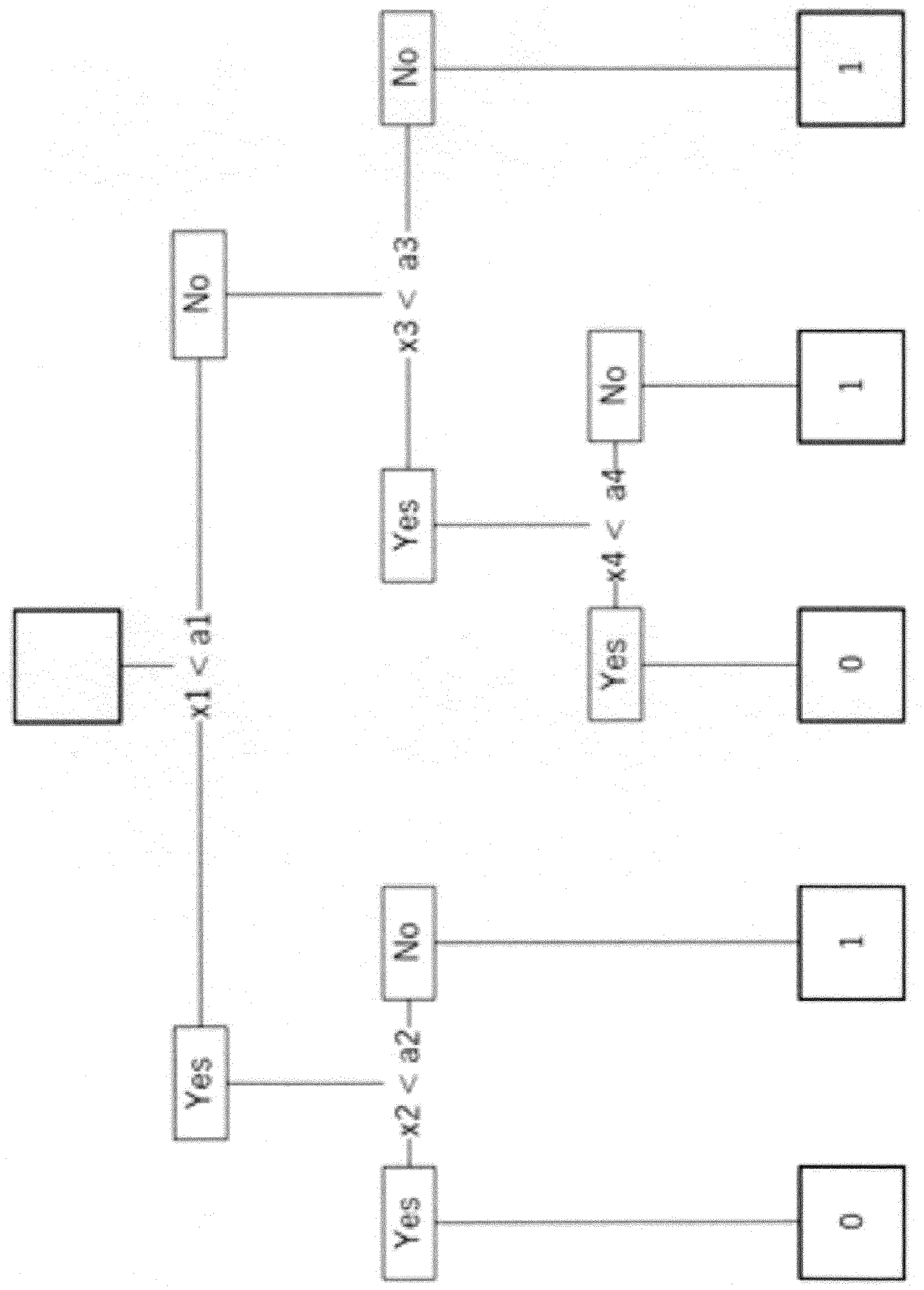


FIG. 5

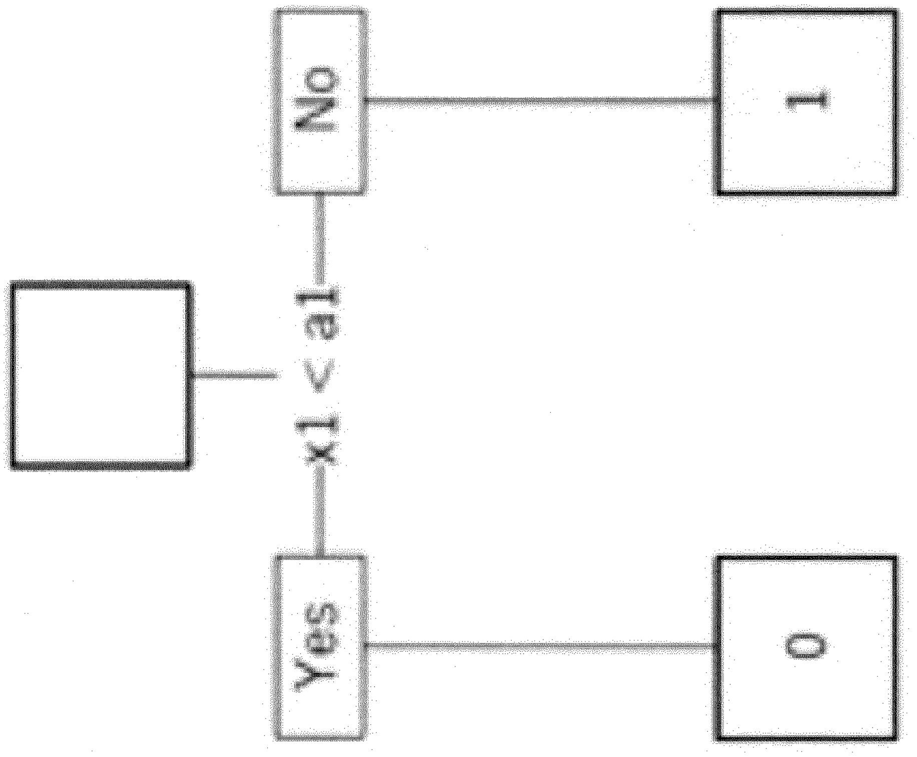


FIG. 6

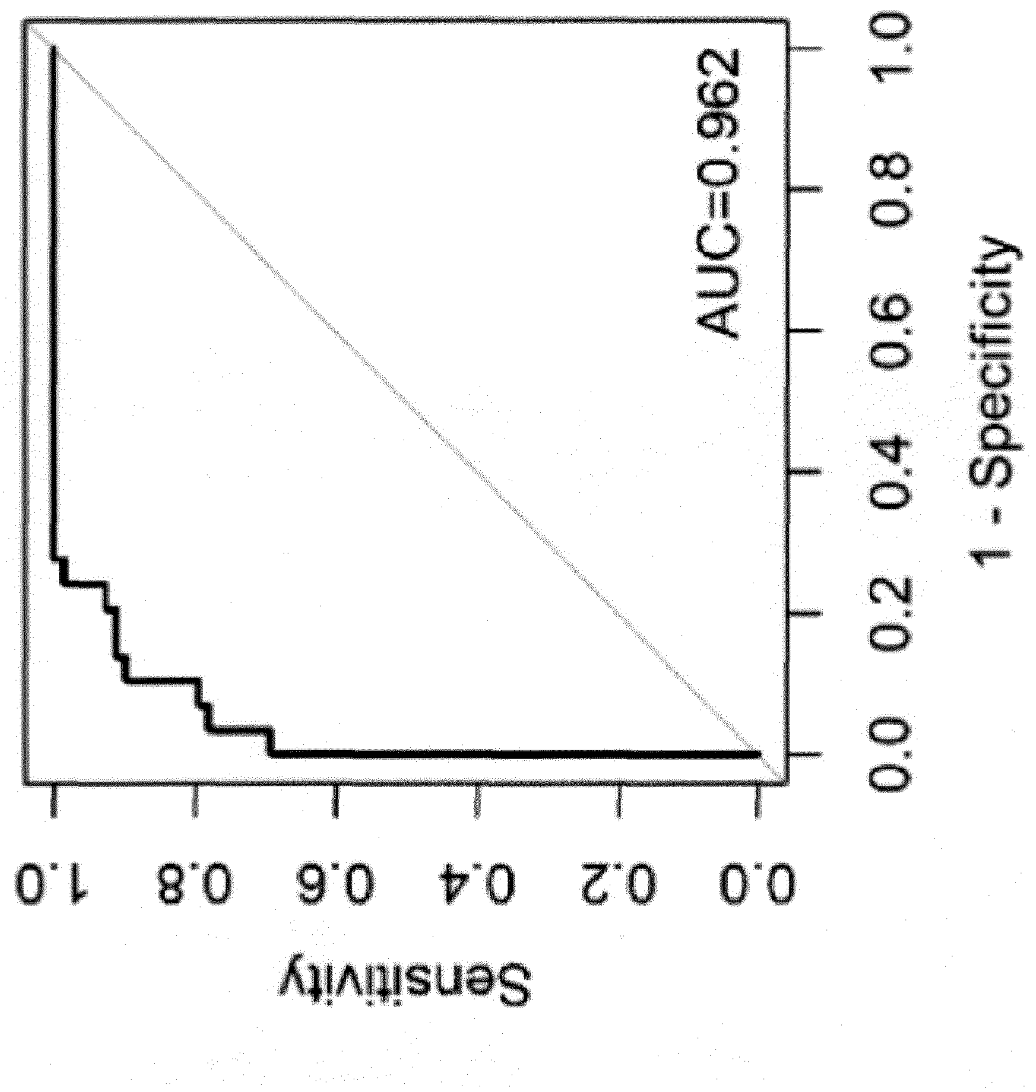
AUC=0.962
Sensitivity
1.0
0.8
0.6
0.4
0.2
0.0
1 - Specificity
0.0
0.2
0.4
0.6
0.8
1.0

FIG. 7

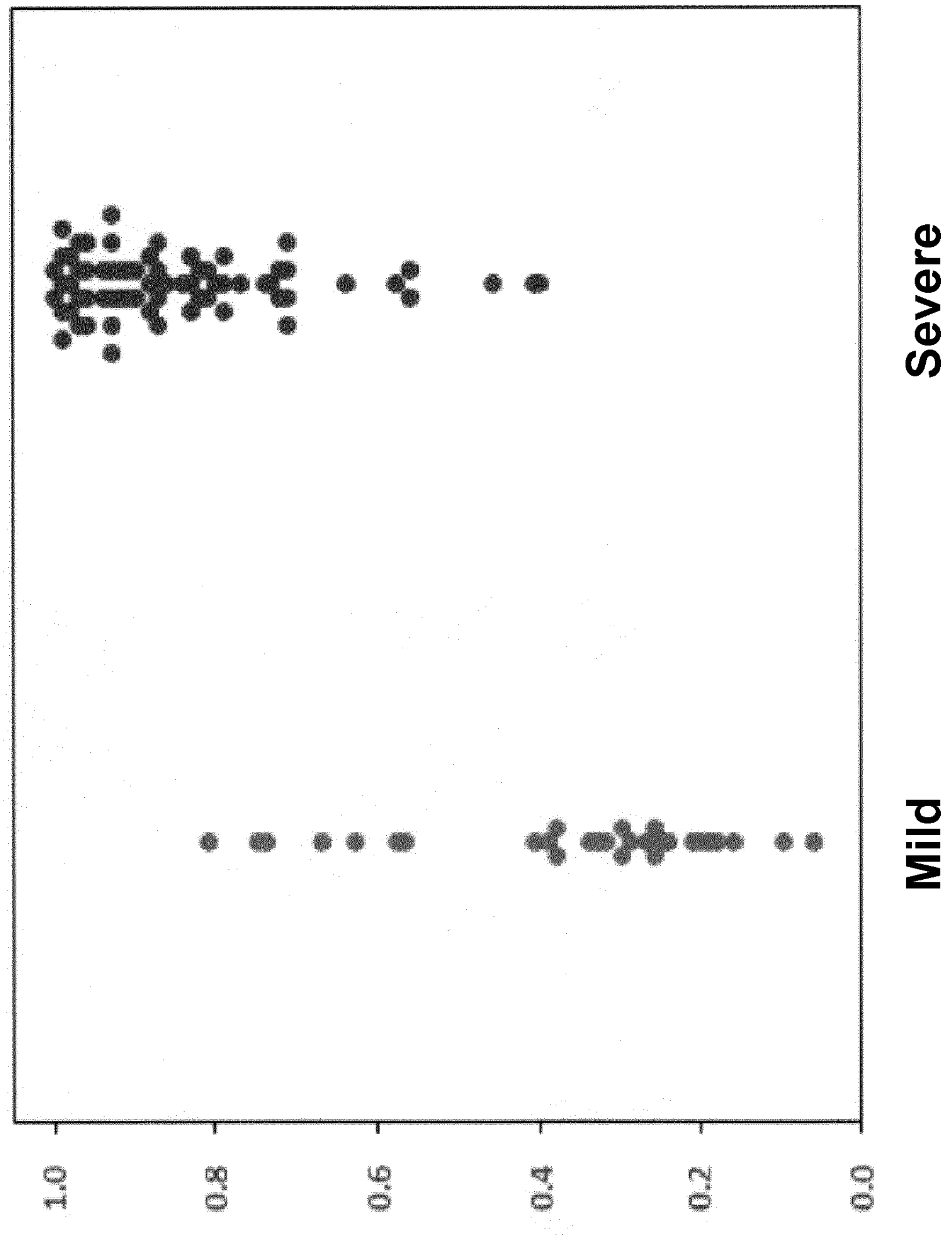


FIG. 8

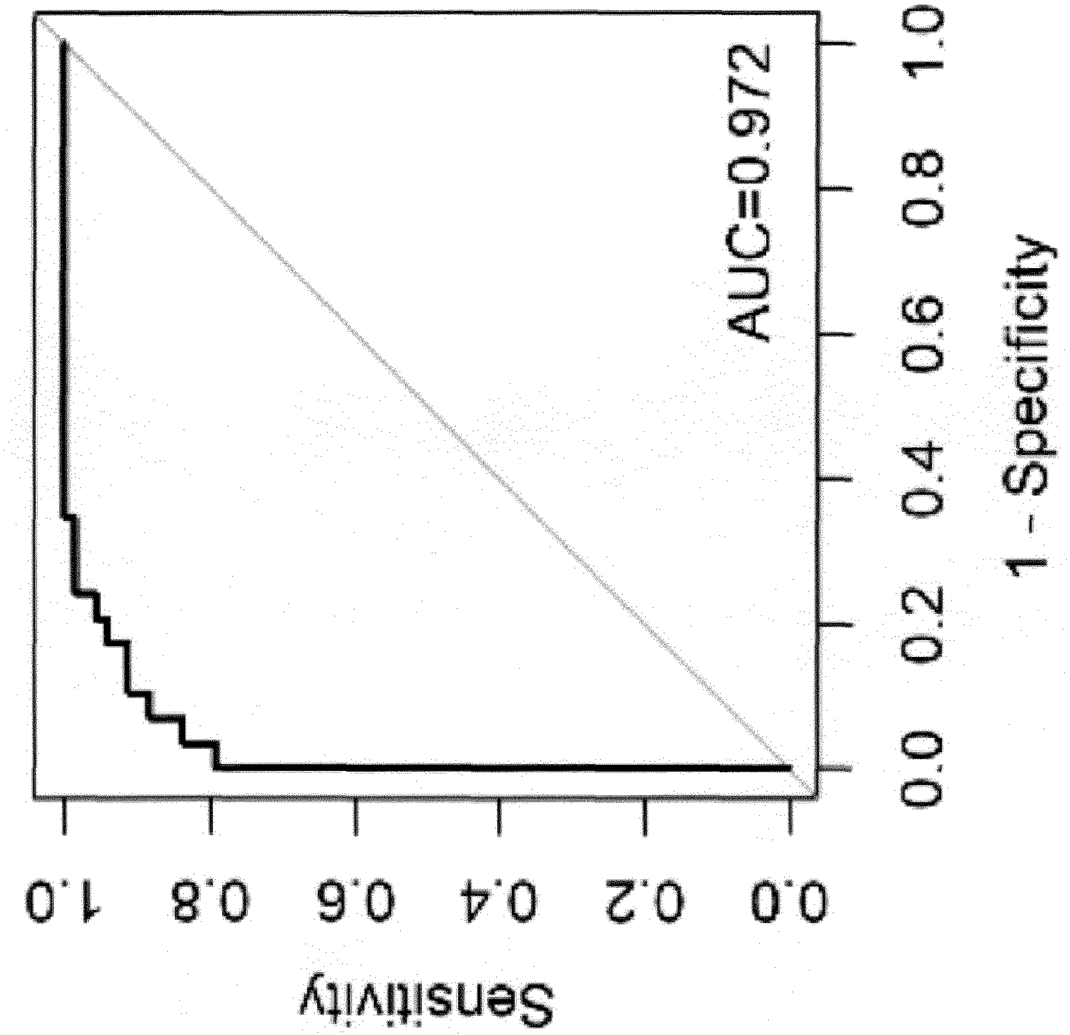
AUC=0.972
1 - Specificity
0.0
0.2
0.4
0.6
0.8
1.0
Sensitivity
0.0
0.2
0.4
0.6
0.8
1.0

FIG. 9

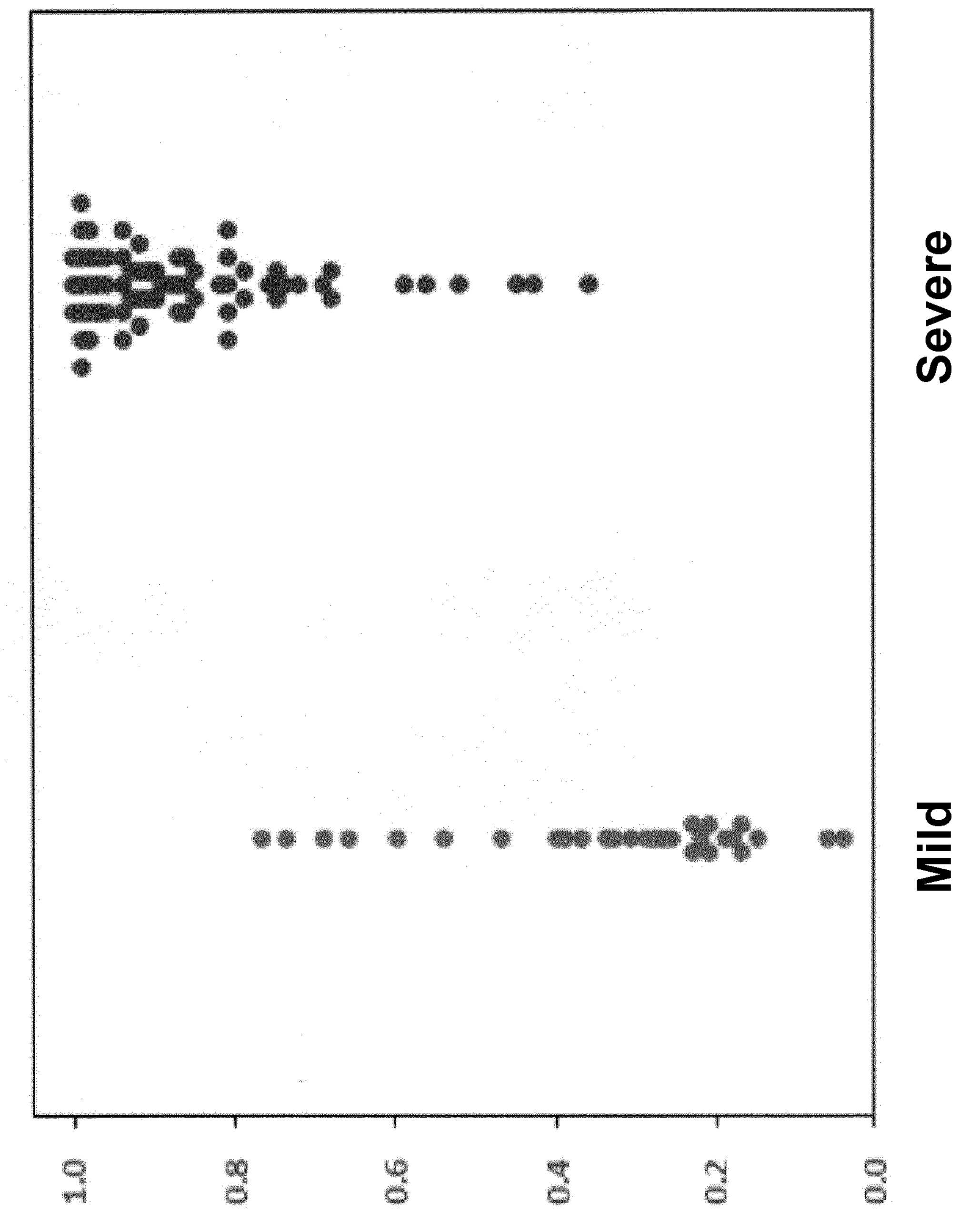


FIG. 10

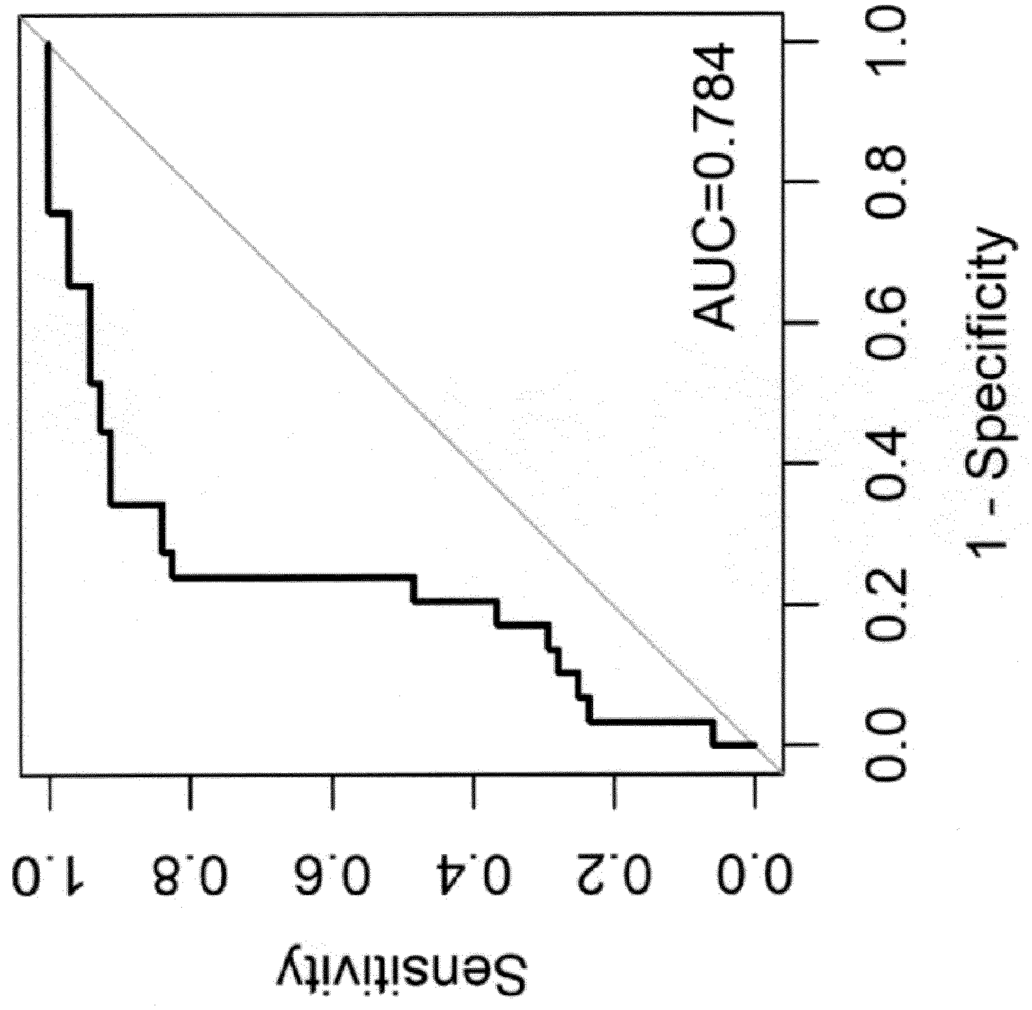


FIG. 11

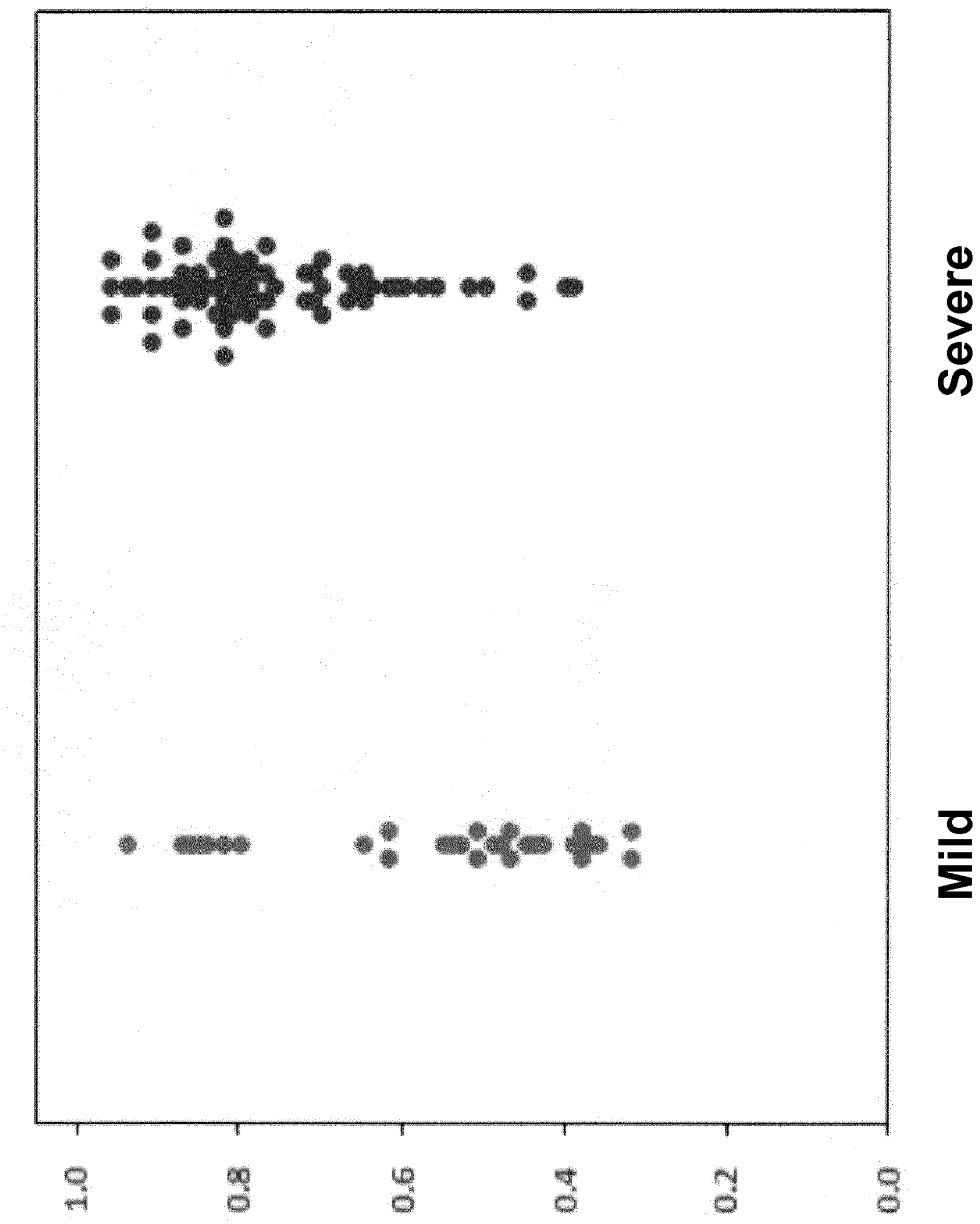


FIG. 12

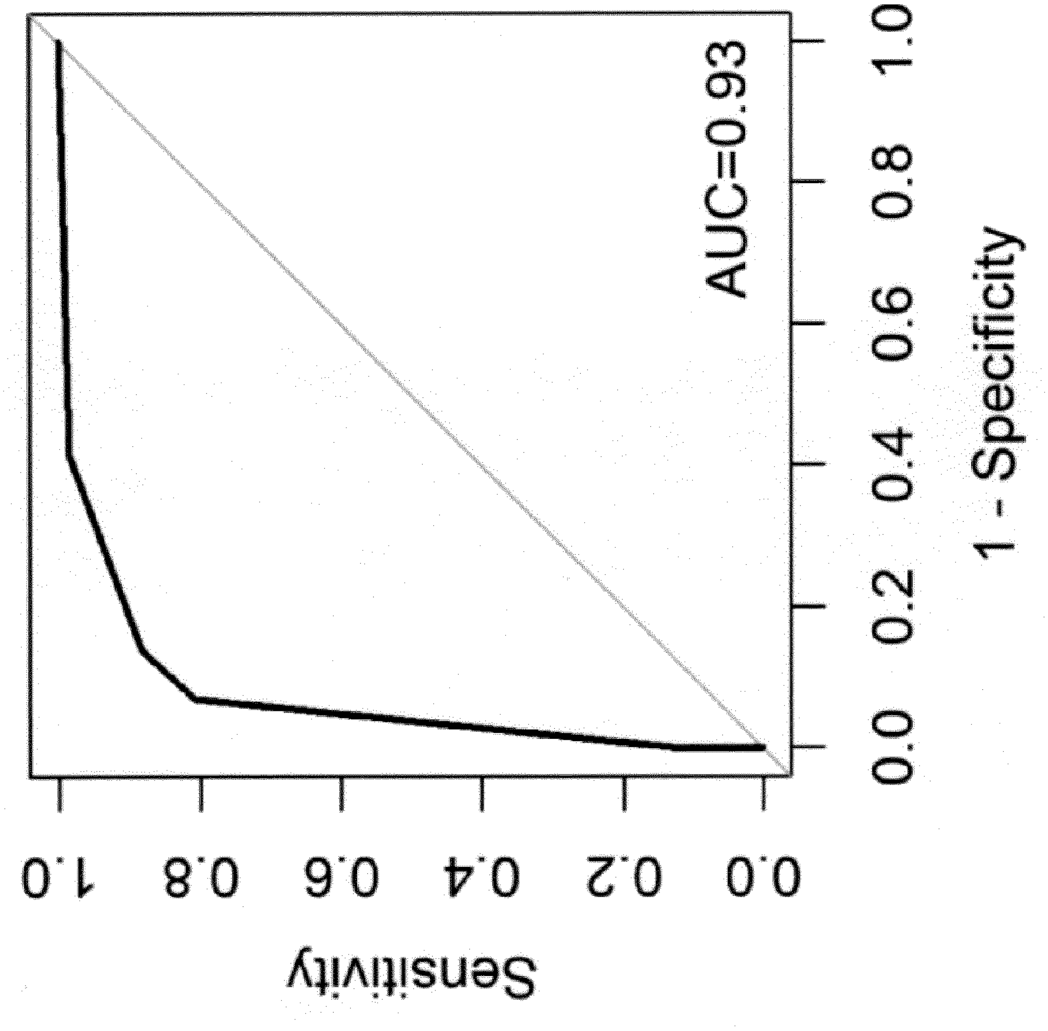
AUC=0.93
Sensitivity
0.0
0.2
0.4
0.6
0.8
1.0
1 - Specificity
0.0
0.2
0.4
0.6
0.8
1.0

FIG. 13

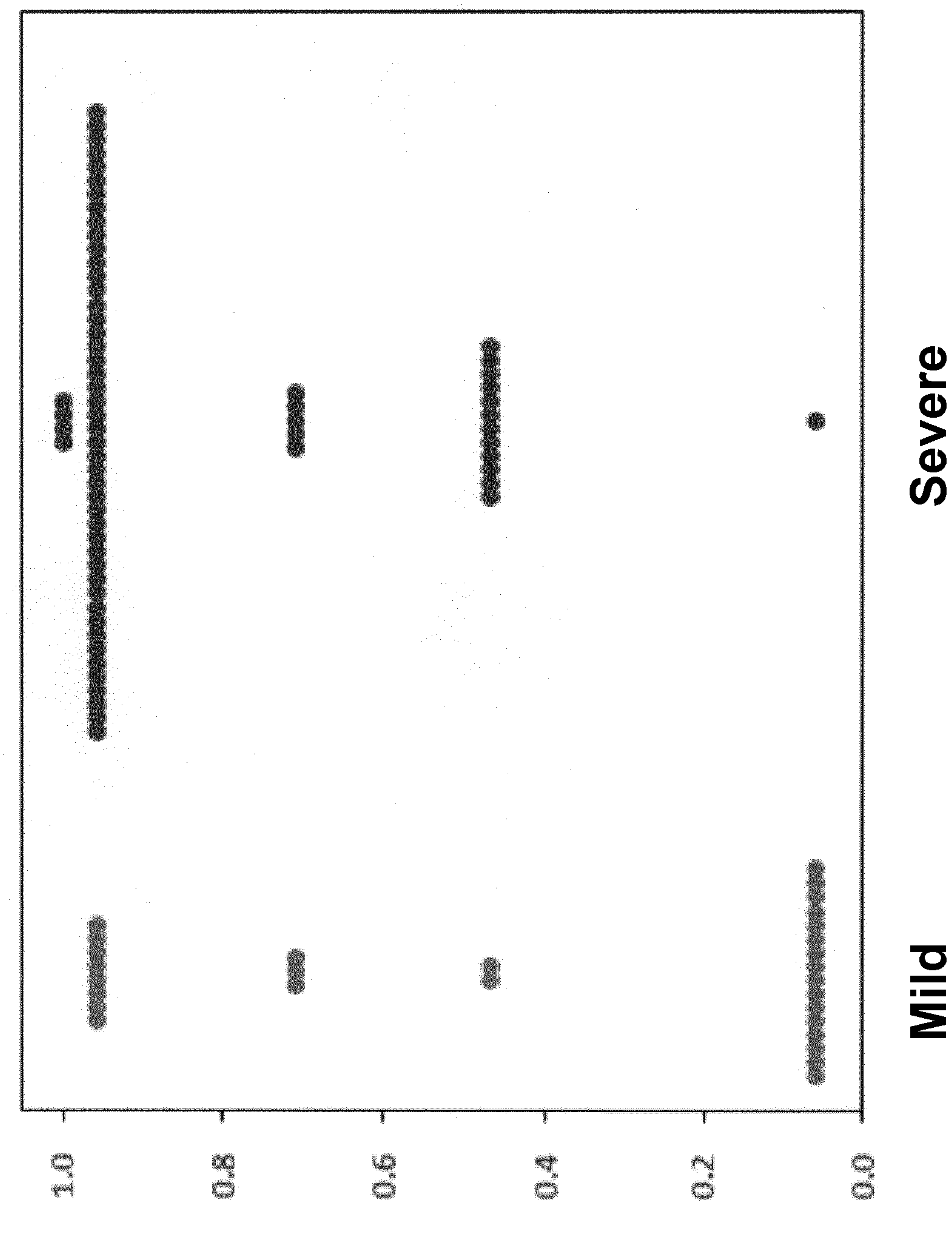


FIG. 14

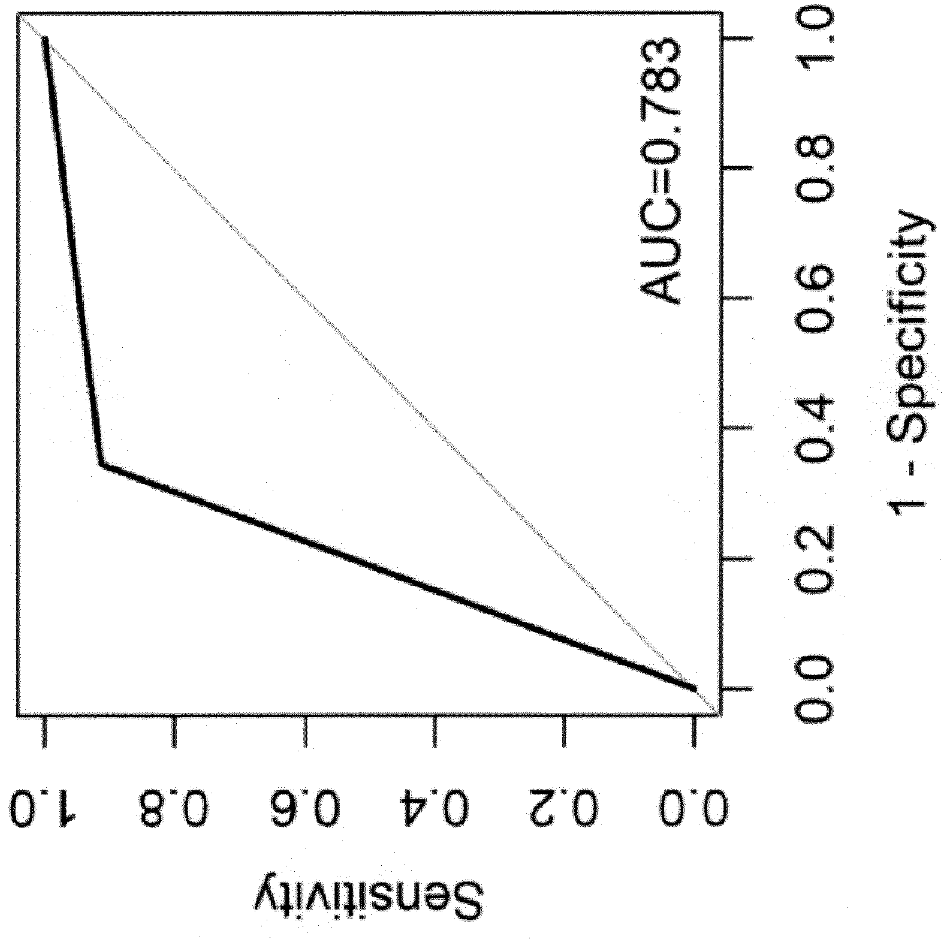

AUC=0.783
Sensitivity
0.0
0.2
0.4
0.6
0.8
1.0
1 - Specificity
0.0
0.2
0.4
0.6
0.8
1.0


FIG. 15

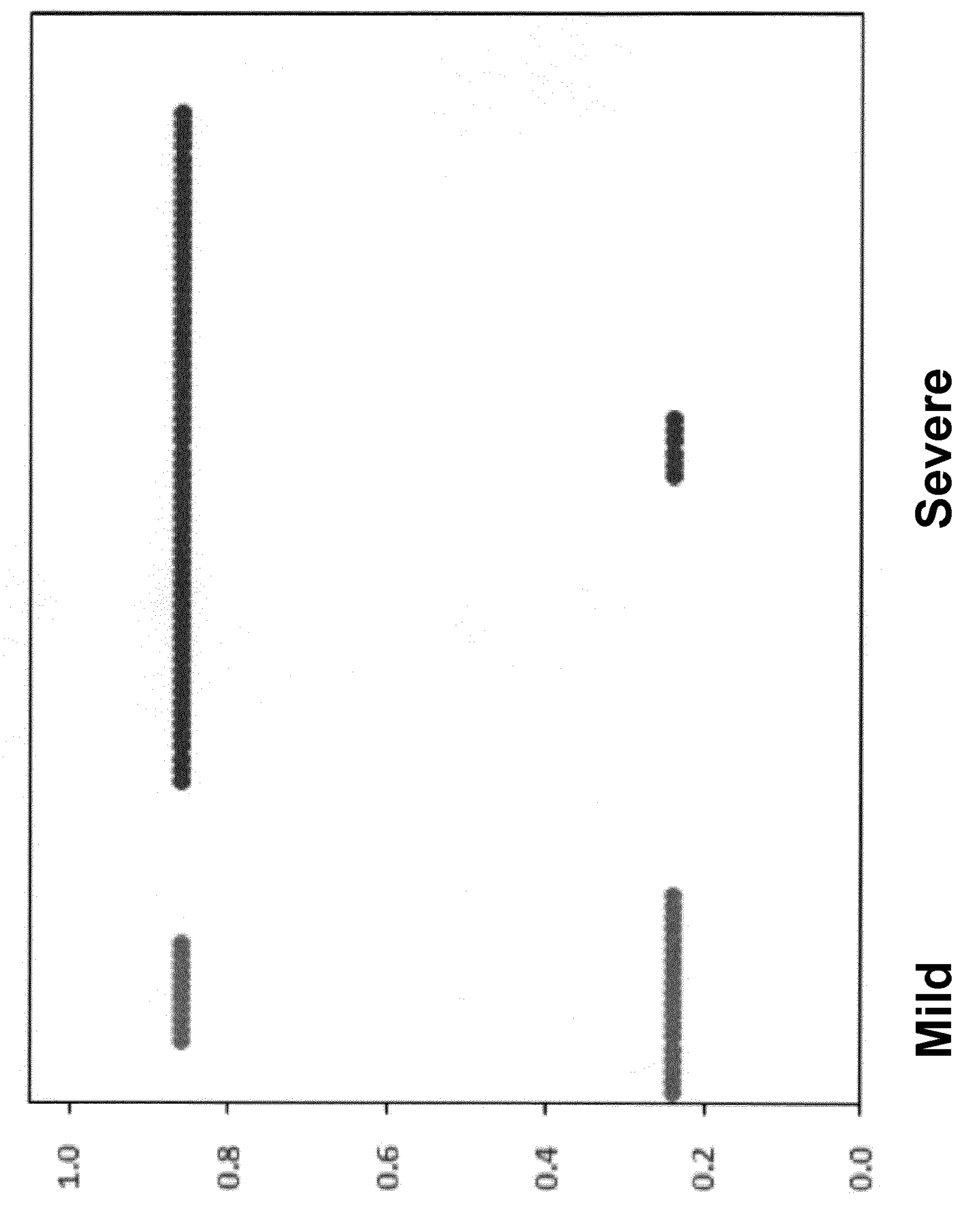


FIG. 16

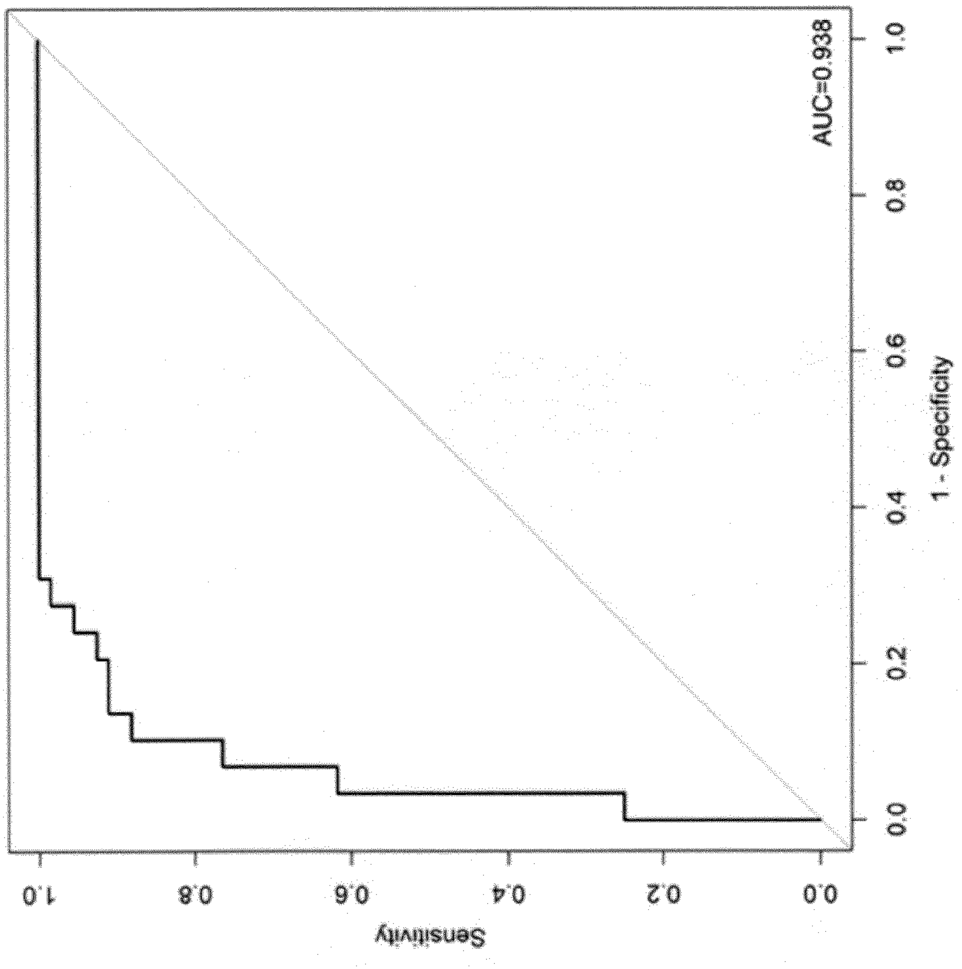


FIG. 17

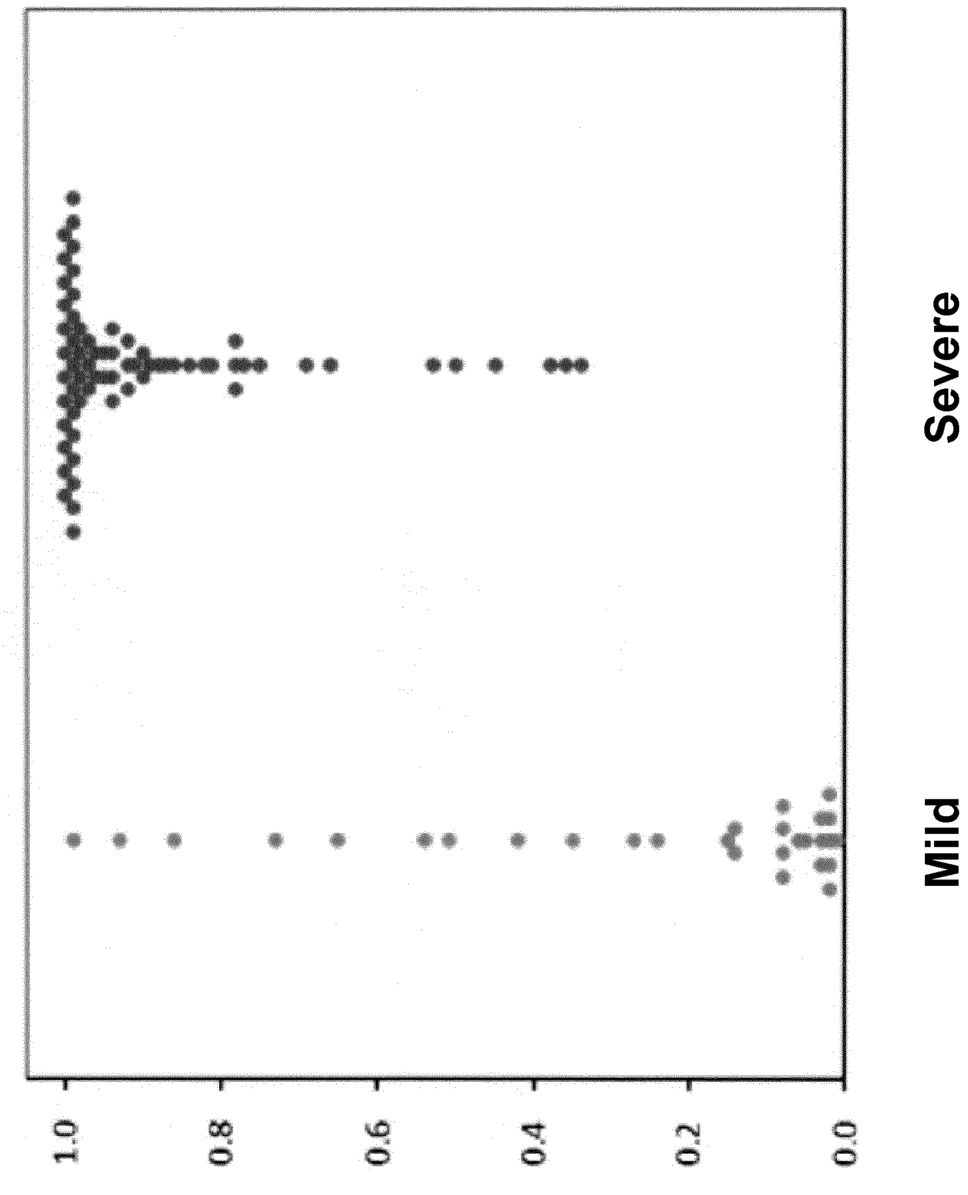


FIG. 18

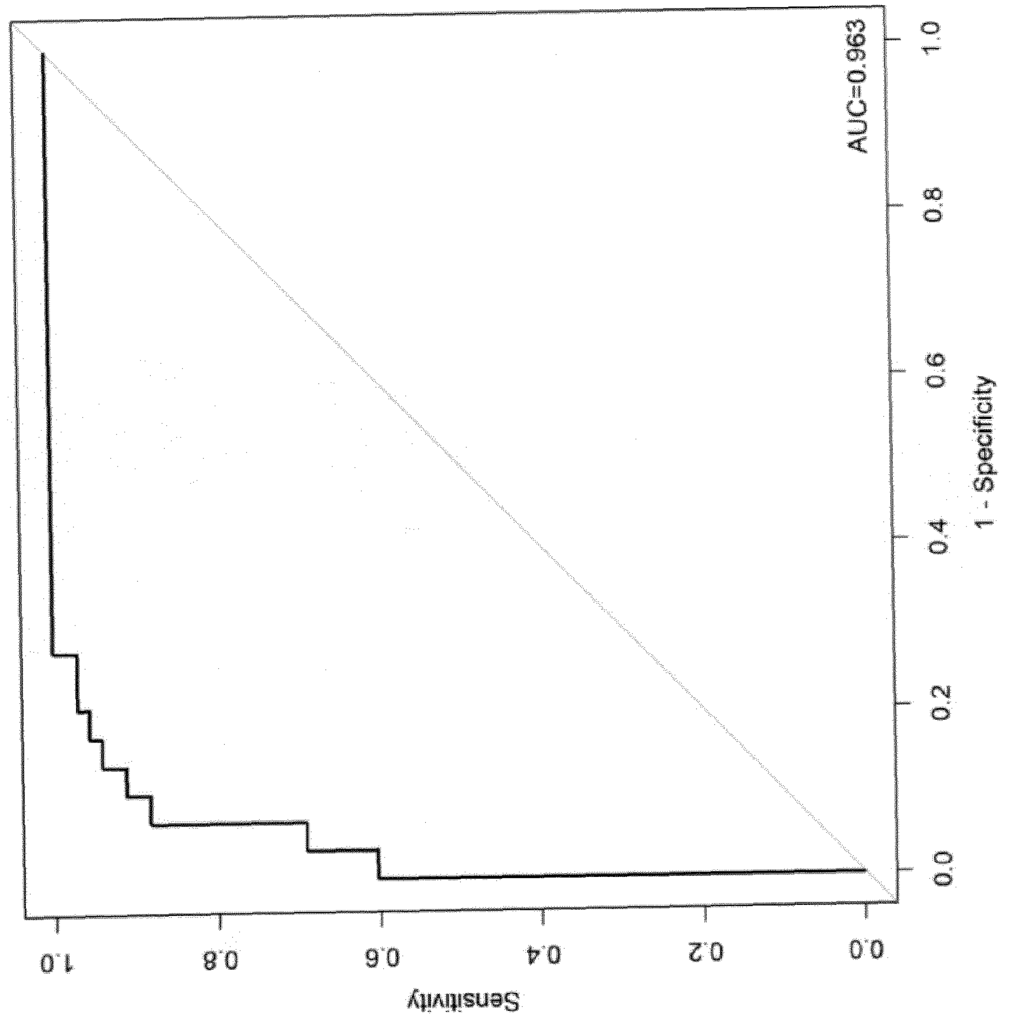


FIG. 19

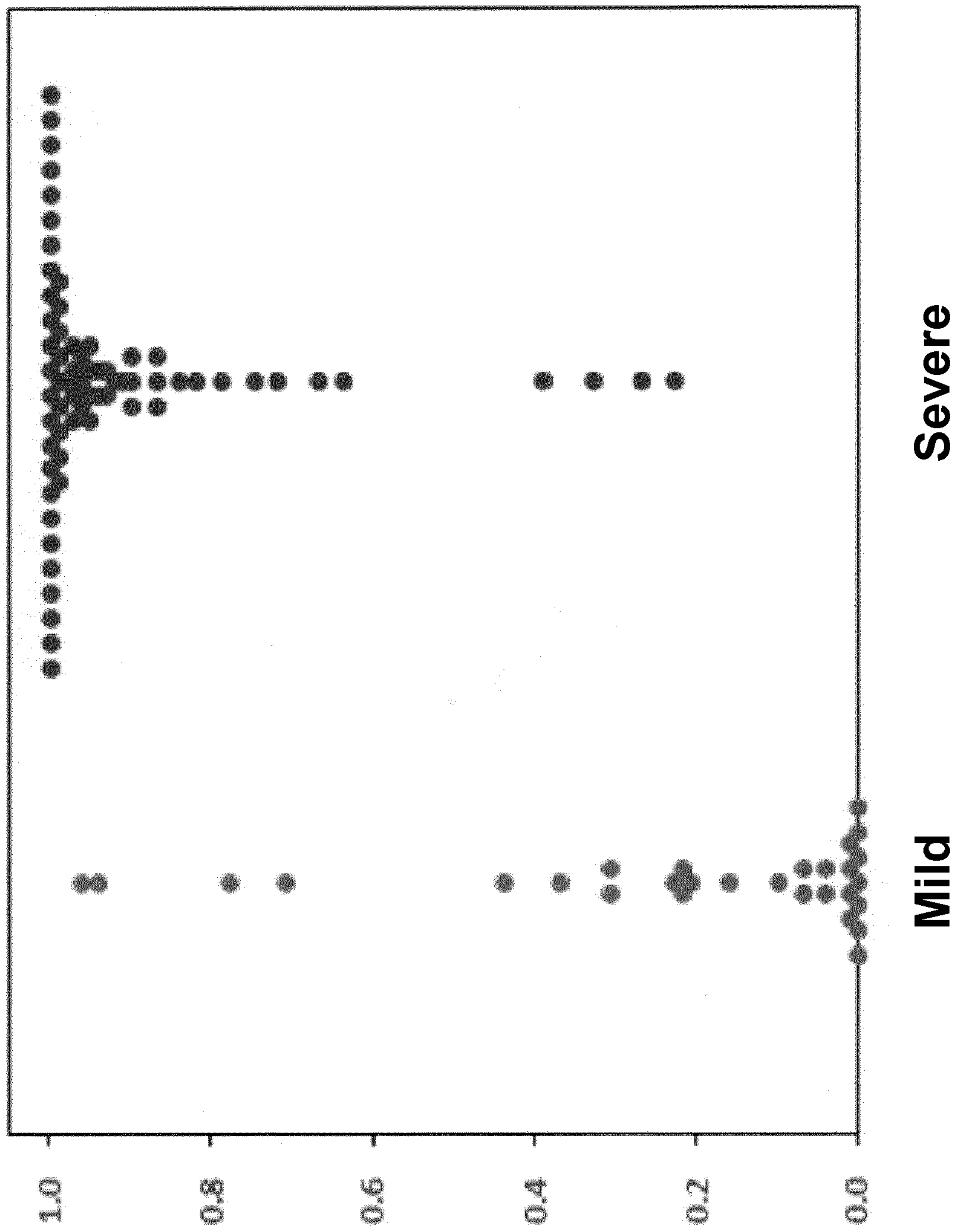


FIG. 20

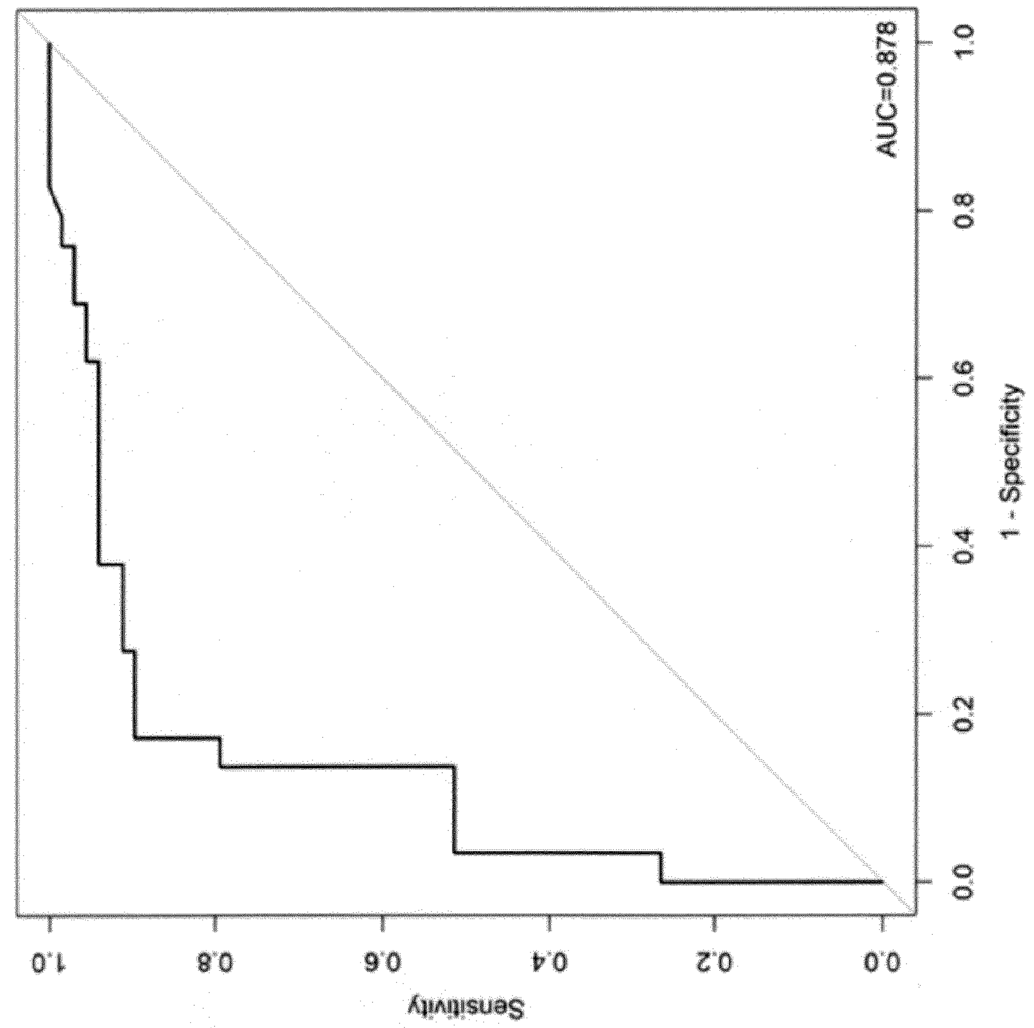
AUC=0.878
Sensitivity
1.0
0.8
0.6
0.4
0.2
0.0
1 - Specificity
0.0
0.2
0.4
0.6
0.8
1.0

FIG. 21

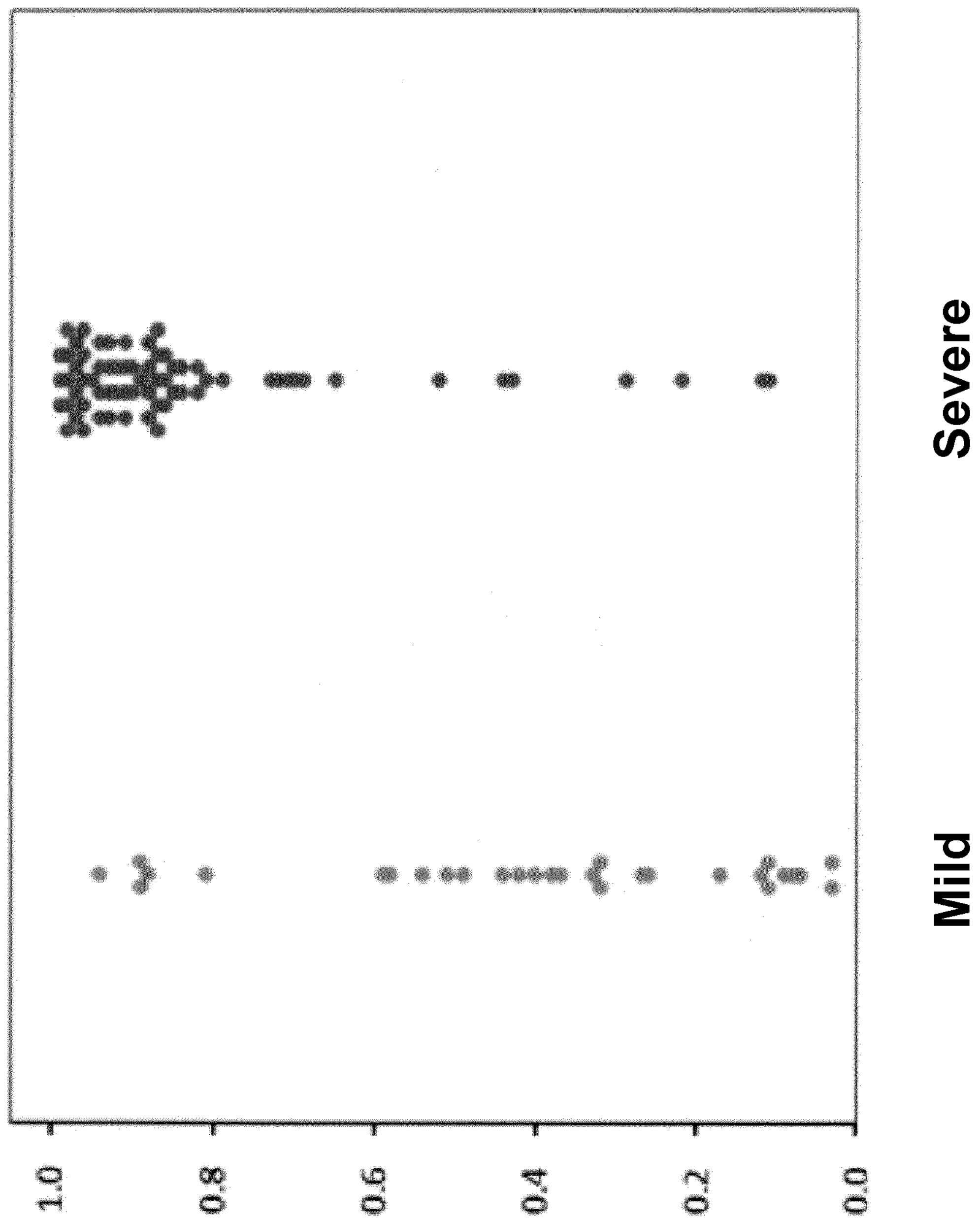


FIG. 22

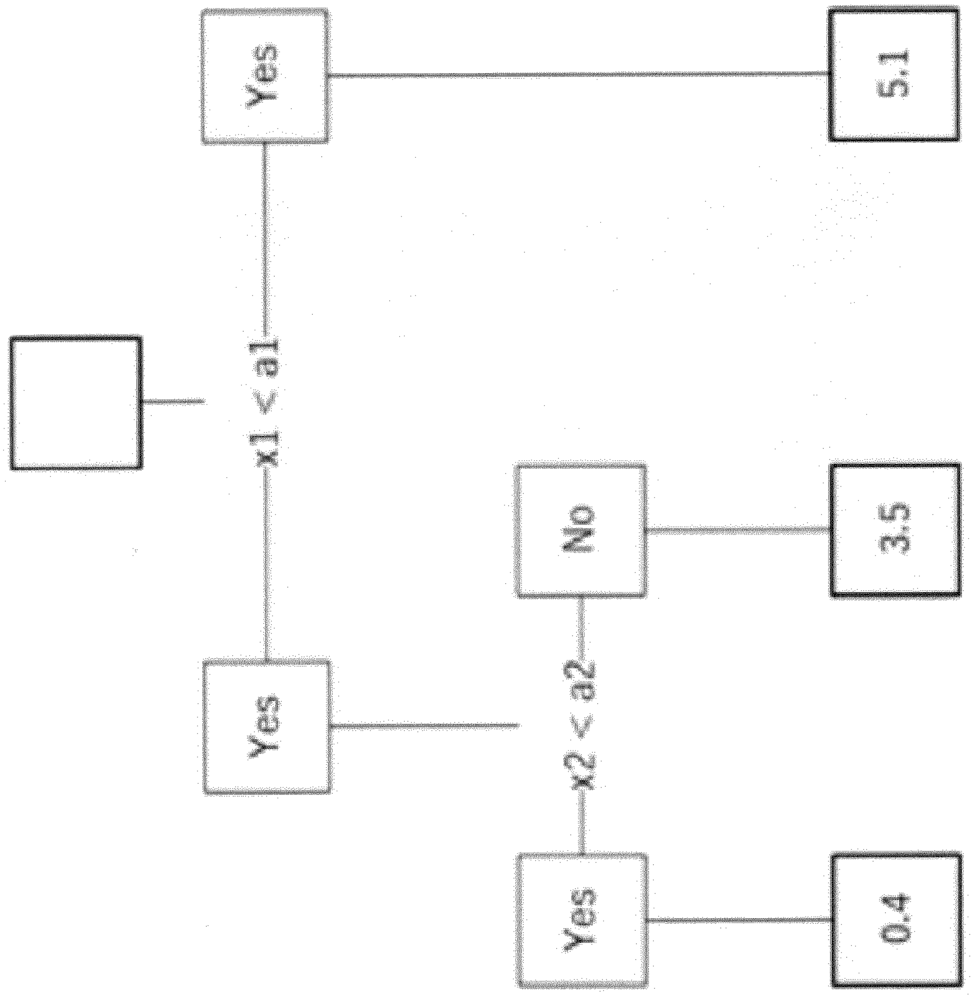


FIG. 23

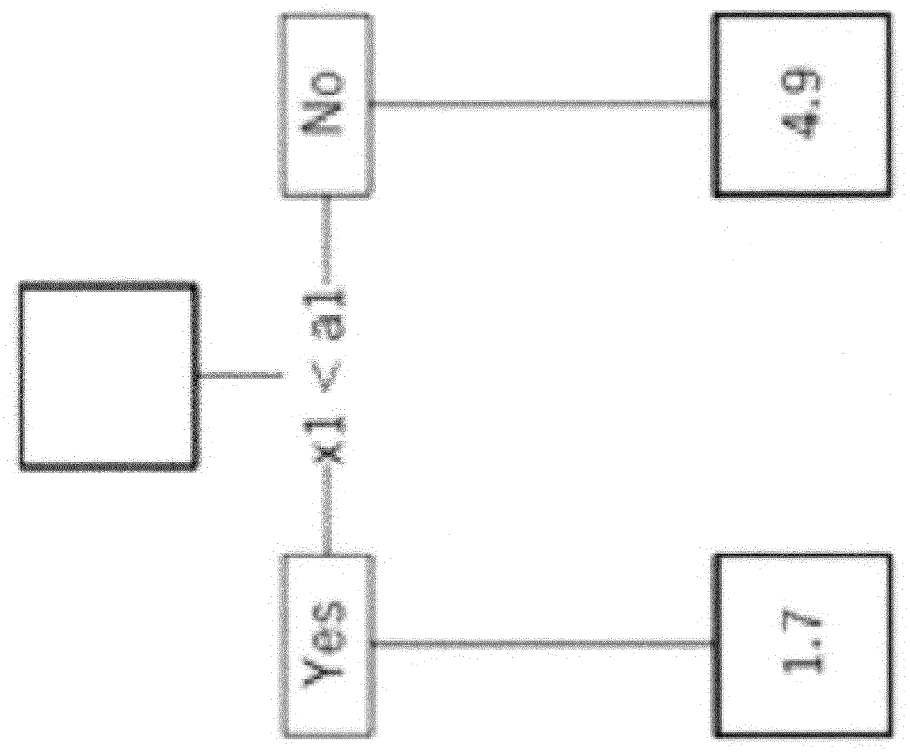


FIG. 24

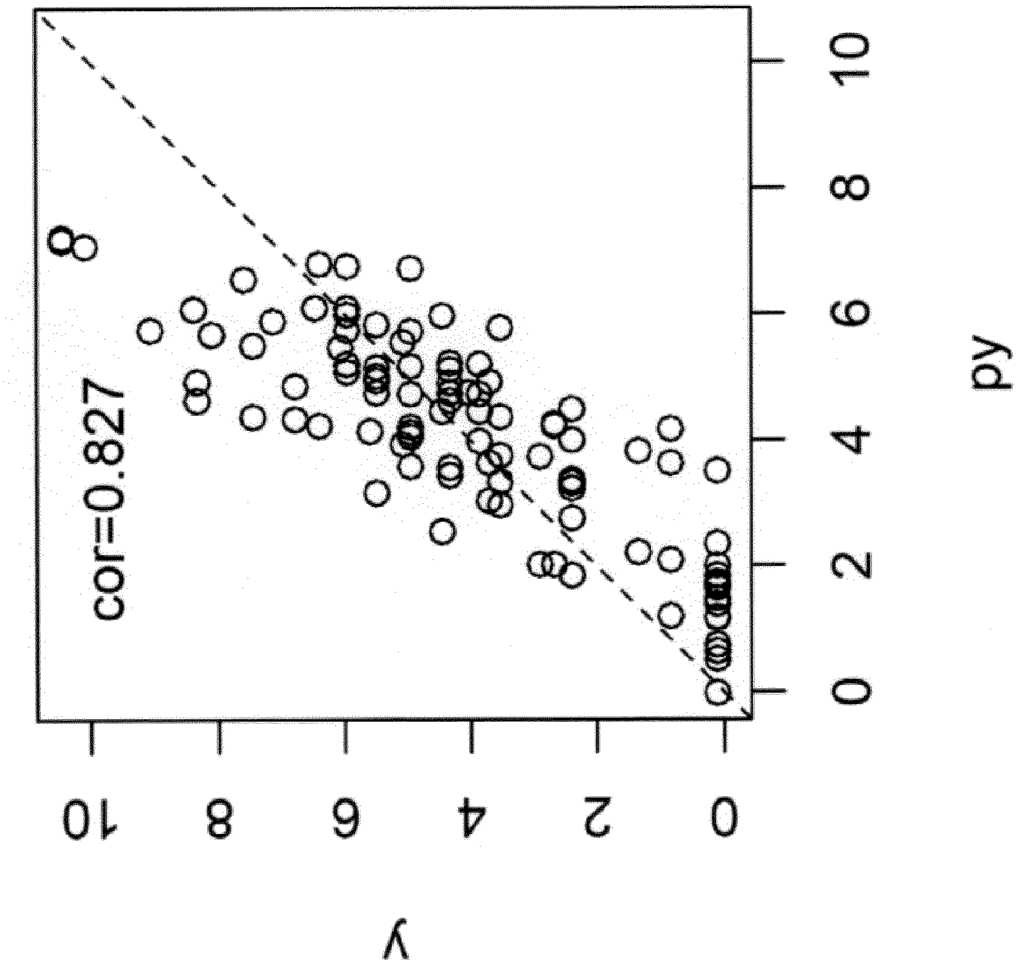


FIG. 25

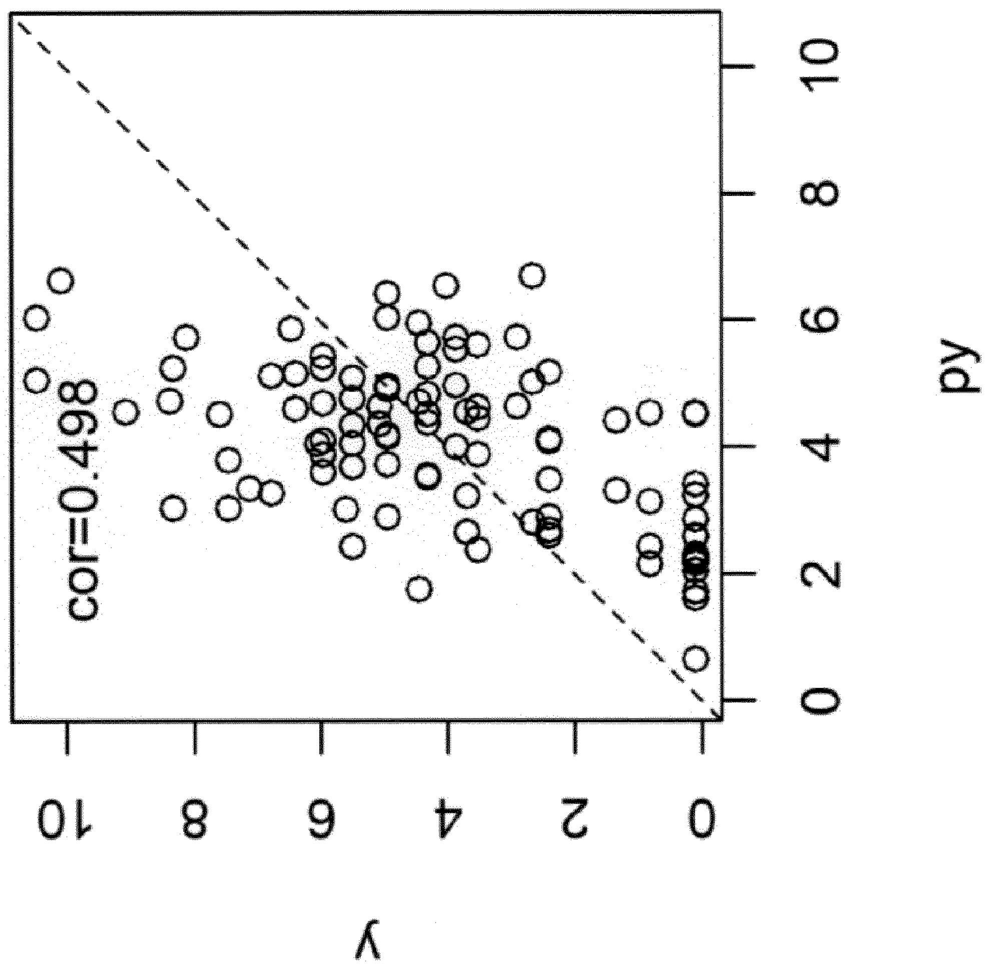


FIG. 26

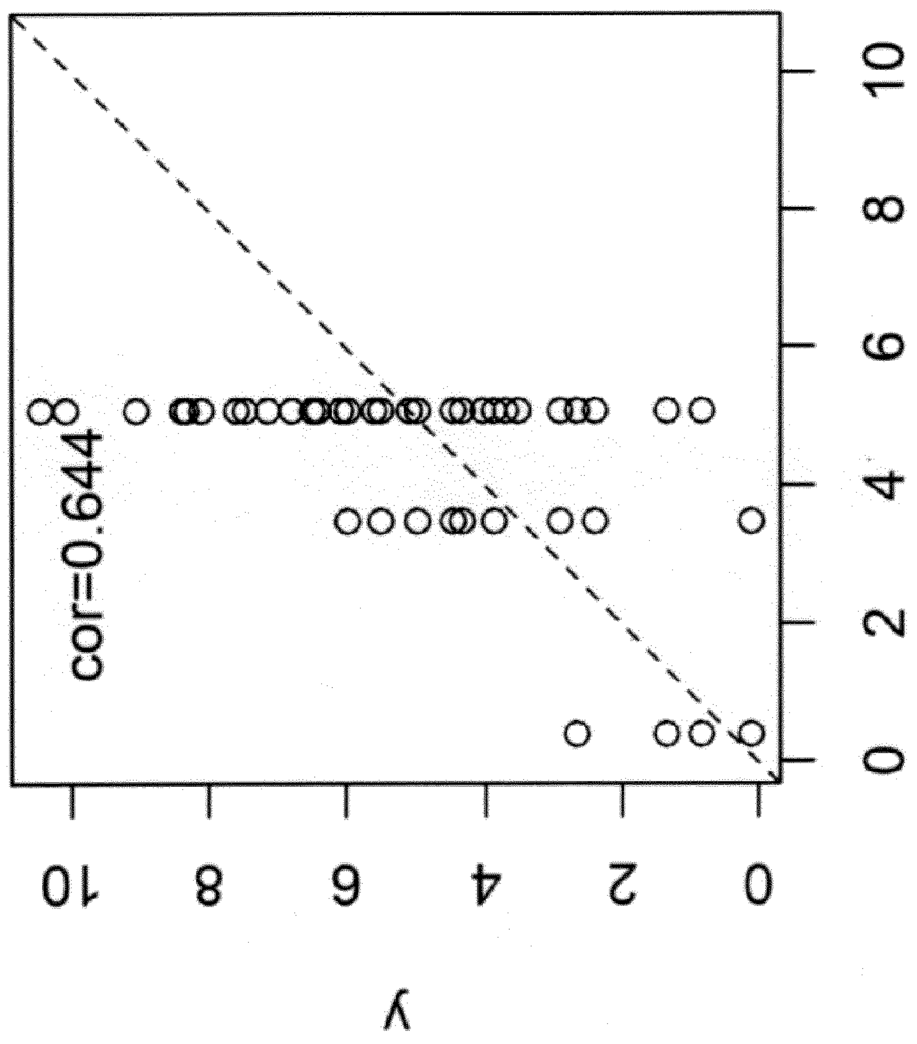


FIG. 27

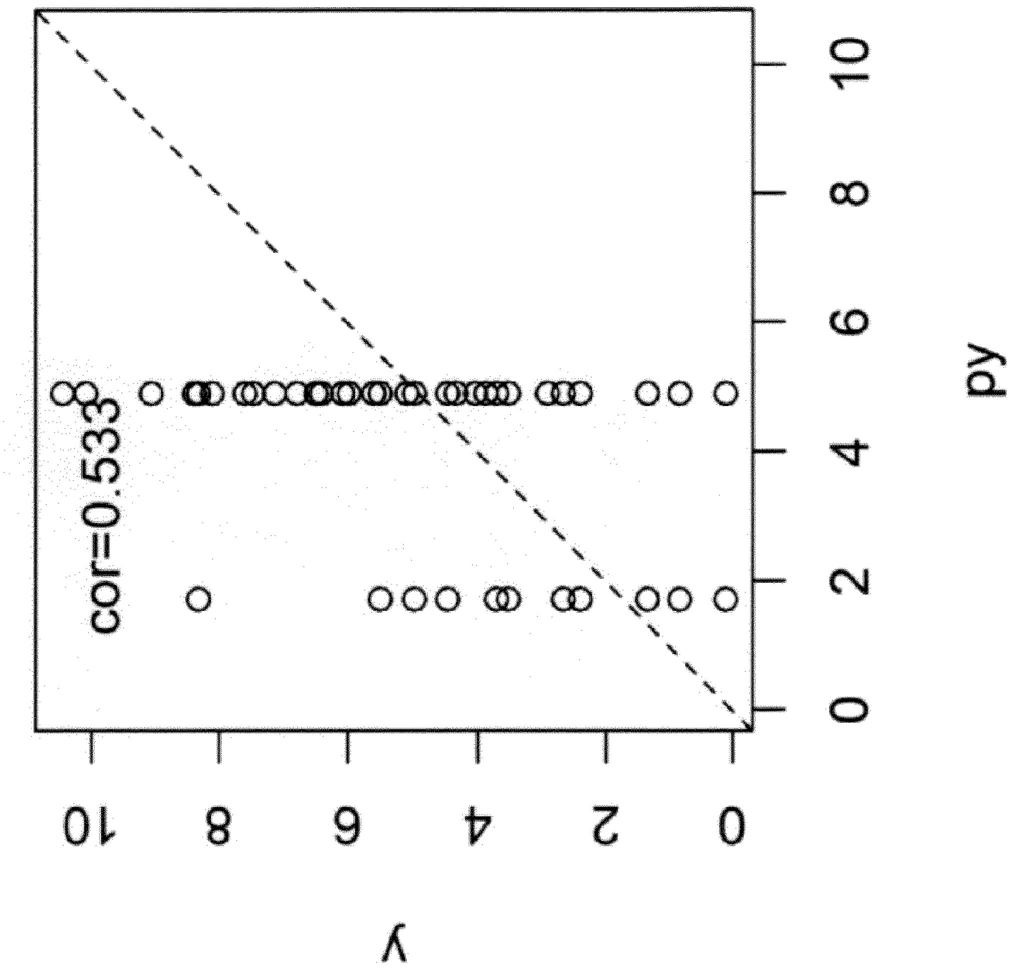


FIG. 28

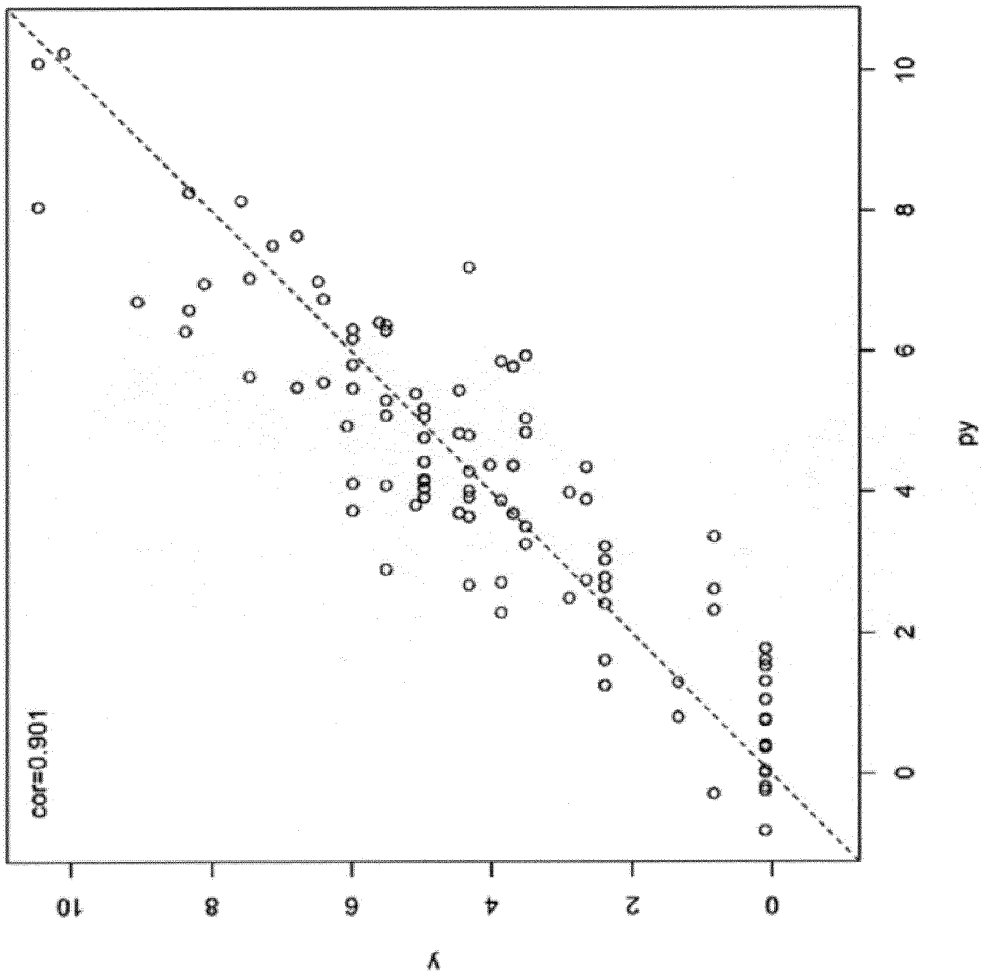


FIG. 29

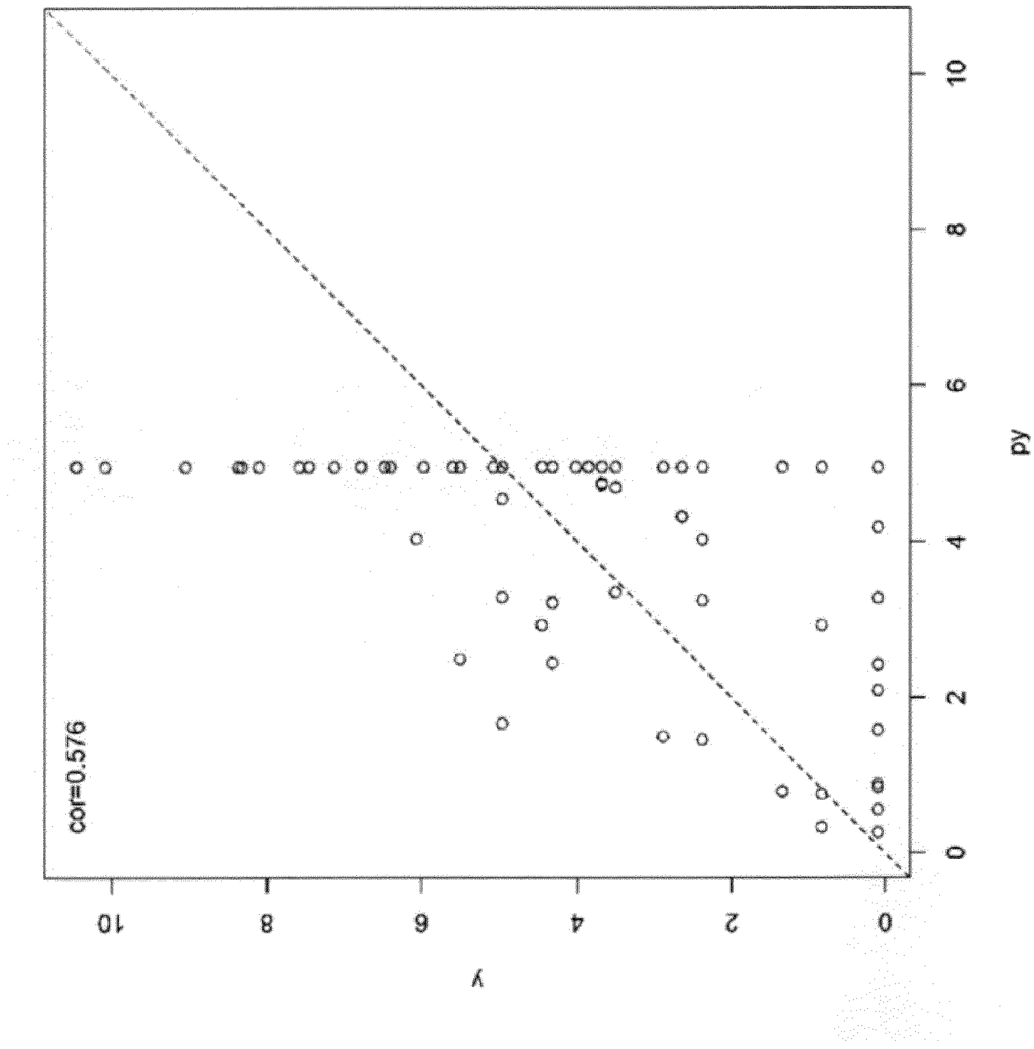


FIG. 30

## INTERNATIONAL SEARCH REPORT

International application No. **PCT/JP2021/032730**

**A. CLASSIFICATION OF SUBJECT MATTER**

***G01N 33/53***(2006.01)i
FI: G01N33/53 Q ZNA; G01N33/53 N

According to International Patent Classification (IPC) or to both national classification and IPC

**B. FIELDS SEARCHED**

Minimum documentation searched (classification system followed by classification symbols)

G01N33/53

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2010/110454 A1 (NATIONAL UNIVERSITY CORPORATION NAGOYA UNIVERSITY) 30 September 2010 (2010-09-30)<br>claims | 1-17 |
| A | JP 2016-177678 A (TOSHIBA CORP) 06 October 2016 (2016-10-06)<br>abstract | 1-17 |
| A | JP 2017-511478 A (GENISPHERE, LLC) 20 April 2017 (2017-04-20)<br>abstract | 1-17 |
| A | JP 2015-121495 A (UNIV NAGOYA) 02 July 2015 (2015-07-02)<br>claims | 1-17 |
| A | WO 2017/195871 A1 (APPLIED MEDICAL ENZYME RES INSTITUTE CORPORATION) 16 November 2017 (2017-11-16)<br>abstract | 1-17 |
| A | WO 2008/144850 A1 (ADVANCED DIAGNOSTIC SYSTEMS LTD) 04 December 2008 (2008-12-04)<br>abstract | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 October 2021** | **09 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/032730**

**C. DOCUMENTS CONSIDERED TO BE RELEVANT**

| Category* | Citation of document, with indication, where appropriate, of the relevant passages | Relevant to claim No. |
|---|---|---|
| A | JP 2011-95132 A (UNIV OF TOKUSHIMA) 12 May 2011 (2011-05-12)<br>claims | 1-17 |
| A | 木戸博, クラススイッチを基盤に免疫応答をモニターする高性能タンパクチップの臨床応用, アレルギー, 01 July 2016, vol. 65, no. 6, pp. 764-769<br>page 768, (KIDO, Hiroshi. Clinical application of a highly sensitive allergen microarray which can monitor immune response by immunoglobulin class switching. Japanese Journal of Allergology.) | 1-17 |
| A | JP 2002-303628 A (POLA CHEM IND INC) 18 October 2002 (2002-10-18)<br>claims | 1-17 |
| A | JP 4292262 B2 (ALLERTEIN THERAPEUTICS LLC) 08 July 2009 (2009-07-08)<br>claims | 1-17 |
| A | FEYZKHANOVA, Guzel. Development of a microarray-based method for allergen-specific IgE and IgG4 detection. Clinical Proteomics., 09 January 2017, vol. 14, no.1, Page. Article number: 1<br>abstract | 1-17 |
| A | MURARO, A. The urgent need for a harmonized severity scoring system for acute allergic reactions. Allergy., 13 January 2018, vol. 73, no. 9, pp. 1792-1800<br>abstract | 1-17 |
| A | SACKESEN, Cansin. A new Luminex-based peptide assay to identify reactivity to baked, fermented, and whole milk. Allergy., 29 July 2018, vol. 74, no. 2, pp. 327-336<br>abstract | 1-17 |
| A | SICHERER, Scott H. Clinical factors associated with peanut allergy in a high-risk infant cohort. Allergy., 23 May 2019, vol. 74, no. 11, pp. 2199-2211<br>abstract | 1-17 |
| A | SUPRUN, Maria. Ovomucoid epitope-specific repertoire of IgE, IgG4, IgG1, IgA1, and IgD antibodies in egg-allergic children. Allergy., 11 May 2020, vol. 75, no. 10, pp. 2633-2643<br>abstract | 1-17 |
| P, A | 山出史也, ペプチドアレイチップを用いた牛乳アレルゲンエピトープ反応性解析による牛乳アレルギー患児の重症度予測, 日本小児アレルギー学会誌, 29 September 2020, vol. 34, no. 4, page 503<br>O-71, (YAMADE, Fumiya. Japanese Journal of Pediatric Allergy and Clinical Immunology.), non-official translation (Severity prediction of child patient with milk allergy by epitope-reactive analysis of milk allergens using petit array chips) | 1-17 |
| A | 中里恵美子, 0歳アレルギー患児における食物特異的IgE,IgG4抗体値とアレルギー諸因子の関連について 2歳以上アレルギー患児群との比較, アレルギー, January 1991, vol. 40, no. 1, pp. 8-20<br>abstract, (NAKAZATO, Emiko. Japanese Journal of Allergology.), non-official translation (Relationship specific IgE and IgG4 antibody to food allergen and other allergic factors in 0-year old allergic children, comparison with the group of allergic children over 2 years old) | 1-17 |

Form PCT/ISA/210 (second sheet) (January 2015)

**INTERNATIONAL SEARCH REPORT**

International application No.
**PCT/JP2021/032730**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/032730**

| Patent document cited in search report | Publication date (day/month/year) | Patent family member(s) | Publication date (day/month/year) |
|---|---|---|---|
| WO 2010/110454 A1 | 30 September 2010 | (Family: none) | |
| JP 2016-177678 A | 06 October 2016 | (Family: none) | |
| JP 2017-511478 A | 20 April 2017 | WO 2015/153151 A1<br>abstract<br>US 2017/0219578 A1<br>EP 3126837 A1<br>CN 106662578 A | |
| JP 2015-121495 A | 02 July 2015 | (Family: none) | |
| WO 2017/195871 A1 | 16 November 2017 | EP 3457134 A1<br>abstract<br>US 2019/0391158 A1<br>CN 109952510 A | |
| WO 2008/144850 A1 | 04 December 2008 | (Family: none) | |
| JP 2011-95132 A | 12 May 2011 | (Family: none) | |
| JP 2002-303628 A | 18 October 2002 | (Family: none) | |
| JP 4292262 B2 | 08 July 2009 | WO 1999/039211 A1<br>claims<br>JP 2002-502039 A<br>US 6238925 B1<br>EP 1051626 A1<br>EP 1939628 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010110454 A
- JP 2020150142 A
- WO 2018154814 A
- WO 2019050017 A
- WO 2011024947 A


### Non-patent literature cited in the description


- **YANAGIDA et al.** *Jpn. J. Pediatr. Allergy Clin. Immunol,* 2014, vol. 28, 201
- **HINO et al.** *Arerugi.,* 2013, vol. 62 (8), 968-79
- **SAKAI et al.** *Asia Pac Allergy.,* 2017, vol. 7 (4), 234-242